# EUROPÄISCHE PATENTSCHRIFT

(11) **EP 2 104 497 B1**
(45) Veröffentlichungstag und Bekanntmachung des Hinweises auf die Patenterteilung: **25.02.2015**
(21) Anmeldenummer: 07856207.1
(22) Anmeldetag: 22.11.2007
(51) Int. Cl.: A61K 31/17, A61K 31/381, A61K 31/40, A61K 31/4164, A61K 31/4184, A61K 31/4402, A61K 31/4409, A61P 9/10, A61P 7/02, A61K 31/18, A61K 31/198, A61K 31/223, A61K 31/4406, A61K 31/4453, A61K 31/4465, C07D 213/74, C07C 307/06, C07D 211/34, C07D 295/185, C07D 213/55

(54) **SULFAMIDDERIVATE ALS INHIBITOREN VON TAFIA**
SULFAMIDE DERIVATIVES AS TAFIA INHIBITORS
DÉRIVÉS DE SULFAMIDES UTILISÉS COMME INHIBITEURS DE TAFI-A

(30) Priorität: 06.12.2006 DE 102006057413
(43) Veröffentlichungstag der Anmeldung: 30.09.2009
(73) Patentinhaber: SANOFI, 75008 Paris (FR)
(72) Erfinder: KALLUS, Christopher, 65926 Frankfurt am Main (DE); BROENSTRUP, Mark, 65926 Frankfurt am Main (DE); CZECHTIZKY, Werngard, 65926 Frankfurt am Main (DE); EVERS, Andreas, 65926 Frankfurt am Main (DE); FOLLMANN, Markus, 42489 Wülfrath (DE); HALLAND, Nis, 65926 Frankfurt am Main (DE); SCHREUDER, Herman, 65926 Frankfurt am Main (DE)
(74) Vertreter: Then, Johann
(86) Internationale Anmeldenummer: PCT/EP2007/010101
(87) Internationale Veröffentlichungsnummer: WO 2008/067909

(56) Entgegenhaltungen:
- EP-A- 0 611 776
- EP-A- 0 641 779
- WO-A-00/44335
- WO-A-99/11606
- WO-A-2005/105781
- US-B1- 6 214 851
- DATABASE CA [Online] CHEMICAL ABSTRACTS SERVICE, COLUMBUS, OHIO, US; BECU, F. ET AL: "Synthetic approaches for thymopentin (TP-5) using the in situ silylation strategy with trimethylsilyl cyanide" XP002510046 gefunden im STN Database accession no. 1991:144007 & BULLETIN DES SOCIETES CHIMIQUES BELGES , 100(1), 15-23 CODEN: BSCBAG; ISSN: 0037-9646, 1991,
- DATABASE CA [Online] CHEMICAL ABSTRACTS SERVICE, COLUMBUS, OHIO, US; TOMATIS, R. ET AL: "N-Terminal Leu-enkephalin rearrangements. I" XP002510047 gefunden im STN Database accession no. 1977:536368 & FARMACO, EDIZIONE SCIENTIFICA , 32(8), 592-601 CODEN: FRPSAX; ISSN: 0430-0920, 1977,
- DATABASE CA [Online] CHEMICAL ABSTRACTS SERVICE, COLUMBUS, OHIO, US; SAVEL'EV, E. P. ET AL: "Synthesis of tRNA ureido derivatives as substrates for the investigation of the ribosome peptidyl transferase center" XP002510048 gefunden im STN Database accession no. 1972:536369 & FEBS LETTERS , 24(2), 201-3 CODEN: FEBLAL; ISSN: 0014-5793, 1972,
- DATABASE CA [Online] CHEMICAL ABSTRACTS SERVICE, COLUMBUS, OHIO, US; WAKI, MICHINORI ET AL: "Peptide antibiotics. XIII. Synthesis of retrogramicidin S" XP002510049 gefunden im STN Database accession no. 1969:4580 & BULLETIN OF THE CHEMICAL SOCIETY OF JAPAN , 41(8), 1909-16 CODEN: BCSJA8; ISSN: 0009-2673, 1968,
- DATABASE CA [Online] CHEMICAL ABSTRACTS SERVICE, COLUMBUS, OHIO, US; RUSCHIG, HEINRICH ET AL: "New orally effective blood sugar reducing compounds" XP002510050 gefunden im STN Database accession no. 1959:7010 & ARZNEIMITTEL-FORSCHUNG , 8, 448-54 CODEN: ARZNAD; ISSN: 0004-4172, 1958,
- DATABASE CA [Online] CHEMICAL ABSTRACTS SERVICE, COLUMBUS, OHIO, US; SCHLOGL, K. ET AL: "Peptides. XI. Determination of structure of peptides. 6. Lysyl peptides" XP002510051 gefunden im STN Database accession no. 1954:60171 & MONATSHEFTE FUER CHEMIE , 84, 937-55 CODEN: MOCMB7; ISSN: 0026-9247, 1953,
- DATABASE CA [Online] CHEMICAL ABSTRACTS SERVICE, COLUMBUS, OHIO, US; BROADBRIDGE, R. J. ET AL: "Design and synthesis of novel Src homology-2 domain inhibitors" XP002510052 gefunden im STN Database accession no. 1999:578840 & PEPTIDE SCIENCE: PRESENT AND FUTURE, PROCEEDINGS OF THE INTERNATIONAL PEPTIDE SYMPOSIUM, 1ST, KYOTO, NOV. 30-DEC. 5, 1997 , MEETING DATE 1997, 605-606. EDITOR(S): SHIMONISHI, YASUTSUGU. PUBLISHER: KLUWER, DORDRECHT, NETH. CODEN: 68BYA5, 1999,
- DATABASE CA [Online] CHEMICAL ABSTRACTS SERVICE, COLUMBUS, OHIO, US; SARUBBI, EDOARDO ET AL: "Peptide aldehydes as inhibitors of HIV protease" XP002510053 gefunden im STN Database accession no. 1993:419997 & FEBS LETTERS , 319(3), 253-6 CODEN: FEBLAL; ISSN: 0014-5793, 1993,
- DATABASE CA [Online] CHEMICAL ABSTRACTS SERVICE, COLUMBUS, OHIO, US; SHIN-WATANABE, T. ET AL: "The structure of .beta.-MAPI, a novel proteinase inhibitor" XP002510054 gefunden im STN Database accession no. 1983:4767 & TETRAHEDRON , 38(12), 1775-80 CODEN: TETRAB; ISSN: 0040-4020, 1982,
- DATABASE CA [Online] CHEMICAL ABSTRACTS SERVICE, COLUMBUS, OHIO, US; BUSTANJI, YASSER ET AL: "Berberine potently inhibits protein tyrosine phosphatase 1B: investigation by docking simulation and experimental validation" XP002510055 gefunden im STN Database accession no. 2006:662074 & JOURNAL OF ENZYME INHIBITION AND MEDICINAL CHEMISTRY , 21(2), 163-171 CODEN: JEIMAZ; ISSN: 1475-6366, 2006,
- SLATER, MARTIN J. ET AL: "Pyrrolidine-5,5-trans-lactams. 4. Incorporation of a P3/P4 Urea Leads to Potent Intracellular Inhibitors of Hepatitis C Virus NS3/4A Protease" ORGANIC LETTERS, Bd. 5, Nr. 24, 2003, Seiten 4627-4630, XP002515766

## Beschreibung

Die Erfindung betrifft neue Verbindungen der Formel I, die das Enzym TAFla (aktivierter Thrombin-aktivierbarer Fibrinolyse Inhibitor) inhibieren, Verfahren zu ihrer Herstellung und Verwendung derselben als Arzneimittel.

Das Enzym TAFIa entsteht beispielsweise durch Thrombinaktivierung aus dem Thrombin-aktivierbaren-Fibrinolyse-Inhibitor-Zymogen (TAFI). Das Enzym TAFI wird auch als Plasma Procarboxypeptidase B, Procarboxypeptidase U oder als Procarboxypeptidase R bezeichnet und ist ein Carboxypeptidase B ähnliches Proenzym (L. Bajzar, Arterioscler. Thromb. Vasc. Biol. 2000, Seiten 2511 - 2518).

Während einer Gerinnselbildung wird Thrombin als das Endprodukt der Koagulationskaskade generiert und induziert die Konversion von löslichem Plasmafibrinogen zu einer unlöslichen Fibrinmatrix. Gleichzeitig aktiviert Thrombin den endogenen Fibrinolyse-Inhibitor TAFI. Aktiviertes TAFI (TAFIa) entsteht also während der Thrombusbildung und der Lyse aus dem Zymogen TAFI unter der Einwirkung von Thrombin; Thrombomodulin im Komplex mit Thrombin verstärkt diesen Effekt etwa 1250 fach. TAFIa spaltet basische Aminosäuren am carboxy-Ende des Fibrins. Der Verlust der carboxy-terminalen Lysine als Bindestellen für Plasminogen führt dann zu einer Inhibition der Fibrinolyse: Effektive Inhibitoren von TAFIa verhindern den Verlust dieser hoch affinen Lysin-Bindungsstellen für Plasminogen und unterstützen auf diese Weise die endogene Fibrinolyse durch Plasmin: TAFIa Inhibitoren wirken profibrinolytisch.

Um die Hämostase im Blut aufrechtzuerhalten, haben sich Mechanismen ausgebildet, die zur Blutgerinnung und zur Auflösung von Gerinnseln führen; diese stehen in einem Gleichgewicht. Wenn ein gestörtes Gleichgewicht die Koagulation begünstigt, entsteht Fibrin in größeren Mengen, so dass pathologische Vorgänge der Thrombusbildung zu schweren Krankheitsbildern im Menschen führen können.
Genauso wie eine überschießende Koagulation zu schwerwiegenden thrombotisch bedingten Krankheitsbildern führen kann, besitzt eine antithrombotische Behandlung das Risiko von nicht erwünschten Blutungen durch eine Störung der Bildung eines notwendigen hämostatischen Pfropfs. Die Hemmung von TAFIa verstärkt - ohne die Koagulation und die Plättchenaggregation zu beeinflussen - die endogene Fibrinolyse d.h. das gestörte Gleichgewicht wird zugunsten der Fibrinolyse verschoben. So kann sowohl dem Aufbau eines klinisch relevanten Thrombus entgegengewirkt, als auch die Lyse eines schon bestehenden Gerinnsels verstärkt werden. Andererseits wird der Aufbau eines hämostatischen Pfropfs nicht beeinträchtigt, sodass eine Blutungsdiathese eher nicht zu erwarten ist (Bouma et al., J. Thrombosis and Haemostasis, 1, 2003, Seiten 1566 - 1574).

Inhibitoren von TAFIa sind bereits in der Internationalen Anmeldung WO2005/105781 beschrieben worden.

Die erfindungsgemäßen TAFIa Inhibitoren eignen sich für eine prophylaktische als auch für eine therapeutische Anwendung am Menschen, die an Erkrankungen leiden, die mit Thrombosen, Embolien, Hyperkoagulabilität oder fibrotischen Veränderungen einhergehen. Sie können zur Sekundär-Prevention eingesetzt werden und eignen sich sowohl für eine akute als auch für eine Langzeittherapie.

Die Erfindung betrifft daher die Verbindung der Formel I 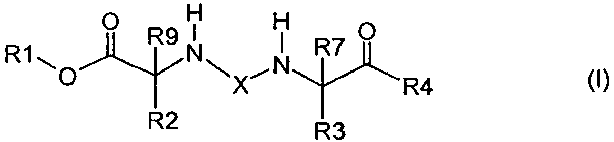 und/oder einer stereoisomeren Form der Verbindung der Formel I und/oder Gemische dieser Formen in jedem Verhältnis, und/oder ein physiologisch verträgliches Salz der Verbindung der Formel I, wobei
X für -SO₂- steht,
R1 für
   1) Wasserstoffatom,
   2) -((C₁-C₆)-Alkyl,
   3) -((C₀-C₄)-Alkylen-(C₃-C₁₂)-Cycloalkyl oder
   4) -((C₁-C₆)-Alkylen-(C₆-C₁₄)-Aryl steht,
R2 für den Rest der Formel II

   -(A1)ₘ-A2 (II)

   steht, worin
   m die ganze Zahl Null oder 1 bedeutet,
   A1 für
      1) -(CH₂)ₙ-, worin n die ganze Zahl Null, 1, 2 oder 3 bedeutet,
      2) -NH-(CH₂)ₙ-, worin n die ganze Zahl Null, 1, 2 oder 3 bedeutet,
      3) -NH(C₁-C₆)-Alkyl)-(CH₂)ₙ-, worin n die ganze Zahl Null, 1, 2 oder 3 bedeutet,
      4) -NH((C₃-C₆)-Cycloalkyl)-(CH₂)ₙ-, worin n die ganze Zahl Null, 1, 2 oder 3 bedeutet,
      5) -O-(CH₂)ₙ-, worin n die ganze Zahl Null, 1, 2 oder 3 bedeutet, oder
      6) -(CH₂)ₙ-SOₓ-, worin n die ganze Zahl Null, 1, 2 oder 3 bedeutet und x die ganze Zahl Null, 1 oder 2 bedeutet, steht,
   A2 für
      1) Het, wobei unter Het ein 4- bis 15-gliedriges heterocyclisches Ringsystem mit 4 bis 15 Ringatomen verstanden wird, die in ein, zwei oder drei miteinander verbundenen Ringsystemen vorliegen und die ein, zwei, drei oder vier gleiche oder verschiedene Heteroatome aus der Reihe Sauerstoff, Stickstoff oder Schwefel enthalten und unsubstituiert oder unabhängig voneinander ein-, zwei- oder dreifach durch ein -(C₁-C₃)-Alkyl, Halogen, -NH₂, -CF₃ oder -O-CF₃ substituiert ist,
      2) -(C₀-C₆)-Alkylen-NH₂,
      3) -(C₁-C₆)-Alkylen-NH-C(=NH)-NH₂,
      4) -(C₁-C₆)-Alkylen-NH-C(=NH)-(C₁-C₄)-Alkyl,
      5) -(C₀-C₄)-Alkylen-O-NH-C(=NH)-NH₂,
      6) -(C₀-C₄)-Alkylen-NH-C(O)-(C₁-C₆)-Alkyl,
      7) -(C₁-C₆)-Alkylen-NH-C(O)-O-(C₁-C₄)-Alkylen-Aryl, wobei Aryl unsubstituiert oder durch -NH₂ substituiert ist oder durch -NH₂ und ein-, zwei- oder dreifach durch R15 substituiert ist,
      8) -(C₃-C₈)-Cycloalkyl-NH₂, oder
      9) -(C₀-C₄)-Alkylen-(C₆-C₁₄)-Aryl, wobei Aryl unsubstituiert oder durch -NH₂ substituiert ist oder durch -NH₂ und ein-, zwei- oder dreifach durch R15 substituiert ist, steht,
R3 für
   1) -(C₁-C₆)-Alkyl,
   2) -(C₀-C₄)-Alkylen-(C₃-C₁₂)-Cycloalkyl,
   3) -(C₁-C₆)-Alkylen-(C₆-C₁₄)-Aryl, wobei Aryl unabhängig voneinander ein-, zwei- oder dreifach durch R15 substituiert ist,
   4) -(C₀-C₈)-Alkylen-N(R5)-PG,
   5) -(C₁-C₆)-Alkylen-NH-C(O)-O-(C₁-C₄)-Alkylen-Aryl, wobei Aryl unabhängig voneinander ein-, zwei- oder dreifach durch R15 substituiert ist,
   6) -(C₀-C₄)-Alkylen-(C₆-C₁₄)-Aryl-(C₀-C₄)-Alkylen-N(R5)-PG,
   7) -(C₀-C₈)-Alkylen-O-PG,
   8) -(C₀-C₄)-Alkylen-(C₆-C₁₄)-Aryl-(C₀-C₄)-Alkylen-O-PG,
   9) -(C₀-C₈)-Alkylen-C(O)-O-PG,
   10) -(C₀-C₄)-Alkylen-(C₆-C₁₄)-Aryl-(C₀-C₄)-Alkylen-C(O)-O-PG oder
   11) Wasserstoffatom, steht,
R4 für-N(R6)₂, steht,
   wobei R6 gleich oder verschieden sind und unabhängig voneinander für
   1) Wasserstoffatom,
   2) -(C₁-C₆)-Alkyl,
   3) -(C₀-C₄)-Alkylen-(C₃-C₁₂₎-Cycloalkyl, wobei Cycloalkyl unsubstituiert oder unabhängig voneinander ein-, zwei-, drei- oder vierfach durch R11, Halogen, -C(O)-O-R11, -(C₁-C₄)-Alkyl-O-R11 oder -O-(C₁-C₄)-Alkyl substituiert ist,
   4) -(C₀-C₆)-Alkylen-(C₆-C₁₄)-Aryl, wobei Aryl und Alkylen unsubstituiert oder unabhängig voneinander ein-, zwei-, drei- oder vierfach durch R11, Halogen, -C(O)-O-R11, -(C₁-C₄)-Alkyl-O-R11, -C(O)-N(R8)₂ oder -O-(C₁-C₄)-Alkyl substituiert sind,
   5) -(C₀-C₈)-Alkylen-N(R5)-PG,
   6) -(C₀-C₄)-Alkylen-(C₆-C₁₄)-Aryl-(C₀-C₄)-Alkyl-N(R5)-PG,
   7) -(C₀-C₈)-Alkylen-O-PG,
   8) -(C₀-C₄)-Alkylen-(C₆-C-₁₄)-Aryl-(C₀-C₄)-Alkyl-O-PG,
   9) -(C₀-C₈)-Alkylen-C(O)-O-R11,
   10) -(C₀-C₄)-Alkylen-(C₆-C-₁₄)-Aryl-(C₀-C₄)-Alkyl-C(O)-O-PG,
   11) -(C₀-C₄)-Alkylen-Het, wobei unter Het ein 4- bis 15-gliedriges heterocyclisches Ringsystem verstanden wird mit 4 bis 15 Ringatomen, die in ein, zwei oder drei miteinander verbundenen Ringsystemen vorliegen und die ein, zwei, drei oder vier gleiche oder verschiedene Heteroatome aus der Reihe Sauerstoff, Stickstoff oder Schwefel enthalten, wobei Het oder Alkylen unsubstituiert oder unabhängig voneinander ein-, zwei- oder dreifach durch R11, Halogen, -C(O)-O-R11, -(C₁-C₄)-Alkyl-O-R11 oder -O-(C₁-C₄)-Alkyl substituiert sind,
   12) -(C₁-C₃)-Fluoralkyl,
   13) -(C₀-C₄)-Alkylen-CH(R11)-C(O)-NH₂,
   14) -(C₀-C₄)-Alkylen-CH(R11)-C(O)-NH-(C₁-C₄)-Alkyl, oder
   15) -(C₀-C₄)-Alkylen-CH(R11)-C(O)-NH-CH(R12)-R13 stehen,
   oder die beiden Reste R6 zusammen mit dem N-Atom, an das sie gebunden sind, einen mono- oder bicyclischen Ring mit 4 bis 9 Ringatomen bilden, der gesättigt, teilweise gesättigt oder aromatisch ist, wobei der Ring unsubstituiert oder ein- oder zweifach durch -(C₁-C₄)-Alkyl, -C(O)-O-R11, Halogen, -(C₁-C₄)-Alkyl-O-R11 oder Phenyl substituiert ist,
R5 für Wasserstoffatom oder-(C₁-C₆)-Alkyl steht,
PG für eine Schutzgruppe für die Amino-, Carboxyl- oder für die Hydroxyfunktion steht,
R7 für Wasserstoffatom oder -(C₁-C₆)-Alkyl steht,
R8 für Wasserstoffatom oder -(C₁-C₆)-Alkyl steht,
R9 für Wasserstoffatom oder -(C₁-C₆)-Alkyl steht,
R11 und R12 gleich oder verschieden sind und unabhängig voneinander für
   1) Wasserstoffatom,
   2) -(C₁-C₆)-Alkyl,
   3) -(C₀-C₄)-Alkylen-Phenyl, wobei Phenyl unsubstituiert oder oder unabhängig voneinander ein-, zwei- oder dreifach durch Halogen, -OH oder -O-(C₁-C₄)-Alkyl substituiert ist,
   4) -(C₀-C₄)-Alkylen-(C₃-C₁₂)-Cycloalkyl, wobei Cycloalkyl unsubstituiert oder unabhängig voneinander ein-, zwei-, drei- oder vierfach durch R13, Halogen, -C(O)-O-R13, -(C₁-C₄)-Alkyl-O-R13, -O-(C₁-C₄)-Alkyl oder -(C₀-C₄)-Alkylen-Phenyl substituiert ist,
   5) -(C₀-C₄)-Alkylen-C(O)-N(R13)₂ oder
   6) -(C₀-C₄)-Alkylen-Indolyl, stehen,
R13 für
   1) Wasserstoffatom,
   2) -(C₁-C₄)-Alkyl,
   3) -(C₀-C₄)-Alkylen-C(O)-O-R14,
   4) -(C₀-C₄)-Alkylen-C(O)-R14 oder
   5) -(C₀-C₄)-Alkylen-O-R14 steht,
R14 für Wasserstoffatom, -(C₁-C₄)-Alkyl, -NH₂ oder -OH, steht und
R15 für Wasserstoffatom, -(C₁-C₄)-Alkyl, -O-CF₃, -NH₂, -OH, -CF₃ oder Halogen steht.

Die Erfindung betrifft ferner die Verbindung der Formel I, wobei
X für -SO₂- steht,
R1 für
1) Wasserstoffatom oder
2) -(C₁-C₄)-Alkyl, steht,
R2 für
1) -(C₁-C₆)-Alkylen-NH₂,
2) -(C₀-C₄)-Alkylen-Pyridyl-NH₂,
3) -(C₀-C₄)-Alkylen-Piperidinyl-NH₂,
4) -(C₀-C₄)-Alkylen-Thiazolyl-NH₂,
5) -(C₁-C₆)-Alkylen-NH-C(=NH)-NH₂,
6) -(C₀-C₄)-Alkylen-(C₃-C₈)-Cycloalkyl-NH₂,
7) -(C₁-C₆)-Alkylen-NH-C(=NH)-(C₁-C₄)-Alkyl,
8) -(C₀-C₄)-Alkylen-O-NH-C(=NH)-NH₂,
9) -(C₁-C₆)-Alkylen-NH-C(O)-O-(C₁-C₄)-Alkylen-Aryl, wobei Aryl unsubstituiert oder durch -NH₂ substituiert ist oder durch -NH₂ und ein-, zwei- oder dreifach durch R15 substituiert ist,
10) -(C₀-C₄)-Alkylen-NH-C(O)-(C₁-C₄)-Alkyl,
11) -(C₀-C₄)-Alkylen-(C₆-C₁₄)-Aryl, wobei Aryl unsubstituiert oder durch -NH₂ substituiert ist oder durch -NH₂ und ein-, zwei- oder dreifach durch R15 substituiert ist, oder
12) -(C₁-C₄)-Alkylen-SOₓ-(C₁-C₄)-Alkylen-NH₂ worin x die ganze Zahl Null, 1 oder 2 bedeutet, steht,
R3 für
1) -(C₁-C₄)-Alkyl,
2) -(C₀-C₄)-Alkylen-(C₃-C₈)-Cycloalkyl,
3) -(C₁-C₆)-Alkylen-Aryl, wobei Aryl unabhängig voneinander ein-, zwei- oder dreifach durch R15 substituiert ist,
4) -(C₁-C₆)-Alkylen-NH-C(O)-O-(C₁-C₄)-Alkylen-Aryl, wobei Aryl unabhängig voneinander ein-, zwei- oder dreifach durch R15 substituiert ist,
5) -(C₁-C₆)-Alkylen-NH-PG,
6) -(C₁-C₆)-Alkylen-O-PG,
7) -(C₁-C₆)-Alkyl, oder
8) Wasserstoffatom, steht,
wobei PG für t-Butyl-, t-Butyloxycarbonyl oder Benzyloxycarbonyl steht,
R4 für-N(R6)₂, steht,
wobei R6 gleich oder verschieden sind und unabhängig voneinander für
1) Wasserstoffatom,
2) -(C₁-C₆)-Alkyl,
3) -(C₀-C₄)-Alkylen-(C₃-C₁₂)-Cycloalkyl, wobei Cycloalkyl unsubstituiert oder unabhängig voneinander ein-, zwei-, drei- oder vierfach durch R11, Halogen, -C(O)-O-R11, -(C₁-C₄)-Alkyl-O-R11 oder -O-(C₁-C₄)-Alkyl substituiert ist,
4) -(C₀-C₄)-Alkylen-C(R11)(R12)-(C₃-C₁₂)-Cycloalkyl, wobei Cycloalkyl unsubstituiert oder unabhängig voneinander ein-, zwei- oder dreifach durch R11, Halogen, -C(O)-O-R11, -(C₁-C₄)-Alkyl-O-R11 oder -O-(C₁-C₄)-Alkyl substituiert ist,
5) -(C_{O}-C₄)-Alkylen-Het, wobei unter Het ein 4- bis 15-gliedriges heterocyclisches Ringsystem verstanden wird mit 4 bis 15 Ringatomen, die in ein, zwei oder drei miteinander verbundenen Ringsystemen vorliegen und die ein, zwei, drei oder vier gleiche oder verschiedene Heteroatome aus der Reihe Sauerstoff, Stickstoff oder Schwefel enthalten, wobei Het oder Alkylen unsubstituiert oder unabhängig voneinander ein-, zwei- oder dreifach durch R11, Halogen, -C(O)-O-R11, -(C₁-C₄)-Alkyl-O-R11 oder -O-(C₁-C₄)-Alkyl substituiert sind,
6) -(C₀-C₆)-Alkylen-Aryl, wobei Aryl oder Alkylen unsubstituiert oder unabhängig voneinander ein-, zwei- oder dreifach durch R11, Halogen, -C(O)-O-R11, -(C₀-C₄)-Alkyl-O-R11 oder-O-(C₁-C₄)-Alkyl substituiert sind,
7) -(C₀-C₄)-Alkylen-C(R11)(R12)-Aryl, wobei Aryl oder Alkylen unsubstituiert oder unabhängig voneinander ein-, zwei- oder dreifach durch R11, Halogen, -C(O)-O-R11, -(C₀-C₄)-Alkyl-O-R11 oder -O-(C₁-C₄)-Alkyl substituiert sind,
8) 1,2,3,4-Tetrahydro-naphthalenyl,
9) -(C₀-C₄)-Alkylen-CH(R11)-C(O)-NH₂,
10) -(C₀-C₄)-Alkylen-CH(R11)-C(O)-NH-(C₁-C₄)-Alkyl,
11) -(C₀-C₄)-Alkylen-CH(R11)-C(O)-NH-CH(R12)-R13,
12) -(C₀-C₆)-Alkylen-C(O)-O-R11, wobei Alkylen unsubstituiert oder unabhängig voneinander ein- oder zweifach durch R11, Halogen, -C(O)-O-R11, -(C₁-C₄)-Alkyl-O-R11 oder -O-(C₁-C₄)-Alkyl substituiert ist,
13) -(C₀-C₄)-Alkylen-C(R11)(R12)-C(O)-O-R11, oder
14) -(C₁-C₃)-Fluoralkyl, stehen,
oder die beiden Reste R6 zusammen mit dem N-Atom, an das sie gebunden sind, einen mono- oder bicyclischen Ring mit 4 bis 9 Ringatomen bilden, der gesättigt, teilweise gesättigt oder aromatisch ist, wobei der Ring unsubstituiert oder ein- oder zweifach durch -(C₁-C₄)-Alkyl, -C(O)-O-R11, Halogen, -(C₁-C₄)-Alkyl-O-R11 oder Phenyl substituiert ist,
R7 für Wasserstoffatom oder-(C₁-C₄)-Alkyl steht,
R9 für Wasserstoffatom oder -(C₁-C₄)-Alkyl steht,
R11 und R12 gleich oder verschieden sind und unabhängig voneinander für
1) Wasserstoffatom,
2) -(C₁-C₄)-Alkyl,
3) -(C₀-C₄)-Alkylen-Phenyl, wobei Phenyl unsubstituiert oder unabhängig voneinander ein-, zwei- oder dreifach durch Halogen, -OH oder -O-(C₁-C₄)-Alkyl substituiert ist,
4) -(C₀-C₄)-Alkylen-(C₃-C₁₂)-Cycloalkyl, wobei Cycloalkyl unsubstituiert oder unabhängig voneinander ein-, zwei-, drei- oder vierfach durch R13, Halogen, -C(O)-O-R13, -(C₁-C₄)-Alkyl-O-R13, -O-(C₁-C₄)-Alkyl oder -(C_{O}-C₄)-Alkylen-Phenyl substituiert ist,
5) -(C₀-C₄)-Alkylen-C(O)-N(R₁₃)₂ oder
6) -(C₀-C₄)-Alkylen-Indolyl, stehen,
R13 für
1) Wasserstoffatom,
2) -(C₁-C₄)-Alkyl,
3) -(C₀-C₄)-Alkylen-C(O)-O-R14,
4) -(C₀-C₄)-Alkylen-C(O)-R14 oder
5) -(C₀-C₄)-Alkylen-O-R14 steht,
R14 für Wasserstoffatom, -(C₁-C₄)-Alkyl, -NH₂ oder -OH, steht und
R15 für Wasserstoffatom, -(C₁-C₄)-Alkyl, -O-CF₃, -NH₂, -OH, -CF₃ oder Halogen steht.

Die Erfindung betrifft ferner die Verbindung der Formel I, wobei
X für -SO₂- steht,
R1 für
1) Wasserstoffatom oder
2) -(C₁-C₄)-Alkyl, steht,
R2 für
1) -(C₁-C₆)-Alkylen-NH₂,
2) -(C₁-C₄)-Alkylen-Pyridyl-NH₂,
3) -(C₁-C₄)-Alkylen-Piperidinyl-NH₂,
4) -(C₁-C₆)-Alkylen-NH-C(=NH)-NH₂,
5) -(C₀-C₄)-Alkylen-(C₃-C₆)-Cycloalkyl-NH₂,
6) -(C₁-C₆)-Alkylen-NH-C(=NH)-(C₁-C₄)-Alkyl,
7) -(C₁-C₄)-Alkylen-O-NH-C(=NH)-NH₂,
8) -(C₁-C₆)-Alkylen-NH-C(O)-O-(C₁-C₄)-Alkylen-Phenyl, wobei Phenyl unsubstituiert oder durch -NH₂ substituiert ist oder durch -NH₂ und ein-, zwei- oder dreifach durch R15 substituiert ist,
9) -(C₁-C₄)-Alkylen-NH-C(O)-(C₁-C₆)-Alkyl,
10) -(C₁-C₄)-Alkylen-Phenyl, wobei Phenyl unsubstituiert oder durch -NH₂ substituiert ist oder durch -NH₂ und ein-, zwei- oder dreifach durch R15 substituiert ist,
11) -(C₁-C₄)-Alkylen-SO₂-(C₁-C₄)-Alkylen-NH₂, oder
12) -(C₁-C₄)-Alkylen-S-(C₁-C₄)-Alkylen-NH₂, steht,
R3 für
1) -(C₁-C₄)-Alkyl,
2) -(C₁-C₄)-Alkylen-(C₃-C₆)-Cycloalkyl,
3) -(C₁-C₄)-Alkylen-Phenyl, wobei Phenyl unabhängig voneinander ein-, zwei- oder dreifach durch R15 substituiert ist,
4) -(C₁-C₆)-Alkylen-NH-C(O)-O-(C₁-C₄)-Alkylen-Phenyl, wobei Phenyl unabhängig voneinander ein-, zwei- oder dreifach durch R15 substituiert ist,
5) Wasserstoffatom steht,
R4 für -N(R6)₂, steht,
wobei R6 gleich oder verschieden sind und unabhängig voneinander für
1) Wasserstoffatom,
2) -(C₁-C₄)-Alkyl,
3) -(C₀-C₄)-Alkylen-(C₃-C₁₂)-Cycloalkyl, wobei Cycloalkyl ausgewählt ist aus der Gruppe Cyclohexyl, Cyclopentyl, Cyclobutyl, Cyclopropyl, Adamantanyl, 1,7,7-Trimethyl-bicyclo[3.1.1]heptanyl, Decahydro-naphthalenyl, Tetrahydronaphthalenyl, Octahydro-4,7-methano-indenyl oder Bicyclo[2.2.1]heptanyl und worin Cycloalkyl unsubstituiert oder unabhängig voneinander ein-, zwei-, drei- oder vierfach substituiert ist durch -(C₁-C₄)-Alkyl, -C(O)-O-R11 oder-(C₁-C₄)-Alkylen-Phenyl, wobei Phenyl unsubstituiert oder durch Halogen substituiert ist,
4) -(C₀-C₄)-Alkylen-C(R11)(R12)-(C₃-C₁₂)-Cycloalkyl, wobei Cycloalkyl ausgewählt ist aus der Gruppe Cyclohexyl, Cyclopentyl, Cyclobutyl, Cyclopropyl, Adamantanyl, 1,7,7-Trimethyl-bicyclo[3.1.1]heptanyl, Decahydronaphthalen, Tetrahydronaphthalenyl, Octahydro-4,7-methano-indenyl oder Bicyclo[2.2.1]heptanyl und worin Cycloalkyl unsubstituiert oder unabhängig voneinander ein-, zwei-, drei- oder vierfach substituiert ist durch -(C₁-C₄)-Alkyl, -C(O)-O-R11 oder -(C1-C4)-Alkylen-Phenyl, wobei Phenyl unsubstituiert oder durch Halogen substituiert ist,
5) -(C₀-C₄)-Alkylen-Het, wobei Het ausgewählt ist aus der Gruppe Acridinyl, Azepinyl, Azetidinyl, Aziridinyl, Benzimidazalinyl, Benzimidazolyl, Benzo[1,3]dioxolyl, Benzofuranyl, Benzothiofuranyl, Benzothiophenyl, Benzoxazolyl, Benzthiazolyl, Benztriazolyl, Benztetrazolyl, Benzisoxazolyl, Benzisothiazolyl, Carbazolyl, 4aH-Carbazolyl, Carbolinyl, Chinazolinyl, Chinolinyl, 4H-Chinolizinyl, Chinoxalinyl, Chinuclidinyl, Chromanyl, Chromenyl, Cinnolinyl, Deca-hydrochinolinyl, Dibenzofuranyl, Dibenzothiophenyl, Dihydrofuran[2,3-b]-tetrahydrofuranyl, Dihydrofuranyl, Dioxolyl, Dioxanyl, 2H, 6H-1,5,2-Dithiazinyl, Furanyl, Furazanyl, Imidazolidinyl, Imidazolinyl, Imidazolyl, 1H-Indazolyl, Indolinyl, Indolizinyl, Indolyl, 3H-Indolyl, Isobenzofuranyl, Isochromanyl, Isoindazolyl, Isoindolinyl, Isoindolyl, Isochinolinyl (Benzimidazolyl), Isothiazolidinyl, 2-Isothiazolinyl, Isothiazolyl, Isoxazolyl, Isoxazolidinyl, 2-Isoxazolinyl, Morpholinyl, Naphthyridinyl, Octahydroisochinolinyl, Oxadiazolyl, 1,2,3-Oxadiazolyl, 1,2,4-Oxadiazolyl, 1,2,5-Oxadiazolyl, 1,3,4-Oxadiazolyl, Oxazolidinyl, Oxazolyl, Oxazolidinyl, Oxothiolanyl, Pyrimidinyl, Phenanthridinyl, Phenanthrolinyl, Phenazinyl, Phenothiazinyl, Phenoxathiinyl, Phenoxazinyl, Phthalazinyl, Piperazinyl, Piperidinyl, Pteridinyl, Purynyl, Pyranyl, Pyrazinyl, Pyroazolidinyl, Pyrazolinyl, Pyrazolyl, Pyridazinyl, Pryidooxazolyl, Pyridoimidazolyl, Pyridothiazolyl, Pyridothiophenyl, Pyridinyl, Pyridyl, Pyrimidinyl, Pyrrolidinyl, Pyrrolinyl, 2H-Pyrrolyl, Pyrrolyl, Tetrahydrofuranyl, Tetrahydroisochinolinyl, Tetrahydrochinolinyl, Tetrahydropyridinyl, 6H-1,2,5-Thiadazinyl, 1,2,3-Thiadiazolyl, 1,2,4-Thiadiazolyl, 1,2,5-Thiadiazolyl, 1,3,4-Thiadiazolyl, Thianthrenyl, Thiazolyl, Thienyl, Thienoimidazolyl, Thienooxazolyl, Thienopyridin, Thienothiazolyl, Thiomorpholinyl, Thiophenyl, Triazinyl, 1,2,3-Triazolyl, 1,2,3-Triazolyl, 1,2,4-Triazolyl, 1,2,5-Triazolyl, 1,3,4-Triazolyl und Xanthenyl, wobei Het oder Alkylen unsubstituiert oder unabhängig voneinander ein- oder zweifach durch -(C₁-C₄)-Alkyl substituiert ist
6) -(C₁-C₆)-Alkylen-Phenyl, wobei Phenyl oder Alkylen unsubstituiert oder unabhängig voneinander ein- oder zweifach durch Halogen, Phenyl, -C(O)-O-R11, -(C₁-C₄)-Alkyl-O-R11, -O-(C₁-C₄)-Alkyl oder -(C₁-C₄)-Alkyl substituiert sind,
7) -(C₀-C₄)-Alkylen-C(R11)(R12)-Phenyl, wobei Phenyl unsubstituiert oder unabhängig voneinander ein-, zwei- oder dreifach durch Phenyl oder Fluor substituiert ist,
8) 1,2,3,4-Tetrahydro-naphthalenyl,
9) -(C₀-C₄)-Alkylen-CH(R11)-C(O)-NH₂,
10) -(C₀-C₄)-Alkylen-CH(R11)-C(O)-NH-(C₁-C₄)-Alkyl,
11) -(C₀-C₄)-Alkylen-CH(R11)-C(O)-NH-CH(R12)-R13,
12) -(C₁-C₆)-Alkylen-C(O)-O-R11, wobei Alkylen unsubstituiert oder unabhängig voneinander ein- oder zweifach durch Halogen, Phenyl, -C(O)-O-R11, -(C₁-C₄)-Alkyl-O-R11, -O-(C₁-C₄)-Alkyl oder-(C₁-C₄)-Alkyl substituiert ist,
13) -(C₀-C₄)-Alkylen-C(R11)(R12)-C(O)-O-R11, oder
14) -(C₁-C₃)-Fluoralkyl, stehen,
oder die beiden Reste R6 zusammen mit dem N-Atom an das sie gebunden sind einen Mono- oder Bicyclischen Ring bilden ausgewählt aus der Gruppe Pyrrolidin, Piperidin, 2-Aza-bicyclo[3.2.2]nonan und 7-Aza-bicyclo[2.2.1]hep-tan, wobei der Ring unsubstituiert oder ein- oder zweifach durch -(C₁-C₄)-Alkyl, -C(O)-O-R11, -(C₁-C₄)-Alkyl-O-R11 oder Phenyl substituiert ist,
R7 für Wasserstoffatom oder -(C₁-C₄)-Alkyl steht,
R9 für Wasserstoffatom oder -(C₁-C₄)-Alkyl steht,
R11 und R12 gleich oder verschieden sind und unabhängig voneinander für
1) Wasserstoffatom,
2) -(C₁-C₄)-Alkyl,
3) -(C₀-C₄)-Alkylen-Phenyl, wobei Phenyl unsubstituiert oder unabhängig voneinander ein-, zwei- oder dreifach durch -OH, Halogen oder -O-(C₁-C₄)-Alkyl substituiert ist,
4) -(C₀-C₄)-Alkylen-(C₃-C₁₂)-Cycloalkyl, wobei Cycloalkyl ausgewählt ist aus der Gruppe Cyclohexyl, Cyclopentyl, Cyclobutyl, Cyclopropyl, Adamantanyl, 1,7,7-Trimethyl-bicyclo[3.1.1]heptanyl, Decahydro-naphthalenyl, Tetrahydronaphthalenyl, Octahydro-4,7-methano-indenyl oder Bicyclo[2.2.1]heptanyl und worin Cycloalkyl unsubstituiert oder unabhängig voneinander ein-, zwei-, drei- oder vierfach durch -(C₁-C₄)-Alkyl, -C(O)-O-R13 oder Phenyl substituiert ist, oder
5) -(C₀-C₄)-Alkylen-Indolyl, stehen,
R13 für
1) Wasserstoffatom,
2) -(C₁-C₄)-Alkyl,
3) -(C₀-C₄)-Alkylen-C(O)-O-R14,
4) -(C₀-C₄)-Alkylen-C(O)-R14 oder
5) -(C₀-C₄)-Alkylen-O-R14 steht, und
R14 für Wasserstoffatom, -(C₁-C₄)-Alkyl, -NH₂ oder -OH steht und
R15 für Wasserstoffatom, -(C₁-C₄)-Alkyl, -O-CF₃, -NH₂, -OH, -CF₃ oder Halogen steht. Die Erfindung betrifft ferner die Verbindung der Formel I, wobei
X für -SO₂- steht,
R1 für
1) Wasserstoffatom oder
2) -(C₁-C₄)-Alkyl, steht,
R2 für
1) -(C₁-C₆)-Alkylen-NH₂,
2) -(C₁-C₄)-Alkylen-Pyridyl-NH₂,
3) -(C₁-C₄)-Alkylen-Piperidinyl-NH₂,
4) -(C₁-C₄)-Alkylen-NH-C(=NH)-NH₂,
5) -(C₁-C₆)-Alkylen-NH-C(=NH)-(C₁-C₄)-Alkyl,
6) -(C₁-C₄)-Alkylen-(C₃-C₆)-Cycloalkyl-NH₂,
7) -(C₁-C₄)-Alkylen-O-NH-C(=NH)-NH₂,
8) -(C₁-C₆)-Alkylen-NH-C(O)-O-(C₁-C₄)-Alkylen-Phenyl,
9) -(C₁-C₄)-Alkylen-NH-C(O)-(C₁-C₆)-Alkyl,
10) -(C₁-C₄)-Alkylen-Phenyl-NH₂,
11) -(C₁-C₄)-Alkylen-SO₂-(C₁-C₄)-Alkylen-NH₂, oder
12) -(C₁-C₄)-Alkylen-S-(C₁-C₄)-Alkylen-NH₂, steht,
R3 für
1) -(C₁-C₄)-Alkyl,
2) -(C₁-C₄)-Alkylen-(C₃-C₆)-Cycloalkyl,
3) -(C₁-C₄)-Alkylen-Phenyl, wobei Phenyl unsubstituiert oder durch -OH substituiert ist,
4) -(C₁-C₆)-Alkylen-NH-C(O)-O-(C₁-C₄)-Alkylen-Phenyl,
5) Wasserstoffatom steht,
R4 für-N(R6)₂, steht,
wobei R6 gleich oder verschieden sind und unabhängig voneinander für
1) Wasserstoffatom,
2) -(C₁-C₆)-Alkyl,
3) -(C₀-C₄)-Alkylen-(C₃-C₈)-Cycloalkyl, wobei Cycloalkyl ausgewählt ist aus der Gruppe Cyclohexyl, Cyclopentyl, Cyclopropyl, Adamantanyl, 1,7,7-Trimethyl-bicyclo[3.1.1]heptanyl, Decahydro-naphthalen, Octahydro-4,7-methano-indenyl oder Bicyclo[2.2.1]heptanyl und worin Cycloalkyl unsubstituiert oder unabhängig voneinander ein-, zwei- oder dreifach durch -(C₁-C₄)-Alkyl oder Phenyl substituiert ist,
4) -C(R11)(R12)-Adamantanyl,
5) -CH(R11)-C(O)-NH-CH(R12)-R13,
6) -(C₀-C₄)-Alkylen-Het, wobei Het ausgewählt ist aus der Gruppe Benzimidazolyl, Isoxazolyl, Piperidin, Pyridin, Pyrrolidinyl, Thiophenyl und Benzo[1,3]dioxol,
7) 1,2,3,4-Tetrahydro-naphthalenyl,
8) -(C₀-C₄)-Alkylen-C(R11)(R12)-Phenyl, wobei Phenyl unsubstituiert oder unabhängig voneinander ein-, zwei- oder dreifach durch Phenyl oder Fluor substituiert ist,
9) -CH(R11)-C(O)-NH₂,
10) -CH(R11)-C(O)-NH-CH(R12)-CH₂-OH,
11) -(C₁-C₆)-Alkylen-Phenyl, wobei Phenyl oder Alkylen unsubstituiert oder unabhängig voneinander ein- oder zweifach durch Chlor, Fluor, -C(O)-O-R11, -(C₁-C₄)-Alkyl-O-R11, -O-(C₁-C₄)-Alkyl, Phenyl oder -(C₁-C₄)-Alkyl substituiert sind,
12) -CH(R11)-C(O)-NH-(C₁-C₄)-Alkyl,
13) -(C₀-C₄)-Alkylen-C(R11)(R12)-Bicyclo[3.1.1]heptanyl, wobei Bicyclo[3.1.1]heptanyl unsubstituiert oder ein- bis vierfach durch -(C₁-C₄)-Alkyl substituiert ist,
14) -(C₁-C₆)-Alkylen-C(O)-O-R11, wobei Alkylen unsubstituiert oder unabhängig voneinander ein- oder zweifach durch Chlor, Fluor, -C(O)-O-R11, - (C₁-C₄)-Alkyl-O-R11, -O-(C₁-C₄)-Alkyl, Phenyl oder -(C₁-C₄)-Alkyl substituiert ist,
15) -(C₀-C₄)-Alkylen-C(R11)(R12)-C(O)-O-R11, oder
16) -CH₂-CF₂-CF₃, stehen,
oder die beiden Reste R6 zusammen mit dem N-Atom an das sie gebunden sind einen Mono- oder Bicyclischen Ring bilden, ausgewählt aus der Gruppe Pyrrolidine, 2-aza-bicyclo[3.2.2]nonan und 7-aza-bicyclo[2.2.1]heptan, wobei der Ring unsubstituiert oder ein- oder zweifach durch -(C₁-C₄)-Alkyl, -C(O)-O-R11, -(C₁-C₄)-Alkyl-O-R11 oder Phenyl substituiert ist,
R7 für Wasserstoffatom oder -(C1-C4₎-Alkyl steht,
R9 für Wasserstoffatom oder -(C₁-C₄)-Alkyl steht,
R11 und R12 gleich oder verschieden sind und unabhängig voneinander für
1) Wasserstoffatom,
2) -(C₁-C₄)-Alkyl,
3) -(C₀-C₄)-Alkylen-Phenyl, wobei Phenyl unsubstituiert oder unabhängig voneinander ein-, zwei- oder dreifach durch -OH, Halogen oder -O-(C₁-C₄)-Alkyl substituiert ist,
4) -(C₀-C₄)-Alkylen-(C₃-C₁₂)-Cycloalkyl, wobei Cycloalkyl ausgewählt ist aus der Gruppe Cyclohexyl, Cyclopentyl, Cyclobutyl, Cyclopropyl, Adamantanyl, 1,7,7-Trimethyl-bicyclo[3.1.1]heptanyl, Decahydro-naphthalenyl, Octahydro-4,7-methano-indenyl oder Bicyclo[2.2.1]heptanyl und worin Cycloalkyl unsubstituiert oder unabhängig voneinander ein-, zwei-, drei- oder vierfach durch -(C₁-C₄)-Alkyl, -C(O)-O-R13 oder Phenyl substituiert ist, oder
5) -(C₀-C₄)-Alkylen-Indolyl, stehen,
R13 für
1) Wasserstoffatom,
2) -(C₁-C₄)-Alkyl,
3) -(C₀-C₄)-Alkylen-C(O)-O-R14,
4) -(C₀-C₄)-Alkylen-C(O)-R14 oder
5) -(C₀-C₄)-Alkylen-O-R14 steht,
R14 für Wasserstoffatom, -(C₁-C₄)-Alkyl, -NH₂ oder -OH steht und
R15 für Wasserstoffatom, -(C₁-C₄)-Alkyl, -O-CF₃, -NH₂, -OH, -CF₃ oder Halogen steht.

Die Erfindung betrifft auch die Verwendung der Verbindung der Formel I, zur Herstellung eines Arzneimittels zur Prophylaxe, Sekundärprevention und Therapie einer oder mehrerer Erkrankungen, die mit Thrombosen, Embolien, Hyperkoagulabilität oder fibrotischen Veränderungen einhergehen ausgewählt aus der Reihe Myokardinfarkt, Angina pectoris und andere Formen des akuten Koronarsyndroms, den Schlaganfall, die peripher vaskulären Erkrankungen, die tiefe Venenthrombose, die Lungenembolie, embolische oder thrombotische Ereignisse bedingt durch kardiale Arrhythmien, kardiovaskuläre Ereignisse wie Restenose nach Revaskularisierung und Angioplastie und ähnlichen Eingriffen wie Stentimplantationen und Bypass-Operationen handelt, oder die Reduktion der Thrombosegefahr nach chirurgischen Eingriffen wie bei Knie- und Hüftgelenksoperationen, oder um die disseminierte intravaskuläre Koagulation, Sepsis und anderen intravaskulären Ereignissen, die mit einer Entzündung einhergehen, Atherosklerose, Diabetes und dem metabolischen Syndrom und deren Folgen, Tumorwachstum und Tumormetastasierung, entzündliche und degenerative Gelenkserkrankungen wie der rheumatoiden Arthritis und der Arthrose, Störungen des hämostatischen Systems wie Fibrinablagerungen, fibrotische Veränderungen der Lunge wie die chronische obstruktive Lungenerkrankung, das adult respiratory distress syndrom oder Fibrinablagerungen des Auges nach Augenoperationen oder Verhinderung oder Behandlung von Narbenbildung handelt.

Unter dem Begriff "(C₁-C₆)-Alkyl" werden Kohlenwasserstoffreste verstanden, deren Kohlenstoffkette geradkettig oder verzweigt ist und 1 bis 6 Kohlenstoffatome enthält, beispielsweise Methyl, Ethyl, Propyl, Iso-Propyl, Butyl, Iso-Butyl, tertiär-Butyl, Pentyl, Iso-Pentyl, Neopentyl, Hexyl, 2,3-Dimethylbutan oder Neohexyl.

Unter dem Begriff "-(C₀₋C₄)-Alkylen" werden Kohlenwasserstoffreste verstanden, deren Kohlenstoffkette geradkettig oder verzweigt ist und 1 bis 4 Kohlenstoffatome enthält, beispielsweise Methylen, Ethylen, Propylen, Iso-Propylen, Iso-Butylen, Butylen oder tertiär-Butylen. "-C₀-Alkylen" ist eine kovalente Bindung.

Unter dem Begriff "-(CH₂)ₙ-, worin n die ganze Zahl Null oder 1 bedeutet" wird für den Fall n gleich 1 der Rest Methylen verstanden und für den Fall das n die ganze Zahl Null bedeutet, hat der Rest die Bedeutung einer kovalenten Bindung.

Unter dem Begriff "-(C₁-C₄)-Alkylen" werden Kohlenwasserstoffreste verstanden, deren Kohlenstoffkette geradkettig oder verzweigt ist und 1 bis 4 Kohlenstoffatome enthält, beispielsweise Methylen (-CH₂-), Ethylen (-CH₂-CH₂-), Propylen (-CH₂-CH₂-CH₂-), Iso-Propylen, Iso-Butylen, Butylen oder tertiär-Butylen.

Unter dem Begriff "-(CH₂)ₙ-, worin n die ganze Zahl Null, 1, 2 oder 3 bedeutet" werden Reste wie Methylen, Ethylen oder Propylen verstanden. Für den Fall das n die ganze Zahl Null bedeutet, hat der Rest die Bedeutung einer kovalenten Bindung.

Unter dem Begriff "(C₃-C₁₂)-Cycloalkyl" werden Reste verstanden wie Verbindungen, die sich von 3- bis 12-gliedrige Mono-, Bi- oder Tricyclen oder überbrücken Cyclen wie den Monocyclen Cyclopropan, Cyclobutan, Cyclopentan, Cyclohexan, Cycloheptan oder Cyclooctan herleiten, die sich von den Bicyclen Bicyclo[4.2.0]octan, Octahydroinden, Decahydro-naphthalen, Decahydro-azulen, Decahydro-benzocyclohepten oder Dodecahydro-heptalen, oder von Tricyclen wie Adamantan herleiten oder von den überbrücken Cyclen wie Spiro[2.5]octan, Spiro[3.4]octan, Spiro[3.5]nonan, Bicyclo[3.1.1]heptan, Bicyclo[2.2.1]heptan, Bicyclo[2.2.2]octan oder Octahydro-4,7-methan-inden herleiten.

Unter dem Begriff "R6 zusammen mit dem N-Atom an das sie gebunden sind einen Mono- oder Bicyclischen Ring mit 4 bis 9 Ringatomen bilden" werden Reste verstanden wie Verbindungen, die sich von 4- bis 8-gliedrige Monocyclen ableiten die gestättigt oder ganz oder teilweise aromatisch sein können zum Beispiel Azetidin, Dihydroazet, Azet, Diazetidin, Diazet, Pyrrolidin, Dihydropyrrol, Pyrrol, Imidazolidin, Dihydroimidazol, Imidazol, Pyrazolin, Pyrazolidin, Piperidin, Dihydropyridin, Tetrahydropyridin, Pyridin, Piperazin, Dihydropyrazin, Pyrazin, Pyridazin, Pyrimidin, Oxazin, Azepan, Tetrahydroazepin, Azepin, Azocan, Dihydroazocin, Hexohydroazocin oder Azocin oder bicyclische Ringe wie 2-aza-bicyclo[3.2.2]nonan oder, 7-aza-bicyclo[2.2.1]heptan.

Unter dem Begriff "-(C₆-C₁₄)-Aryl" werden aromatische Kohlenstoffreste verstanden mit 6 bis 14 Kohlenstoffatomen im Ring. -(C₆-C₁₄)-Arylreste sind beispielsweise Phenyl, Naphthyl, zum Beispiel 1-Naphthyl, 2-Naphthyl, 1,2,3,4-Tetrahydronaphthalenyl, Anthryl oder Fluorenyl. Naphthylreste und insbesondere Phenylreste sind bevorzugte Arylreste.

Unter dem Begriff "4- bis 15-gliedriger Het-Ring" oder "Het" werden Ringsysteme verstanden mit 4 bis 15 Kohlenstoffatomen, die in ein, zwei oder drei miteinander verbundenen Ringsystemen vorliegen und die ein, zwei, drei oder vier gleiche oder verschiedene Heteroatome aus der Reihe Sauerstoff, Stickstoff oder Schwefel enthalten. Beispiele für diese Ringsysteme sind die Reste Acridinyl, Azepinyl, Azetidinyl, Aziridinyl, Benzimidazalinyl, Benzimidazolyl, Benzo[1,3]dioxol, Benzofuranyl, Benzothiofuranyl, Benzothiophenyl, Benzoxazolyl, Benzthiazolyl, Benztriazolyl, Benztetrazolyl, Benzisoxazolyl, Benzisothiazolyl, Carbazolyl, 4aH-Carbazolyl, Carbolinyl, Chinazolinyl, Chinolinyl, 4H-Chinolizinyl, Chinoxalinyl, Chinuclidinyl, Chromanyl, Chromenyl, Cinnolinyl, Deca-hydrochinolinyl, Dibenzofuranyl, Dibenzothiophenyl, Dihydrofuran[2,3-b]-tetrahydrofuranyl, Dihydrofuranyl, Dioxolyl, Dioxanyl, 2H, 6H-1,5,2-Dithiazinyl, Furanyl, Furazanyl, Imidazolidinyl, Imidazolinyl, Imidazolyl, 1H-Indazolyl, Indolinyl, Indolizinyl, Indolyl, 3H-Indolyl, Isobenzofuranyl, Isochromanyl, Isoindazolyl, Isoindolinyl, Isoindolyl, Isochinolinyl (Benzimidazolyl), Isothiazolidinyl, 2-Isothiazolinyl, Isothiazolyl, Isoxazolyl, Isoxazolidinyl, 2-Isoxazolinyl, Morpholinyl, Naphthyridinyl, Octahydroisochinolinyl, Oxadiazolyl, 1,2,3-Oxadiazolyl, 1,2,4-Oxadiazolyl, 1,2,5-Oxadiazolyl, 1,3,4-Oxadiazolyl, Oxazolidinyl, Oxazolyl, Oxazolidinyl, Oxothiolanyl, Pyrimidinyl, Phenanthridinyl, Phenanthrolinyl, Phenazinyl, Phenothiazinyl, Phenoxathiinyl, Phenoxazinyl, Phthalazinyl, Piperazinyl, Piperidinyl, Pteridinyl, Purynyl, Pyranyl, Pyrazinyl, Pyroazolidinyl, Pyrazolinyl, Pyrazolyl, Pyridazinyl, Pryidooxazolyl, Pyridoimidazolyl, Pyridothiazolyl, Pyridothiophenyl, Pyridinyl, Pyridyl, Pyrimidinyl, Pyrrolidinyl, Pyrrolinyl, 2H-Pyrrolyl, Pyrrolyl, Tetrahydrofuranyl, Tetrahydroisochinolinyl, Tetrahydrochinolinyl, Tetrahydropyridinyl, 6H-1,2,5-Thiadazinyl, 1,2,3-Thiadiazolyl, 1,2,4-Thiadiazolyl, 1,2,5-Thiadiazolyl, 1,3,4-Thiadiazolyl, Thianthrenyl, Thiazolyl, Thienyl, Thienoimidazolyl, Thienooxazolyl, Thienopyridin, Thienothiazolyl, Thiomorpholinyl, Thiophenyl, Triazinyl, 1,2,3-Triazolyl, 1,2,3-Triazolyl, 1,2,4-Triazolyl, 1,2,5-Triazolyl, 1,3,4-Triazolyl und Xanthenyl.

Bevorzugte Het-Ringe sind die Reste Isoxazolyl, Benzo[1,3]dioxo.l und Thiophenyl. Unter dem Begriff "Halogen" wird Fluor, Chlor, Brom oder Jod verstanden, bevorzugt sind Fluor, Chlor oder Brom, insbesondere Chlor oder Brom.

Unter dem Begriff "Aminosäure" werden Verbindungen wie natürlich vorkommende α-Aminosäuren Glycin, Alanin, Valin, Leucin, Isoleucin, Phenylalanin, Tyrosin, Tryptophan, Serin, Threonin, Cystein, Methionin, Asparagin, Glutamin, Lysin, Histidin, Arginin, Glutaminsäure und Asparaginsäure verstanden. Insbesondere bevorzugt sind Histidin, Tryptophan, Serin, Threonin, Cystein, Methionin, Asparagin, Glutamin, Lysin, Arginin, Glutaminsäure und Asparaginsäure. Ferner gehören dazu auch nicht natürlich vorkommenden Aminosäuren wie 2-Aminoadipinsäure, 2-Aminobuttersäure, 2-Aminoisobuttersäure , 2,3-Diamino-propionsäure, 2,4-Diaminobuttersäure, 1,2,3,4-Tetra-hydroisochinolin-1-carbonsäure, 1,2,3,4-Tetrahydroisochinolin-3-carbonsäure, 2-Amino-pimelinsäure, Phenylglycin, 3-(2-Thienyl)-alanin, 3-(3-Thienyl)-alanin, Sarkosin, 2-(2-Thienyl)-glycin, 2-Amino-heptansäure, Pipecolinsäure, Hydroxylysin, N-Methylisoleucin, 6-N-Methyllysin, N-Methylvalin, Norvalin, Norleucin, Ornithin, allo-Isoleucin, 4-Hydroxy-prolin, allo-Hydroxylysin, allo-Threonin, 3-Hydroxyprolin, 3-(2-Naphtyl)-alanin, 3-(1-Naphtylalanin), Homophenylalanin, Homocystein, 2-Amino-3-phenylamirioethyl-propion-säure, Homocysteinsäure, Homotryptophan, Cysteinsäure, 3-(2-Pyridyl)-alanin, 3-(3-Pyridyl)-alanin, 3-(4-Pyridyl)-alanin, Phosphinothricin, 4-Fluorphenyl-alanin, 3-Fluor-phenylalanin, 4-Fluorphenylalanin, 3-Fluorphenylalanin, 3-Fluorphenylalanin, 2-Fluorphenylalanin, 4-Chlorphenylalanin, 4-Nitrophenylalanin, 4-Aminophenylalanin, Cyclo-hexylalanin, Citrullin, 5-Fluortryptophan, 5-Methoxytryptophan oder 2-Amino-3-phenylamino-propionsäure verstanden.

Unter dem Begriff "Peptid-Bindung" werden Strukturen wie 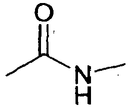 verstanden.

Unter dem Begriff "Schutzgruppe für die Amino-, Carboxyl- oder für die Hydroxyfunktion" werden Schutzgruppen verstanden wie geeignete Schutzgruppen für Aminofunktionen, beispielweise die t-Butoxycarbonyl-, die Benzyloxycarbonyl- oder die Phtalolylgruppe sowie die Trityl- oder Tosylschutzgruppe, geeignete Schutzgruppen für die Carboxylfunktion sind beispielweise Alkyl-, Aryl- oder Arylalkylester und geeignete Schutzgruppen für die Hydroxyfunktion sind beispielsweise Alkylester, t-Butyl-, Benzyl- oder Tritylgruppen. Schutzgruppen können durch wohlbekannte oder hier beschriebene Techniken eingeführt und entfernt werden (siehe Green, T.W., Wutz, P.G.M., Protective Groups in Organic Synthesis (1991), 2nd Ed., Wiley-Interscience, oder Kocienski, P., Protecting Groups (1994), Thieme).

Unter dem Begriff "-(C₁-C₃)-Fluoralkyl" wird ein partiell oder vollständig fluorierter Alkylrest verstanden, der sich beispielsweise von folgenden Resten ableitet -CF₃, -CHF₂, -CH₂F, -CHF-CF₃, -CHF-CHF₂, -CHF-CH₂F, -CH₂-CF₃, -CH₂-CHF₂, -CH₂-CH₂F, -CF₂-CF₃, -CF₂-CHF₂, -CF₂-CH₂F, -CH₂-CHF-CF₃, -CH₂-CHF-CHF₂, -CH₂-CHF-CH₂F, -CH₂-CH₂-CF₃, -CH₂-CH₂-CHF₂, CH₂-CH₂-CH₂F, -CH₂-CF₂-CF₃, -CH₂-CF₂-CHF₂, -CH₂-CF₂-CH₂F, -CHF-CHF-CF₃, -CHF-CHF-CHF₂, -CHF-CHF-CH₂F, -CHF-CH₂-CF₃, -CHF-CH₂-CHF₂, -CHF-CH₂-CH₂F, -CHF-CF₂-CF₃, -CHF-CF₂-CHF₂, -CHF-CF₂-CH₂F, -CF₂-CHF-CF₃, -CF₂-CHF-CHF₂, -CF₂-CHF-CH₂F, -CF₂-CH₂-CF₃, -CF₂-CH₂-CHF₂, -CF₂-CH₂-CH₂F, -CF₂-CF₂-CF₃, -CF₂-CF₂-CHF₂ oder -CF₂-CF₂-CH₂F.

Unter dem Begriff "-SO₂-" wird ein Sulfonyl-Rest verstanden.

Unter dem Begriff "-C(O)-" wird ein Carbonyl-Rest verstanden.

Die Erfindung betrifft ferner ein Verfahren zur Herstellung der Verbindung der Formel I, das dadurch gekennzeichnet, dass man
a) eine Verbindung der Formel II 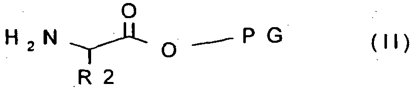 wobei R2 und PG die in der Verbindung der Formel I genannten Bedeutungen haben, mit einer Verbindung der Formel IX, 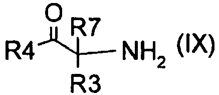 wobei R3, R4, R7 und PG die in der Verbindung der Formel I genannten Bedeutungen haben, zu einer Verbindung der Formel X umgesetzt, 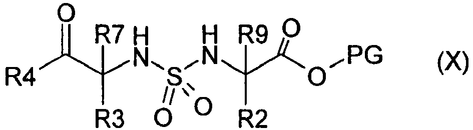 wobei R2, R3, R4, R7, R9 und PG die in der Verbindung der Formel I genannten Bedeutungen haben, und dann in eine Verbindung der Formel I überführt, oder
b) eine nach den Verfahren a) hergestellte Verbindung der Formel I, oder eine geeignete Vorstufe der Formel I, die aufgrund ihrer chemischen Struktur in enantiomeren Formen auftritt, durch Salzbildung mit enantiomerenreinen Säuren oder Basen, Chromatographie an chiralen Stationärphasen oder Derivatisierung mittels chiraler enantiomerenreinen Verbindungen wie Aminosäuren, Trennung der somit erhaltenen Diastereomeren, und Abspaltung der chiralen Hilfsgruppen in die reinen Enantiomeren auftrennt, oder
c) die nach den Verfahren a) oder b) hergestellte Verbindung der Formel I entweder in freier Form isoliert oder im Falle des Vorliegens von sauren oder basischen Gruppen in physiologisch verträgliche Salze umwandelt.

Ein weiteres Verfahren zur Herstellung der erfindungsgemäßen Verbindungen gemäß (I) ist die Umsetzung der Verbindungen (IX) mit Verbindungen vom Typ (II) analog zu A. In einem Verfahren gemäß Borghese et al. (Org. Process Res. Dev. 2006, 10, 770-775) werden Verbindungen der Formel X hergestellt, welche anschließend entschützt werden und Verbindungen der Formel I ergeben:

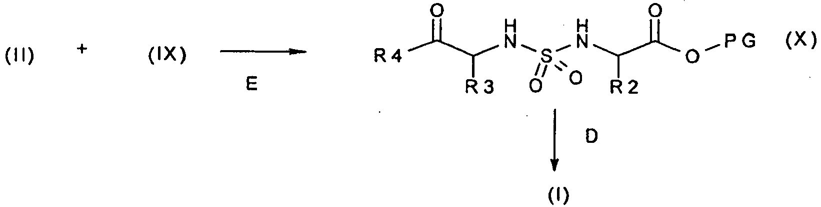

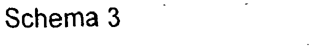

Unter den Aminen der Formel NH(R6)₂ werden kommerziell erhältliche oder nach literaturbekannten Verfahren hergestellte Amine oder Dipeptidderivate verstanden. Die Verbindungen (II) sind kommerziell erhältlich oder können durch Alkylierung von (Benzhydryliden-amino)-essigsäure-tert-butylester in geeigneten Lösungsmittel wie THF oder DMF unter Einwirkung von Basen wie Lithiumhexa-methyldisilazan, KOH, NaOH, CsOH, K₂CO₃ oder NaH und anschließender Entschützung unter sauren Bedingungen, beispielsweise in verdünnter Salzsäure oder wässriger Citronensäure erhalten werden (Schema 4, vgl. z.B. J. Ezquerra et al., Tetrahedron Lett. 1993, 34 (52), 8535-8538). Die Verbindungen (XI) sind kommerziell erhältlich oder literaturbekannt, wobei X eine geeignete Abgangsgruppe wie Brom, Iod, Chlor, Tosylat oder Mesylat darstellt.

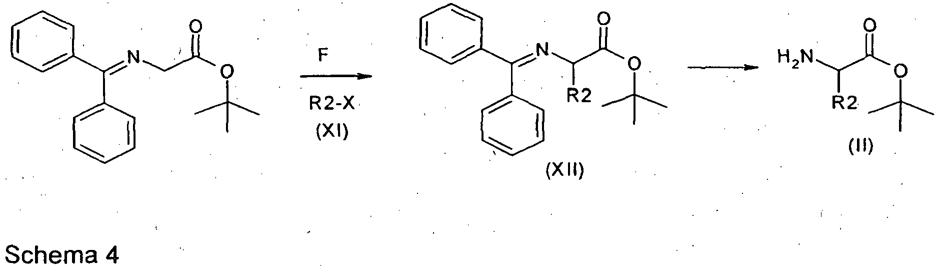

Eine nach Schema 1 oder 3 hergestellte Verbindung der Formel I, oder eine geeignete Vorstufe der Formel I; die aufgrund ihrer chemischen Struktur in enantiomeren Formen auftritt, kann durch Salzbildung mit enantiomerenreinen Säuren oder Basen, Chromatographie an chiralen Stationärphasen oder Derivatisierung mittels chiraler enantiomerenreinen Verbindungen wie Aminosäuren, Trennung der somit erhaltenen Diastereomeren, und Abspaltung der chiralen Hilfsgruppen in die reinen Enantiomeren aufgetrennt werden (Verfahren b), oder
die nach Schema 1 oder hergestellte Verbindung der Formel I kann entweder in freier Form isoliert oder im Falle des Vorliegens von sauren oder basischen Gruppen in physiologisch verträgliche Salze umwandelt werden (Verfahren d).

Im Verfahrensschritt c) wird die Verbindung der Formel I, sofern sie als Gemisch von Diastereomeren oder Enantiomeren auftritt oder bei der gewählten Synthese als deren Gemische anfällt, in die reinen Stereoisomeren getrennt, entweder durch Chromatographie an einem gegebenenfalls chiralen Trägermaterial, oder, sofern die racemische Verbindung der Formel I zur Salzbildung befähigt ist, durch fraktionierte Kristallisation der mit einer optisch aktiven Base oder Säure als Hilfsstoff gebildeten diastereomeren Salze. Als chirale Stationärphasen für die dünnschicht- oder säulenchromatographische Trennung von Enantiomeren eignen sich zum Beispiel modifizierte Kieselgelträger (sogenannte Pirkle-Phasen) sowie hochmolekulare Kohlenhydrate wie Triacetylcellulose. Für analytische Zwecke sind nach entsprechender, dem Fachmann bekannter Derivatisierung, auch gaschromatographische Methoden an chiralen Stationärphasen anwendbar. Zur Enantiomerentrennung der racemischen Carbonsäuren werden mit einer optisch aktiven, in der Regel kommerziell erhältlichen Base wie (-)-Nicotin, (+)- und (-)-Phenylethylamin, Chininbasen, L-Lysin oder L-und D-Arginin die unterschiedlich löslichen diastereomeren Salze gebildet, die schwerer lösliche Komponente als Feststoff isoliert, das leichter lösliche Diastereomer aus der Mutterlauge abgeschieden und aus den so gewonnenen diastereomeren Salzen die reinen Enantiomeren gewonnen. Auf prinzipiell gleiche Weise kann man die racemischen Verbindungen der Formel I, die eine basische Gruppe wie eine Aminogruppe enthalten, mit optisch aktiven Säuren, wie (+)-Campher-10-sulfonsäure, D- und L- Weinsäure, D-und L-Milchsäure sowie (+) und (-)-Mandelsäure in die reinen Enantiomeren überführen. Auch kann man chirale Verbindungen, die Alkohol- oder Amin-funktionen enthalten, mit entsprechend aktivierten oder gegebenenfalls N-geschützten enantiomerenreinen Aminosäuren in die entsprechenden Ester oder Amide, oder umgekehrt chirale Carbonsäuren mit carboxygeschützten enantiomerenreinen Aminosäuren in die Amide oder mit enantiomerenreinen Hydroxycarbonsäuren wie Milchsäure, in die entsprechenden chiralen Ester überführen. Sodann kann die Chiralität des in enantiomerenreiner Form eingebrachten Aminosäure- oder Alkoholrestes zur Trennung der Isomeren genutzt werden, indem man eine Trennung der nunmehr vorliegenden Diastereomeren durch Kristallisation oder Chromatographie an geeigneten Stationärphasen vornimmt und danach den mitgeführte chiralen Molekülteil mittels geeigneter Methoden wieder abspaltet. ,

Weiterhin ergibt sich bei einigen der erfindungsgemäßen Verbindungen die Möglichkeit, zur Herstellung der Gerüststrukturen diastereo- oder enantiomerenreine Ausgangsprodukte einzusetzen. Dadurch können auch andere oder vereinfachte Verfahren zur Aufreinigung der Endprodukte eingesetzt werden. Diese Ausgangsprodukte wurden zuvor nach literatur-bekannten Verfahren enantiomeren- oder diastereomerenrein hergestellt. Das kann insbesondere bedeuten, dass in der Synthese der Grundgerüste entweder enantioselektive Verfahren zum Einsatz kommen, oder aber eine Enantiomeren- (oder Diastereomeren-) Trennung auf früher Synthesestufe und nicht erst auf der Stufe der Endprodukte durchgeführt wird. Ebenso kann eine Vereinfachung der Trennungen dadurch erreicht werden, dass zwei- oder mehrstufig vorgegangen wird.

Saure oder basische Produkte der Verbindung der Formel I können in Form ihrer Salze oder in freier Form vorliegen. Bevorzugt sind pharmakologisch verträgliche Salze, beispielsweise Alkali- oder Erdalkalimetallsalze wie Hydrochloride, Hydrobromide, Sulfate, Hemisulfate, alle möglichen Phosphate sowie Salze der Aminosäuren, natürlicher Basen oder Carbonsäuren.
Die Herstellung physiologisch verträglicher Salze aus zur Salzbildung befähigten Verbindungen der Formel I, einschließlich deren stereoisomeren Formen, gemäß Verfahrensschritt c) erfolgt in an sich bekannter Weise. Die Verbindungen der Formel I bilden mit basischen Reagenzien wie Hydroxiden, Carbonaten, Hydrogencarbonaten, Alkoholaten sowie Ammoniak oder organischen Basen, beispielsweise Trimethyl- oder Triethylamin, Ethanolamin, Diethanolamin oder Triethanolamin, Trometamol oder auch basischen Aminosäuren, etwa Lysin, Ornithin oder Arginin, stabile Alkali-, Erdalkali- oder gegebenenfalls substituierte Ammoniumsalze. Sofern die Verbindungen der Formel I basische Gruppen aufweisen, lassen sich mit starken Säuren auch stabile Säureadditionssalze herstellen. Hierfür kommen sowohl anorganische als auch organische Säuren wie Chlorwasserstoff-, Bromwasserstoff-, Schwefel-, Hemischwefel-, Phosphor-, Methansulfon-, Benzolsulfon-, p-Toluolsulfon-, 4-Brombenzol-sulfon-, Cyclohexylamidosulfon-, Trifluormethylsulfon-, 2-Hydroxyethansulfon-, Essig-, Oxal-, Wein-, Bernstein-, Glycerolphosphor-, Milch-, Äpfel-, Adipin-, Citronen-, Fumar-, Malein-, Glucon-, Glucuron- Palmitin-, oder Trifluoressigsäure in Frage.

Die Erfindung betrifft auch Arzneimittel, gekennzeichnet durch einen wirksamen Gehalt an mindestens einer Verbindung der Formel I und/oder eines physiologisch verträglichen Salzes der Verbindung der Formel I und/oder eine gegebenenfalls stereoisomeren Form der Verbindung der Formel I, zusammen mit einem pharmazeutisch geeigneten und physiologisch verträglichen Trägerstoff, Zusatzstoff und/oder anderen Wirk- und Hilfsstoffen.

Aufgrund der pharmakologischen Eigenschaften eignen sich die erfindungsgemäßen Verbindungen zur Prophylaxe, Sekundär-Prevention und Therapie all solcher Erkrankungen, die durch eine Hemmung von TAFla behandelbar sind. So eignen sich TAFla Inhibitoren sowohl für eine prophylaktische als auch für eine therapeutische Anwendung am Menschen. Sie eignen sich sowohl für eine akute Behandlung als auch für eine Langzeittherapie. TAFla Inhibitoren können eingesetzt werden in Patienten, die an Störungen des Wohlbefindens oder Krankheiten leiden, die mit Thrombosen, Embolien, Hyperkoagulabilität oder fibrotischen Veränderungen einhergehen.
Dazu gehören der Myokardinfarkt, die Angina pectoris und alle anderen Formen des akuten Koronarsyndroms, der Schlaganfall, die peripher vaskulären Erkrankungen, die tiefe Venenthrombose, die Lungenembolie, embolische oder thrombotische Ereignisse bedingt durch kardiale Arrhythmien, kardiovaskuläre Ereignisse wie Restenose nach Revaskularisierung, Angioplastie und ähnlichen Eingriffen wie Stentimplantationen und Bypass-Operationen. Weiterhin können TAFla Inhibitoren eingesetzt werden bei allen Eingriffen, die zu einem Kontakt des Blutes mit Fremdoberflächen führen wie z. B. bei Dialysepatienten und Patienten mit Verweilkathetern. TAFla Inhibitoren können eingesetzt werden, um die Thrombosegefahr nach chirurgischen Eingriffen wie bei Knie- und Hüftgelernksoperationen zu reduzieren.

TAFIa Inhibitoren eignen für die Behandlung von Patienten mit disseminierter intravaskulärer Koagulation, Sepsis und anderen intravaskulären Ereignissen, die mit einer Entzündung einhergehen. Weiterhin eignen sich TAFla Inhibitoren für die Prophylaxe und Behandlung von Patienten mit Atherosklerose, Diabetes und dem metabolischen Syndrom und deren Folgen: Störungen des hämostatischen Systems (beispielsweise Fibrinablagerungen) wurden impliziert in Mechanismen, die zu Tumorwachstum und Tumormetastasierung führen, sowie bei entzündlichen und degenerativen Gelenkserkrankungen wie der rheumatoiden Arthritis und der Arthrose. TAFla Inhibitoren eignen sich zur Verlangsamung oder Verhinderung solcher Prozesse.

Weitere Indikationen für den Einsatz von TAFla Inhibitoren sind fibrotische Veränderungen der Lunge wie die chronische obstruktive Lungenerkrankung, das adult respiratory distress syndrome (ARDS) und des Auges wie Fibrinablagerungen nach Augenoperationen. TAFla Inhibitoren eignen sich auch zur Verhinderung und/oder Behandlung von Narbenbildung.

Die Applikation der erfindungsgemäßen Arzneimittel kann durch orale, inhalative, rektale oder transdermale Applikation oder durch subkutane, intraartikuläre, intraperitoneale oder intravenöse Injektion erfolgen. Bevorzugt ist die orale Applikation. Eine Beschichtung mit TAFla Inhibitoren von Stents und anderen Oberflächen, die im Körper mit Blut in Kontakt kommen, ist möglich.

Die Erfindung betrifft auch ein Verfahren zur Herstellung eines Arzneimittels, das dadurch gekennzeichnet, dass man mindestens eine Verbindung der Formel I mit einem pharmazeutisch geeigneten und physiologisch verträglichen Träger und gegebenenfalls weiteren geeigneten Wirk-, Zusatz- oder Hilfsstoffen in eine geeignete Darreichungsform bringt.

Geeignete feste oder galenische Zubereitungsformen sind beispielsweise Granulate, Pulver, Dragees, Tabletten, (Mikro)Kapseln, Suppositorien, Sirupe, Säfte, Suspensionen, Emulsionen, Tropfen oder injizierbare Lösungen sowie Präparate mit protrahierter Wirkstoff-Freigabe, bei deren Herstellung übliche Hilfsmittel wie Trägerstoffe, Spreng-, Binde-, Überzugs-, Quellungs-, Gleit- oder Schmiermittel, Geschmacksstoffe, Süßungsmittel und Lösungsvermittler Verwendung finden. Als häufig verwendete Hilfsstoffe seien Magnesiumcarbonat, Titandioxid, Laktose, Mannit und andere Zucker, Talkum, Milcheiweiß, Gelatine, Stärke, Cellulose und ihre Derivate, tierische und pflanzliche Öle wie Lebertran, Sonnenblumen-, Erdnuss- oder Sesamöl, Polyethylenglykol und Lösungsmittel wie etwa steriles Wasser und ein- oder mehrwertige Alkohole wie Glycerin, genannt.

Vorzugsweise werden die pharmazeutischen Präparate in Dosierungseinheiten hergestellt und verabreicht, wobei jede Einheit als aktiven Bestandteil eine bestimmte Dosis der erfindungsgemäßen Verbindung der Formel I enthält. Bei festen Dosierungseinheiten wie Tabletten, Kapseln, Dragees oder Suppositorien, kann diese Dosis bis zu 1000 mg, bevorzugt jedoch 50 bis 300 mg und bei Injektionslösungen in Ampullenform bis zu 300 mg, vorzugsweise aber 10 bis 100 mg, betragen.

Für die Behandlung eines erwachsenen, etwa 70 kg schweren Patienten sind je nach Wirksamkeit der Verbindung gemäß Formel I, Tagesdosen von 2 mg bis 1000 mg Wirkstoff, bevorzugt 50 mg bis 500 mg indiziert. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrerer kleinerer Dosierungseinheiten als auch durch Mehrfachgabe unterteilter Dosen in bestimmten Intervallen erfolgen.

TAFla Inhibitoren können sowohl als Monotherapie als auch in Kombination oder gemeinsam mit allen Antithrombotika (Antikoagulanzien und Plättchenaggregationshemmer), Thrombolytika (Plasminogenaktivatoren jeglicher Art), anderen profibrinolytisch wirksamen Substanzen, Blutdrucksenkern, Regulatoren des Blutzuckers, Lipidsenkern und Antiarrhythmika verabreicht werden.

### Beispiele

Endprodukte werden in der Regel durch massenspektroskopische Methoden (FAB-, ESI-MS) und ¹H-NMR bestimmt, angegeben sind jeweils der Hauptpeak oder die beiden Hauptpeaks. Temperaturangaben in Grad Celsius, Ausb. bedeutet Ausbeute, RT bedeutet Raumtemperatur (21 °C bis 24 °C). Verwendete Abkürzungen sind entweder erläutert oder entsprechen den üblichen Konventionen.

Wenn nicht anders aufgeführt, wurden die LC/MS-Analysen unter folgenden Bedingungen durchgeführt:
Methode A: = Methode Säule: YMC Jsphere H80 20x2 mm, Packungsmaterial 4 µm,
Laufmittel: CH₃CN : H₂O + 0,05% Trifluoressigsäure (TFA), Gradient: 4:96 (0 min.) nach 95:5 (2,0 min.) nach 95:5 (2,4 min) nach 4:96 (2,45 min) Fluss: 1,0 mL/min.,
Temperatur: 30 °C.
Methode B: Säule: YMC Jsphere 33x2,1 mm, Packungsmaterial 4 µm, Laufmittel: CH₃CN + 0,05% TFA : H₂O + 0,05% TFA, Gradient: 5:95 (0 min.) nach 95:5 (2,5 min.) nach 95:5 (3,0 min), Fluss: 1,3 mL/min., Temperatur: 30°C.
Methode C: Säule: YMC Jsphere 33x2,1 mm, Packungsmaterial 4 µm, Laufmittel: CH₃CN + 0,08% Ameisensäure : H₂O + 0,1% Ameisensäure, Gradient: 5:95 (0 min.) nach 95:5 (2,5 min.) nach 95:5 (3,0 min), Fluss: 1,3 mL/min., Temperatur: 30°C.
Methode D: Säule: YMC Jsphere 33x2,1 mm, Packungsmaterial 4 µm, Laufmittel: CH₃CN + 0_{;}05% TFA : H₂O + 0,05% TFA, Gradient: 5:95 (0-0.5 min.) nach 95:5 (3,5 min.) nach 95:5 (4,0 min), Fluss: 1,3 mL/min., Temperatur: 30°C.
Methode E: Säule: YMC Jsphere 33x2,1 mm, Packungsmaterial 4 µm, Laufmittel: CH3CN + 0,05% TFA : H₂O + 0,05% TFA, Gradient: 2:98 (0-1.0 min.) nach 95:5 (5.0 min.) nach 95:5 (6,2 min), Fluss: 1,0 mL/min., Temperatur: 30°C.
Methode F: Säule: YMC Jsphere 33x2,1 mm, Packungsmaterial 4 µm, Laufmittel: CH₃CN + 0,05% TFA : H₂O + 0,05% TFA, Gradient: 5:95 (0 min.) nach 95:5 (3,4 min.) nach 95:5 (4,4 min), Fluss: 1,0 mL/min., Temperatur: 30°C.

Soweit nicht anders angegeben, wurden chromatographische Trennungen an Kieselgel mit Ethylacetat/Heptan-Gemischen als Laufmittel durchgeführt. Präparative Trennungen an Reversed Phase-(RP) Kieselgel (HPLC) wurden, soweit nicht anders angegeben, unter folgenden Bedingungen durchgeführt: Säule Merck Hibar RT 250-25 LiChrospher 100 RP-18e 5µm, mobile Phase A: H₂O + 0,1% TFA, Phase B: 80% Acetonitril + 0,1% TFA, Fluss 25 ml/ min., 0-7 min. 100% A , 7-22 min. auf 100% B, 22-30 min. 100% B, 30-33 min. auf 100% A, 33-35 min. 100% A.
Das Abdampfen von Lösungsmitteln geschah in der Regel unter vermindertem Druck bei 35 °C bis 45 °C am Rotationsverdampfer.

### Beispiel 1

### (S)-6-Amino-2-{3-[(R)-1-(3-methyl-butylcarbamoyl)-2-phenyl-ethyl]-ureido}-hexansäure Hydrochlorid

### Beispiel 1a)

### (R)-1-(3-Methyl-butylcarbamoyl)-2-phenyl-ethyl]-carbaminsäure-tert-butyl ester

Eine Lösung von N-Boc-D-Phenylalanin (2,653g, 10mmol) in Tetrahydrofuran (THF) (80mL) wurde sukzessive mit 1-Hydroxybenzotriazol-Hydrat (1,685g, 11mmol) und N,N'-Dicyclohexylcarbodümid (DCC, 2,270 g, 11 mmol) versetzt und 2 h bei RT gerührt. Anschließend wurde Isoamylamin (1,162mL, 10mmol) zugesetzt und bei RT weitergerührt. Nach Stehenlassen über Nacht wurde abfiltriert, das Filtrat eingeengt, in Ethylacetat aufgenommen, erneut filtriert, sukzessive mit gesättigter NaHCO₃-Lösung und 1 N HCl gewaschen, die organische Phase über MgSO₄ getrocknet, filtriert und eingeengt.
LC/MS-Daten: Rₜ(min) 1,568; berechnet (ber.): [M+H]⁺ = 335.47 gefunden (gef.): 235.15(- tert-Butyloxycarbonyl während der Messung) (Methode A)

### Beispiel 1b)

### (R)-2-Amino-N-(3-methyl-butyl)-3-phenyl-propionamid

Eine Lösung des Rohprodukts aus Beispiel 1a) (2,710g, 8,103mmol) in Dichlormethan/Trifluoressigsäure (TFA) (60mL, 1:1 v/v) wurde 30min bei RT gerührt. Die Lösung wurde eingeengt, in Ethylacetat aufgenommen und mit 1 N HCl gewaschen. Die wässrige Phase wurde mit Kaliumhydroxyd schwach alkalisch gestellt und dreimal mit Ethylacetat extrahiert. Die vereinigten-organischen Phasen wurden über MgSO₄ getrocknet, filtriert und eingeengt.
LC/MS-Daten: Rₜ(min) 0,978; ber.: [M+H]⁺ = 235.35 gef.: 235.15 (Methode A)

### Beispiel 1c)

### (S)-6-tert-Butoxycarbonylamino-2-{3-[(R)-1-(3-methyl-butylcarbamoyl)-2-phenyl-ethyl]-ureido}-hexansäure-tert-butylester

Zu einer Lösung von 1,1'-Carbonyldümidazol (0,955g, 5,889mmol) in Dimethylformamid (DMF) (21 mL) wurde das Rohprodukt aus Beispiel 1b) (1,380g, 5,889mmol) gegeben und 1 h bei RT gerührt. Anschließend wurden Triethylamin (1,633mL, 11,780mmol) und (S)-2-Amino-6-tert-butoxycarbonylamino-hexansäure-tert-butylester Hydrochlorid (1,996g, 5,889mmol) hinzugegeben und über Nacht bei RT stehen gelassen. Die Lösung wurde eingeengt, zwischen Wasser und Ethylacetat verteilt, die organische Phase über MgSO₄ getrocknet, filtriert und eingeengt. Das erhaltene Rohprodukt wurde über präparative HPLC gereinigt.
LC/MS-Daten: Rₜ(min) 1,757; ber.: [M+H]⁺ = 563,76 gef.: 563,35 (Methode A)

### Beispiel 1d)

### (S)-6-Amino-2-{3-[(R)-1-(3-methyl-butylcarbamoyl)-2-phenyl-ethyl]-ureido}-hexansäure Hydrochlorid

Das Produkt aus Beispiel 1c) (0,500g, 0,889mmol) wurde in Dichlormethan/TFA (10mL,1:1, v/v) gelöst und bei RT für 2 h gerührt. Die Lösung wurde eingeengt und über präparative HPLC gereinigt. Die vereinigten Produktfraktionen wurden mit 2N HCl versetzt, eingeengt und gefriergetrocknet.
LC/MS-Daten: Rₜ(min) 0.971; ber.: [M+H]⁺ = 407.54 gef.: 407.30 (Methode A)

### Beispiel 11

### (S)-6-Amino-2-{3-[(R)-2-cyclohexyl-1-(2,4-difluoro-benzylcarbamoyl)-ethyl]-ureido}-hexansäure

### Beispiel 11a)

### (R)-2-Amino-3-cyclohexyl-propansäure Trifluoracetat

Eine Lösung von (R)-N-Boc-2-Amino-3-cyclohexyl-propansäure (3,0 g, 11,1 mmol) in 20 ml CH₂Cl₂ wurde mit 5 ml TFA versetzt und über Nacht bei RT gerührt. Nach vollständiger Entschützung wurde das CH₂Cl₂ abgedampft, der verbleibende Feststoff mit 50 ml H₂O versetzt und lyophilisiert. Ausb.: 2,84 g (90%) farbloser Feststoff des (R)-2-Amino-3-cyclohexyl-Propansäure Trifluoracetates.

### Beispiel 11b)

### (S)-6-tert-Butoxycarbonylamino-2-[3-((R)-1-carboxy-2-cyclohexyl-ethyl)-ureido]-hexansäure tert-butyl ester

Kommerzielle (S)-2-Amino-6-tert-butoxycarbonylamino-hexansäure tert-butyl ester hydrochlorid (1,95 g, 5,75 mmol) wurde in 30 ml DMF mit NEt₃ (0,8 ml, 5,754 mmol) und 1,1'-Carbonyl-diimidazol (0,933 g, 5,754 mmol) versetzt und 30 min bei RT gerührt. Anschließend wurden (R)-2-Amino-3-cyclohexyl-Propansäure Trifluoracetat (1,64 g, 5,754 mmol) und NEt₃ (1,6 ml, 11,5 mmol) zugesetzt und das Gemisch auf 80°C bis zum vollständigen Umsatz des intermediär gebildeten Imidazolides erhitzt. Das Produkt wurde durch Flashchromatographie auf Kieselgel (CH₂Cl₂/MeOH-Gradient) gereinigt. Ausb.: 2,1 g (73%) des (S)-6-tert-Butoxycarbonylamino-2-[3-((R)-1-carboxy-2-cyclohexyl-ethyl)-ureido]-hexansäure-tert-butylesters.

### Beispiel 11c)

### (S)-6-Amino-2-{3-[(R)-2-cyclohexyf-1-(2,4-difluoro-benzylcarbamoyl)-ethyl]-ureido}-hexansäure Trifluoracetat

Eine Lösung von (S)-6-tert-Butoxycarbonylamino-2-[3-((R)-1-carboxy-2-cyclohexyl-ethyl)-ureido]-hexansäure-tert-butyl ester (80 mg, 0,16 mmol) und 2,4-Difluorbenzyl-amin (22,9 mg, 0,16 mmol) in 3 ml CH₂Cl₂ und 1 ml DMF wurde in der angegebenen Reihenfolge mit N-Methylmorpholin (53 µl, 0,48 mmol), 1-Hydroxybenzotriazol (28 mg, 0,208 mmol) und 1-(3-Dimethylaminopropyl)-3-ethylcarbodiimid Hydrochlorid (36,8 mg, 0,192 mmol) versetzt und bei RT etwa 14 h gerührt. Extrahieren mit CH₂Cl₂/H₂O, Trocknen der organischen Phase mit MgSO₄ und Eindampfen ergab (S)-6-tert-Butoxycarbonylamino-2-{3-[(R)-2-cyclohexyl-1-(2,4-difluorobenzyl-carbamoyl)-ethyl]-ureido}-hexansäure-tert-butylester als Rohprodukt. Das gesamte Rohprodukt wurde in 4 ml CH₂Cl₂ gelöst, mit 1 ml TFA versetzt, nach 4h mit weiteren 0,5 ml TFA versetzt, und etwa 10 h bei RT entschützt. Die Reinigung des entschützten Rohproduktes über präp. HPLC ergab 25 mg (27%) des (S)-6-Amino-2-{3-[(R)-2-cyclohexyl-1-(2,4-difluoro-benzylcarbamoyl)-ethyl]-ureido}-hexansäure Trifluoracetats.

### Beispiel 130:

### (S)-6-Amino-2-{3-[(R)-5-benzyloxycarbonylamino-1-((S)-1-carbamoyl-2-methyl-propylcarbamoyl)-pentyl]-ureido}-hexansäure

### Beispie1130a) [(R)-5-tert-Butoxycarbonylamino-5-((S)-1-carbamoyl-2-methyl-propylcarbamoyl)-pentyl]-carbaminsäure benzylester

Eine Lösung von kommerziell erhältlicher (R)-6-Benzyloxycarbonylamino-2-tert-butoxycarbonylamino-hexansäure (1 g, 2,63 mmol) und kommerziell erhältlichem (S)-2-Amino-3-methyl-butyramid Hydrochlorid (0,40 g, 2,63 mmol) in 12 ml CH₂Cl₂ und 4 ml DMF wurde in dieser Reihenfolge mit N-Methylmorpholin (0,87 ml, 7,9 mmol), 1-Hydroxybenzotriazol (0,46 g, 3,41 mmol) und 1-(3-Dimethylaminopropyl)-3-ethylcarbodiimid Hydrochlorid (0,65 g, 3,41 mmol) versetzt und etwa 14 h bei RT gerührt. Flashchromatographie (Gradient Heptan/AcOEt nach CH₂Cl₂/MeOH) ergab 1 g des Produktes (79%).

### Beispiel 130b)

### [(R)-5-Amino-5-((S)-1-carbamoyl-2-methyl-propylcarbamoyl)-pentyl]-carbaminsäure benzyl ester hydrochlorid

Eine Lösung von [(R)-5-tert-Butoxycarbonylamino-5-((S)-1-carbamoyl-2-methyl-propylcarbamoyl)-pentyl]-carbaminsäure benzylester (1 g, 2,09 mmol) in 30 ml CH₂Cl₂ wurde mit 5 ml H₂O und 5 ml konz. HCl/H₂O versetzt und bis zur vollständigen Abspaltung der Boc-Schutzgruppe auf 40 °C erhitzt. Extraktion mit H₂O/ CH₂Cl₂, Trocknen der organischen Phase über MgSO₄ und Eindampfen ergab 230 mg (27%) des Produktes.

### Beispiel 130c)

### (S)-2-{3-[(R)-5-Benzyloxycarbonylamino-1-((S)-1-carbamoyl-2-methyl-propylcarbamoyl)-pentyl]-ureido}-6-tert-butoxycarbonylamino-hexansäure-tert-butylester Trifluoracetat

Kommerziell erhältliches (S)-2-Amino-6-tert-butoxycarbonylamino-hexansäure-tert-butylester Hydrochlorid (89 mg, 0,26 mmol) wurde in 4 ml DMF mit NEt₃ (0,12 ml, 0,53 mmol) und 1,1'-Carbonyl-diimidazol (43 mg, 0,26 mmol) versetzt und 1 h bei RT gerührt. Anschließend wurde [(R)-5-Amino-5-((S)-1-carbamoyl-2-methyl-propylcarbamoyl)-pentyl]-carbaminsäure-benzylester Hydrochlorid (100 mg, 0,24 mmol) zugesetzt und das Gemisch bis zum vollständigen Umsatz des intermediär gebildeten Imidazolides auf 80 °C erhitzt. Präp. HPLC ergab 76 mg (39%) des (S)-2-{3-[(R)-5-Benzyloxycarbonylamino-1-((S)-1-carbamoyl-2-methyl-propylcarbamoyl)-pentyl]-ureido}-6-tert-butoxycarbonylamino-hexansäure-tert-butylester Trifluoracetats.

### Beispiel 130d)

### (S)-6-Amino-2-{3-[(R)-5-benzyloxycarbonylamino-1-((S)-1-carbamoyl-2-methyl-propylcarbamoyl)-pentyl]-ureido}-hexansäure Trifluoracetat

(S)-2-{3-[(R)-5-Benzyloxycarbonylamino-1-((S)-1-carbamoyl-2-methyl-propylcarbamoyl)-pentylj-ureido}-6-tert-butoxycarbonylamino-hexansäure-tert-butylester Trifluoracetat (37 mg, 0,045 mmol) wurde in 5 ml CH₂Cl₂ und 1 ml TFA gelöst und 14 h bei RT gerührt. Präp. HPLC ergab 21 mg (70%) des (S)-6-Amino-2-{3-[(R)-5-benzyloxycarbonylamino-1-((S)-1-carbamoyl-2-methyl-propylcarbamoyl)-pentyl]-ureido}-hexansäure Trifluoracetats.
LC/MS: Rₜ (min) = 1,17 ber.: [M+H]⁺ = 551,32, gefunden gef.: 551,31 (Methode B).

### Beispiel 143

### (S)-6-Amino-2-(3-{(S)-1-[(S)-1-((S)-1-methoxycarbonyl-2-methyl-propylcarbamoyl)-2-methyl-propylcarbamoyl]-2-phenyl-ethyl}-sulfamidyl)-hexansäure

### Beispiel 143a)

### (S)-2-((S)-2-Amino-3-methyl-butyryl-amino)-3-methyl-buttersäuremethylester

600mg (1,65 mmol) kommerziell erhältlicher (S)-2-((S)-2-Benzyloxycarbonylamino -3-methyl-butyrylamino)-3-methyl-buttersäuremethylester (Z-Val-Val-OMe) wurde in 10 mL Methanol gelöst, mit 20 mg Palladium auf Kohle (10%) versetzt und für 2 h bei RT unter einer Wasserstoffatmosphäre (1bar) gerührt. Die Reaktionsmischung wurde abfiltriert und eingeengt und ergab quantitativ die Titelverbindung.
LC/MS: Rₜ(min) 0,85; ber.: [M+H]⁺ 231,17 gef.: 231,16 (Methode B).

### Beispiel 143b)

### (S)-2-[(S)-2-((S)-2-Benzyloxycarbonylamino-3-phenyl-propionylamino)-3-methyl-butyrylamino]-3-methyl-buttersäuremethylester

247mg Z-Phe-OH (0,825 mmol, 1eq) wurden in trockenem 10 mL DMF bei 0 °C unter Argon gelöst. Anschließend wurden 56 mg 1-Hydroxybenzotriazol (0,5 eq), 221mg 1-Ethyl-3-(dimethylaminopropyl)carbodiimid-Hydrochlorid (1,4 eq) und 346 µL Hünigs Base (2,4 eq) zugesetzt und für 30 min gerührt. Anschließend wurden 190 mg der Verbindung aus Beispiel 143a) zugesetzt und die Mischung für 20 h bei RT gerührt. Die Reaktionmischung wurde mit 50 mL gesättigter NaHCO₃-Lösung versetzt und mit Ethylacetat (2 x 30 mL) extrahiert. Die organische Phase wurde über Na₂SO₄ getrocknet, filtriert und eingeengt. Das Rohprodukt wurde an Kieselgel mit Heptan/Ethylacetatgemischen chromatographiert. Man erhielt 314 mg der gewünschten Verbindung.
LC/MS: Rₜ(min) 1,85; ber.: [M+H]⁺ 512.28 gef. 512,36 (Methode B).

### Beispiel 143c)

### (S)-2-[(S)-2-((S)-2-Amino-3-phenyl-propionylamino)-3-methyl-butyrylamino]-3-methyl-buttersäuremethylester

Die Z-Entschützung von Z-Phe-Val-Val-OMe zu Phe-Val-Val-OMe wurde wie in Beispiel 143a) beschrieben und lieferte 247 mg der Titelverbindung durchgeführt.
LC/MS: Rₜ(min) 1,09; ber.: [M+H]⁺ 378,24 gef. 378,33 (Methode B).

### Beispiel 143d)

### 2-Oxo-oxazolidin-3-sulfbnylchlorid

Zu einer Lösung von 2,25g Chlorsulforiylisocyanat (15,9 mmol, 1,0eq) in Dichlormethan (100 mL) unter Argon wurde bei 0 °C langsam eine Lösung von 1,13 mL 2-Bromethanol (15,9 mmol, 1,0 eq) in Dichlormethan (20 mL) so zugegeben, dass die Temperatur 10 °C nicht überstieg. Nach Beendigung der Zugabe wurde für 30 min bei 0 °C weitergerührt. Das so erhaltene Produkt wurde direkt im nächsten Schritt weiter umgesetzt.

### Beispiel 143e)

### (S)-6-tert-Butoxycarbonylamino-2-(2-oxo-oxazolidin-3-sulfonylamino)-hexansäure-tert-butylester

Zu der in Beispiel 143d) erhaltenen Lösung wurde eine Suspension von 5,39g H-Lys(Boc)-OtBu-Hydrochlorid (15,9 mmol, 1,0 eq) und 7,1 mL Triethylamin (50,9 mmol, 3,2 eq) in Dichlormethan (70mL) so zugegeben, dass die Temperatur 10 °C nicht überstieg. Nach Beendigung der Zugabe ließ man auf RT kommen und für weitere 2 h rühren. Die Reaktionsmischung wurde dann mit 200 mL 0,2 M Salzsäure versetzt, die organische Phase abgetrennt und mit 100 mL 0,2 M Salzsäure gewaschen und eingeengt. Man erhielt 5,5 g des gewünschten Materials als farbloses Öl, welches bei Stehenlassen kristallisierte.
LC/MS: Rₜ(min) 1,76; ber.: [M+H]⁺ 452,14 gef. 452,18 (Methode B).

### Beispiel 143f)

### (S)-6-tert-Butoxycarbonylamino-2-(3-{(S)-1-[(S)-1-((S)-1-methoxycarbonyl-2-methyl-propylcarbamoyl)-2-methyl-propylcarbamoyl]-2-phenyl-ethyl}-sulfamidyl)-hexansäure-tert-butyl ester

240 mg Phe-Val-Val-OMe (Verbindung aus Beispiel 143c), 0,636 mmol, 1 eq) wurde mit 345 mg der Verbindung aus Beispiel 143e) in 7 mL Acetonitril gelöst und mit 106 µL Triethylamin versetzt. Das Reaktionsgemisch wurde 20 h bei 80 °C gerührt und nach dem Abkühlen eingedampft. Das Rohprodukt wurde durch Chromatographie an Kieselgel mit Heptan/Ethylacetatgemischen als Laufmittel gereinigt. Es wurden 275 mg der Titelverbindung erhalten.
LC/MS: Rₜ(min) 1,733; ber.: [M+H]⁺ 742,41 gef. 742,35 (Methode A).

### Beispiel 143g)

### (S)-6-Amino-2-(3-{(S)-1-[(S)-1-((S)-1-methoxycarbonyl-2-methyl-propylcarbamoyl)-2-methyl-propylcarbamoyl]-2-phenyl-ethyl}-sulfamidyl)-hexansäure

Eine Lösung von 270 mg der Verbindung aus Beispiel 143f) in 4mL Dichlormethan/TFA (1:1, v/v) wurde 2 h bei RT gerührt und anschließend eingedampft. Der Rückstand wurde durch präparative HPLC gereinigt und lieferte 131 mg der Titelverbindung als Trifluoracetat.
LC-MS: Rₜ(min) 1,16; ber.: [M+H]⁺, 586,29 gef. 586,39 (Methode B).

### Beispiel 144

### (S)-6-Amino-2-{3-[(R)-1-(bicyclo[2.2.1]hept-2-ylcarbamoyl)-2-cyclohexyl-ethyl]-sulamidyl}-hexansäure

Die Titelverbindung wurde analog zu Beispiel 143 unter Einsatz eines kommerziell erhältlichen endo-Norborbonylamin anstelle des Dipeptids in Beispiel 143c).
LC-MS: Rₜ(min) 1,34; ber.: [M+H]⁺ 473,28 gef. 473,36 (Methode B).

### Beispiel 145

(S)-6-Amino-2-[3-((S)-1-cyclohexylcarbamoyl-2-phenyl-ethyl)-sulfamidyl]-hexansäure Die Titelverbindung wurde analog zu Beispiel 143 unter Einsatz eines kommerziell erhältlichen Cyclohexylamins anstelle des Dipeptids in Beispiel 143c).
LC-MS: Rₜ(min) 1,20; ber.: [M+H]⁺ 455,24 gef. 455,33 (Methode B).

Analog zu Beispiel 143 wurden folgende Beispiele hergestellt:

| Beispiel | Formel | LC/MS Methode | Rₜ | [M+H]⁺ ber. | [M+H]⁺ gef. |
|---|---|---|---|---|---|
| 146 | 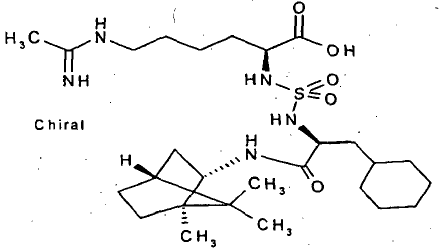 | D | 2,20 | 556,35 | 556,36 |
| 147 | 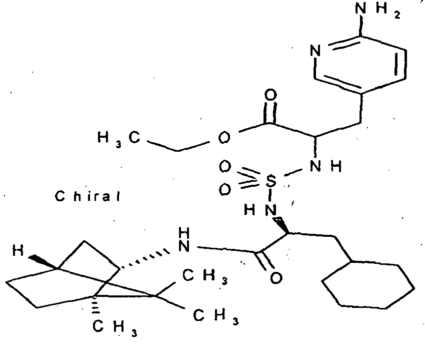 | D | 2,40 | 578,34 | 578,41 |
| 148 | 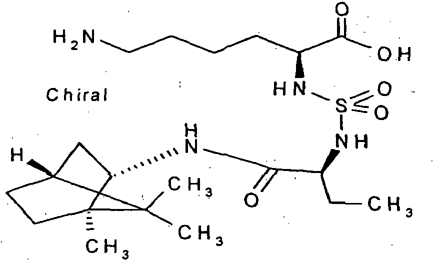 | D | 1,80 | 447,26 | 447,28 |
| 149 | 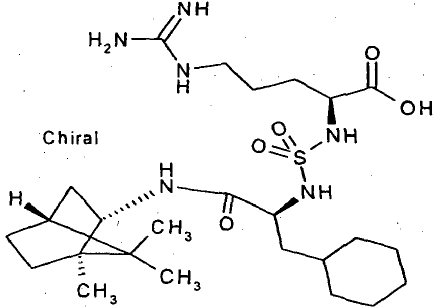 | D | 2,21 | 543,33 | 543,38 |
| 150 | 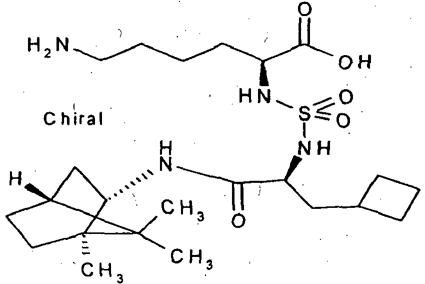 | C | 1,58 | 485,28 | 485,39 |
| 151 | 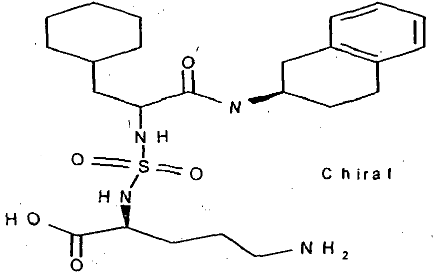 | A | 1,14 | 496,67 | 495,35 |
| 152 | 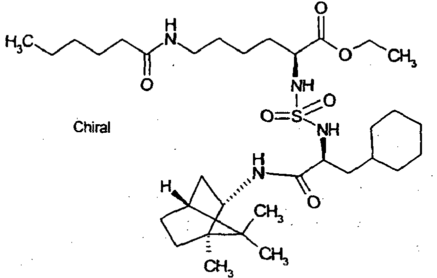 | D | 3,21 | 641,43 | 641,34 |
| 153 | 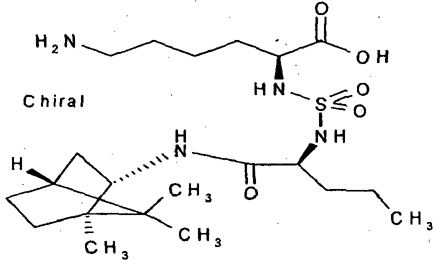 | D | 1,96 | 461,28 | 461,23 |
| 154 | 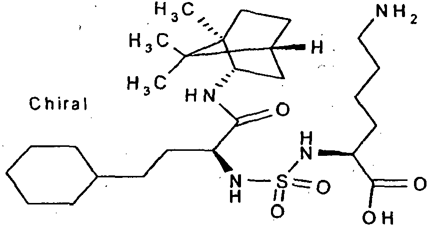 | D | 2,29 | 529,34 | 529,34 |
| 155 | 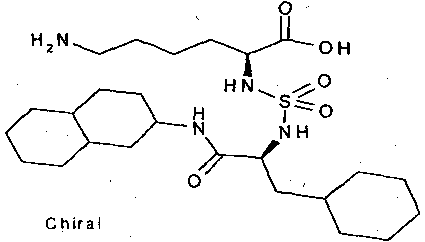 | B | 1,51 | 515,33 | 515,34 |
| 156 | 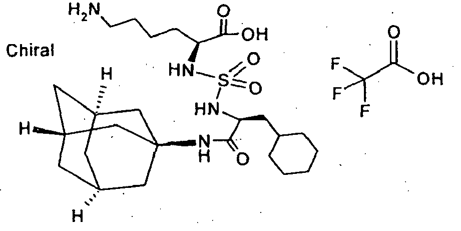 | F | 1,63 . | 513,31 | 513,33 |
| 157 | 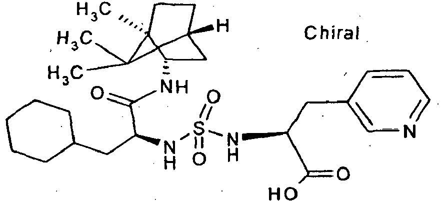 | C | 1,91 | 533,28 | 533,17 |
| 158 | 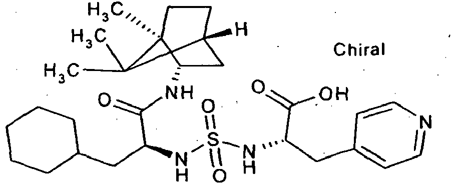 | C | 1,84 | 533,28 | 533,23 |
| 159 | 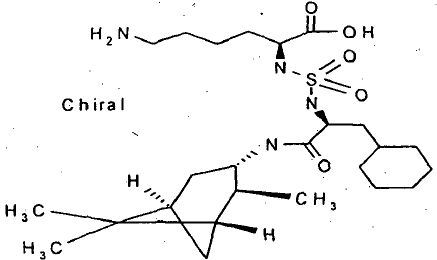 | B | 1,51 | 515,33 | 515,56 |
| 160 | 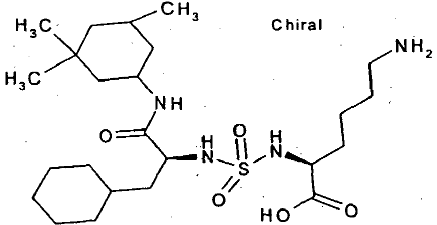 | B | 1,50 | 503,33 | 503,49 |
| 161 | 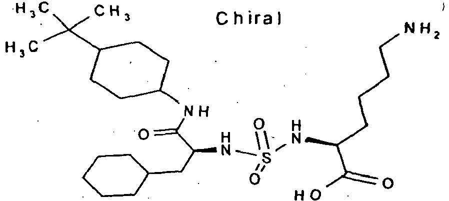 | B | 1,58 | 517,34 | 517,49 |
| 162 | 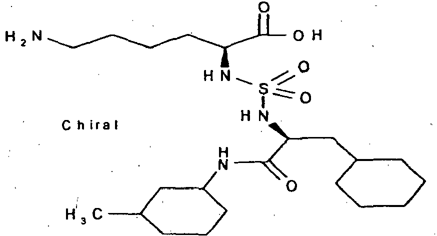 | B | 1,38 | 475,30 | 475,45 |

### Beispiel 163

### (S)-6-Amino-2-{[(S)-2-cyclohexyl-1-((1R,2S,4R)-1,7,7-trimethyl-bicyclo[2.2.1]hept-2-ylcarbamoyl)-ethylsulfamidyl]}-hexansäure

### 1) (S)-6-Benzyloxycarbonylamino-2(2-oxo-oxazolidin-sulfonylamino)-hexansäure benzylester

Zu einer Lösung von 5,21 g Chlorsulfonylisocyanat (36,9 mmol, 1,0 equiv.) in Dichloromethane (300 mL) wurde bei 0°C unter Argon langsam eine Lösung von 2,61 mL 2-Bromethanol (36,9 mmol, 1,0 equiv.) in Dichloromethan (20 mL) gegeben, so daß die interne Temperatur unter 10 °C blieb. Anschließend wurde weitere 30 min bei 0 °C gerührt. Zu dieser Lösung wurde eine Lösung von1 5,0 g H-Lys(Z)-OBzL•HCl (36,9 mmol, 1,0 equiv.) und 16,5 mL Triethylamin (118,0 mmol, 3,2 equivalente (equiv.)) in 120 mL CH₂Cl₂ getropft, so, daß die Temperatur des Reaktionsgemischs nicht über 10 °C kam. Nach der Zugabe wurde das Eisbad entfernt und die Mischung bei RT 4 h gerührt. Anschließend wurde die organische Lösung dreimal mit 100 mL 0,2M HCl (aq.) gewaschen, über Na₂SO₄ getrocknet und eingeegngt. Man erhielt 18,4 g der rohen Titelverbindung als farbloses Öl, welches direkt in Schritt 3 weiter eingesetzt wurde.
LC-MS: Rₜ(min) 1,82; Ber.: [M+H]⁺ 520,17 Gef.: 520,30 (Method B).

### 2) [(S)-2-Cyclohexyl-1-((1R,2S,4R)-1,7,7-trimethyl-bicyclo[2.2.1]hept-2-ylcarbamoyl)-ethyl]-carbaminsäure-tert-butylester

Zu einer Lösung von 5,0 g (S)-2-tert-Butoxycarbonylamino-3-cyclohexyl-propion-säure (Boc-Cha-OH, 18,4 mmol, 1,0 equiv.) in DMF (60 mL) wurden bei 0 °C unter Argon 3,53 g 1-Ethyl-3-(3-dimethylaminopropyl)carbodiimid Hydrochlorid (18,4 mmol, 1,0 equiv.), 1,25 g 1-Hydroxybenzotriazol (9,2 mmol, 0,5 equiv.) und 7,3 mL Hünigs Base gegeben und die Mischung wurde 30 min gerührt. Anschließend wurden 2,83 g (R)-(+)-Bornylamin (18,4 mmol, 1,0 equiv.) und 3,7 mL Hünigs Base zugegeben und die Mischung 16 h bei RT gerührt. Das Reaktionsgemisch wurde gequencht mit NaHCO₃ (gesättigt, aq.) und dreimal mit Ethylacetat extrahiert. Die vereinigten organischen Phasen wurden zweimal mit Wasser gewaschen und über Na₂SO₄ getrocknet und eingeengt. Reinigung durch Flash-Chromatographie an Kieselgel mit Heptan/Ethylacetatgemischen als Eluens ergab 6,58 g (88% Ausbeute) der Titelverbindung als farbloses Öl.
LC-MS: Rₜ(min) 2,42; Ber.: [M+H]⁺ 407,33 Gef.: 407,32 (Method B).

### 3) (S)-2-Amino-3-cyclohexyl-N-((1R,2S,4R)-1,7,7-trimethyl-bicyclo[2.2.1]hept-2-yl)-propionamid Trifluoracetat

Zu einer Lösung von 6,5 g [(S)-2-Cyclohexyl-1-((1R,2S,4R)-1,7,7-trimethyl-bicyclo[2.2.1]hept-2-ylcarbamoyl)-ethyl]-carbaminsäure-tert-butyl ester (16,0 mmol) in 50 mL CH₂Cl₂ wurden unter Argon bei 0 °C langsam 50 mL TFA gegeben. Man ließ auf RT kommen. Nach 3 h wurde das Reaktionsgemisch eingeengt. Die Titelverbindung wurde als schwach gelbes Öl erhalten, welches direkt im nächsten Schritt eingesetzt wurde.
LC-MS: Rₜ(min) 1,60; Ber.: [M+H]⁺ 307,27 Gef.: 307,39 (Method C).

### 4) (S)-6-Benzyloxycarbonylamino-2-{[(S)-2-cyclohexyl-1-((1R,2S,4R)-1,7,7-trimethyl-bicyclo[2.2.1]hept-2-ylcarbamoyl)-ethylsulfamidyl]}-hexansäurebenzylester

11,63 g (S)-6-Benzyloxycarbonylamino-2(2-oxo-oxazolidine-sulfonylamino)-hexansäurebenzylester (22,4 mmol, 1,4 equiv.) und 4,9 g (S)-2-Amino-3-cyclohexyl-N-((1R,2S,4R)-1,7,7-trimethyl-bicyclo[2.2.1]hept-2-yl)-propionamid Trifluoracetat (16,0 mmol, 1,0 equiv.) wurden in 80 mL MeCN suspendiert und nach Zugabe von 8,9 mL Et₃N wurde die Mischung 20 h unter Rückfluß erhitzt. Nach dem Abkühlen wurde die flüchtigen Bestandteile am Rotationsverdampfer entfernt und der Rückstand durch Flash-Chrömatographie an Kieselgel mit Heptan/Ethylacetat-Gemischen als Eluens gereinigt. Man erhielt 9,0 g (76% Ausbeute) der Titelverbindung als farblosen Schaum.
LC-MS: Rₜ(min) 2,61; Ber.: [M+H]⁺ 739,41 Gef.: 739,43 (Method B).

### 5) (S)-6-Amino-2-{[(S)-2-cyclohexyl-1-((1R,2S,4R)-1,7,7-trimethyl-bicyclo[2.2.1]hept-2-ylcarbamoyl)-ethylsulfamidyl]}-hexansäure

9,0 g (S)-6-Benzyloxycarbonylamino-2-{[(S)-2-cyclohexyl-1-((1R,2S,4R)-1,7,7-trimethyl-bicyclo[2.2.1]hept-2-ylcarbamoyl)-ethylsulfamidyl]}-hexansäurebenzylester (12,2 mmol) wurden in 90 mL Methanol gelöst und nach Zugabe von 600 mg 10% Pd/C 3,5 h bei RT und Normaldruck hydriert. Das Reaktionsgemisch wurde über Celite filtriert und eingeengt. Man erhielt 6,1g (97%) der Titelverbindung als farbloses Öl. 100 mg der Verbindung wurden in 5 mL MeCN gelöst. Nach Zugabe von 50 mL Wasser wurde eine Suspension erhalten. Nach Gefriertrocknung erhielt man einen farblosen Feststoff.
LC-MS: Rₜ(min) 1,70; Ber.: [M+H]⁺ 515,33 Gef.: 515,35 (Method F).
¹H-NMR (DMSO-d₆) δ 0,68 (s, 3H), 0,82 (s, 3H), 0,83-0,91 (m, 2H), 0,89 (s, 3H), 0,97 (dd, 1H, *J* = 4,8, 13,0 Hz), 1,08-1,34 (m, 7H), 1,35-1,55 (m; 5H), 1,56-1,72 (m, 9H), 1,78 (d, 1 H, *J =* 13,0 Hz), 2,04-2,13 (m. 1 H), 2,75 (t, 2H, *J* = 7,1 Hz), 3,51 (t, 1 H, *J* = 5,5 Hz), 3,83 (t, 1H, *J* = 7,0 Hz), 4,03-4,10 (m, 1 H), 6,91-7,05 (br, 1H), 7,77 (d, 1H, *J* = 8,8 Hz), 7,5-8,2 (br, 2H).

### Beispiel 164

### 3-(6-Amino-pyridin-3-ylmethyl)-2-[(S)-2-cyclohexyl-1-((1R,2S,4R)-1,7,7-trimethyl-bicyclo[2.2.1]hept-2-yl-carbamoyl)-ethylsulfamidyl]-propionsäure

### 1) 2-Amino-3-(6-tert-butoxycarbonylamino-pyridin-3-yl)-propionsäure-tert-butylester

660 mg N-(Diphenylmethylen)glycin-tert-butylester (2,23 mmol, 1,0 equiv) wurden in 15 mL trockenem THF gelöst und unter Argon auf 0 °C gekühlt. Anschließend wurden 2,23 mL 1 M Lithium-hexamethyldisilazan (LiHMDS)-Lösung in THF tropfenweise zugegeben und die Mischung 15 min bei 0°C gerührt. Danach wurden 642 mg (5-Bromomethyl-pyridin-2-yl)-carbaminsäure-tert-butyl ester (2,23 mmol, 1,0 equiv) zugegeben und die Mischung 2 h bei 0 °C gerührt. Die Mischung wurde mit18 mL ges. Citronensäure gequencht und 1 h bei RT gerührt. Die Mischung wurde mit Ethylacetat (2x 30 mL) extrahiert und die organischen Phasen mit 50 mL 1 M HCl gewaschen. Die wäßrigen Phasen wurden vereint und mit 2M NaOH auf pH 10 gestellt und anschließend 3x mit Ethylacetat extrahiert. Die vereinigten organischen Phasen wurden über Na₂SO₄ getrocknet und eingeeengt. Der Rückstand wurde durch Flash-Chromatographie an Kieselgel mit Heptan/Ethylacetatgemischen als Eluens gereinigt. Man erhielt 600 mg (80% Ausbeute) der Titelverbindung als farblosen Feststoff. LC-MS: Rₜ(min) 1,06; Ber.: [M+H]⁺ 338,21 Gef.: 338,27 (Method B).

### 2) 3-(6-tert-Butoxycarbonylamino-pyridin-3-yl)-2-(2-oxo-oxazolidine-3-sulfonylamino)-propionsäure-tert-butyl ester

Zu einer Lösung von 251 mg Chlorsulfonylisocyanate (1,78 mmol, 1,0 equiv.) in Dichloromethan (10 mL) unter Argon bei 0 °C wurde langsam eine Lösung von 0,126 mL 2-Bromethanol (1,78 mmol, 1,0 equiv.) in Dichlormethan (10 mL) getropft, so dass die Temperatur 10 °C nicht überstieg. Nach der Zugabe wurde die Mischung für weitere 30 min bei 0 °C gerührt. Zu dieser Lösung wurde tropfenweise eine Mischung aus 600 mg 2-Amino-3-(6-tert-butoxycarbonylamino-pyridin-3-yl)-propiohsäure-tert-butylester (1,78 mmol, 1,0 equiv.) und 0,545 mL Triethylamin (3,91 mmol, 2,2 equiv.) in 5 mL CH₂Cl₂ gegeben, so dass die Temperatur nicht über 10 °C stieg. Nach der Zugabe wurde das Eisbad entfernt und das Gemisch weitere 3 h bei RT gerührt. Nach dem Einengen wurde der Rückstand an Kieselgel mit Heptan/Ethylacetatgemischen als Eluens chromatographiert. Man erhielt 320 mg (37% Ausbeute) der Titelverbindung als farblosen Feststoff.
LC-MS: Rₜ(min) 1,40; ber.: [M+H]⁺ 487,19 gef.: 487,26 (Method B).

### 3) 3-(6-tert-Butoxycarbonylamino-pyridin-3-ylmethyl)-2-[(S)-2-cyclohexyl-1-((1R,2S,4R)-1,7,7-trimethyl-bicyclo[2.2.1]hept-2-yl-carbamoyl)-ethylsulfamidyl] propionsäure-tert-butylester

320 mg 3-(6-tert-Butoxycarbonylamino-pyridin-3-yl)-2-(2-oxo-oxazolidin-3-sulfonylamino)-propionsäure-tert-butylester (0,66 mmol, 1,0 equiv.) und 277 mg (S)-2-Amino-3-cyclohexyl-N-((1R,2S,4R)-1,7,7-trimethyl-bicyclo[2.2.1]hept-2-yl)-propionamid Trifluoracetat (0,66 mmol, 1,0 equiv.), hergestellt wie oben beschrieben, wurden in 12 mL MeCN suspendiert und nach Zugabe von 0,37 mL Et₃N für 20 h am Rückfluß erhitzt. Nach dem Abkühlen wurden die flüchtigen Bestandteile abgedampft und der Rückstand durch Flash-Chromatographie an Kieselgel mit Heptan/Ethylacetatgemischen als Eluens gereinigt. Man erhielt 139 mg (30% Ausbeute) der Titelverbindung als farblosen Feststoff.
LC-MS: Rₜ(min) 2,19; Ber.: [M+H]⁺ 706,42 Gef.: 706,54 (Method B).

### 4) 3-(6-Amino-pyridin-3-ylmethyl)-2-[(S)-2-cyclohexyl-1-((1R,2S,4R)-1,7,7-trimethyl-bicyclo[2.2.1]hept-2-yl-carbamoyl)-ethylsulfamidyl]-propionsäure Trifluoracetat

135 mg 3-(6-tert-Butoxycarbonylamino-pyridin-3-ylmethyl)-2-[(S)-2-cyclohexyl-1-((1R,2S,4R)-1,7,7-trimethyl-bicyclo[2.2.1]hept-2-yl-carbamoyl)-ethylsulfamidyl] propionsäure-tert-butylester (0,19 mmol) wurden in 1,0 mL CH₂Cl₂ gelöst und auf 0 °C gekühlt. Anschließend wurden 0,8 mL TFA zugegeben und die Mischung bei RT gerührt. Nach 1 h wurden die flüchtigen Bestandteile evaporiert und der Rückstand durch RP-HPLC gereinigt. Man erhielt 70 mg (55% Ausbeute) der Titelverbindung als farblosen Feststoff.
LC-MS: Rₜ(min) 1,61; Ber.: [M+H]⁺ 550,31 Gef.: 550,39 (Method B), 1:1-Mischung der Diastereomeren.
¹H-NMR (DMSO-ds) δ 0,67 (s, 3H), 0,69 (s, 3H), 0,82 (s, 6H), 0,79-0,92 (m, 4H), 1,08-1,39 (m, 16H), 1,56-1,78 (m, 16H), 2,01 (t, 1H, *J* = 12,0 Hz), 2,11 (t, 1H, *J* = 12,0 Hz), 2,70 (dd, 1 H, *J* = 6,4, 13,9 Hz), 2,79 (dd, 2H, *J* = 7,0, 13,9 Hz), 2,94 (dd, 1 H, *J* = 5,5, 14,1 Hz), 3,73-3,90 (m, 2H), 4,01-4,13 (m, 1H), 6,80 (d, 0,5H, *J* = 8,0 Hz), 6,94 (dd, 2H, *J* = 5,3, 8,6 Hz), 7,01 (d, 1H, *J =* 9,1 Hz), 7,11 (d, 1H, *J =* 8,7 Hz), 7,18 (d, 1H, *J* = 9,1 Hz), 7,69-7,74 (m, 3H), 7,79 (d, 2H, *J* = 9,4 Hz), 7,85 (dd, 1H, *J* = 1,9, 9,1 Hz), 7,88-7,99 (br, 4H)

Analog zu Beispiel 163 wurden folgende Beispiele hergestellt:

| | | | | | |
|---|---|---|---|---|---|
| 165 | 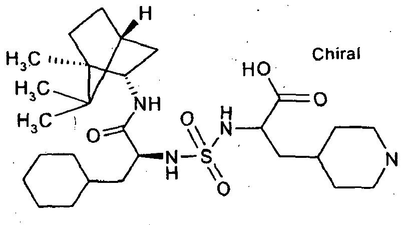 | B | 1,55 | 541, 34 | 541,39 |
| 166 | 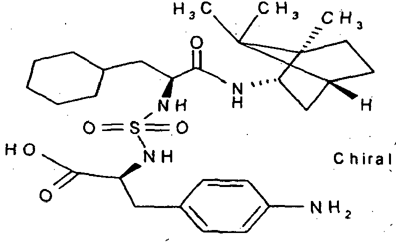 | B | 1,50 | 549,31 | 549,35 |
| 167 | 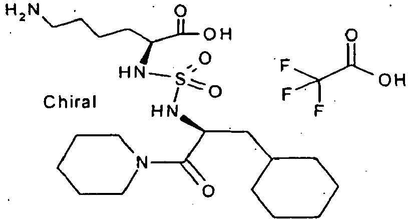 | B | 1,28 | 447,26 | 447,25 . |
| 168 | 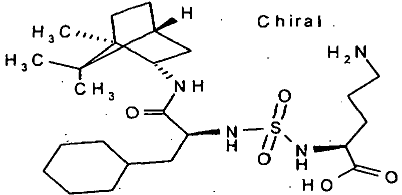 | A | 1,20 | 501,71 | 501,25 |
| 169 | 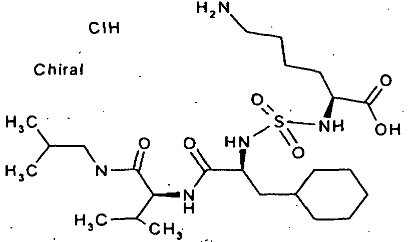 | A | 1,00 | 534,74 | 534,35 |
| 170 | 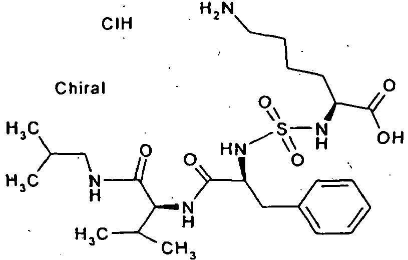 | A | 0,94 | 528,70 | 528,25 |
| 171 | 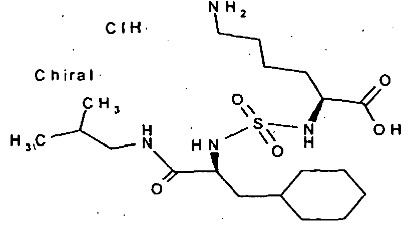 | A | 0,96 | 435,61 | 435,25 |
| 172 | 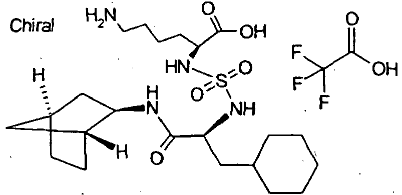 | B | 1,32 | 473,28 | 473,30 |
| 173 | 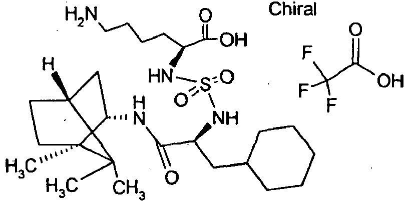 | B | 1,51 | 515,33 | 515,33 |
| 174 | 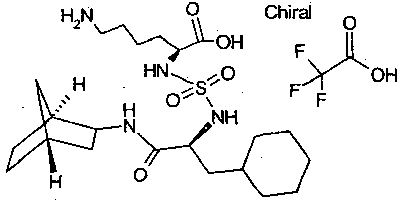 | B | 1,33 | 473,30 | 473,28 |
| 175 | 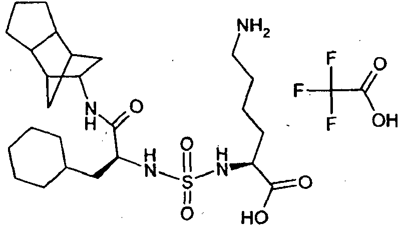 | B | 1,45 | 513,31 | 513,34 |
| 176 | 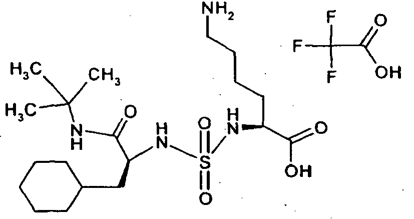 | B | 1,26 | 435,26 | 435,28 |
| 177 | 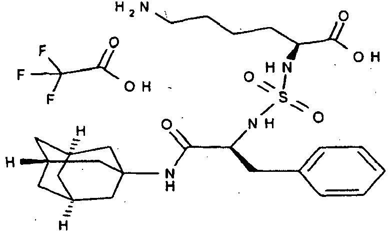 | B | 1,35 | 507,26 | 507,24 |
| 178 | 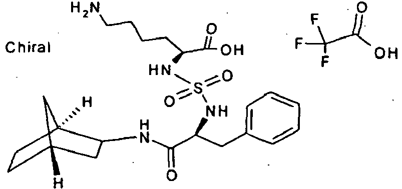 | F | 1,30 | 467,23 | 467,35 |
| 179 | 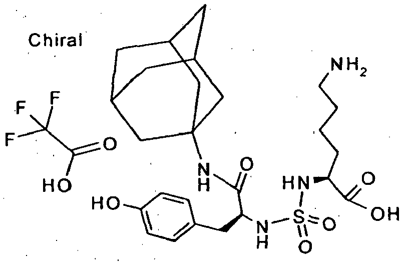 | F | 1,36 | 523,23 | 523,41 |
| 180 | 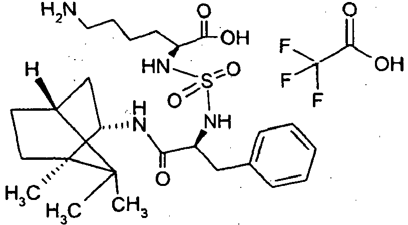 | F | 1,53 | 509,28 | 509,40 |
| 181 | 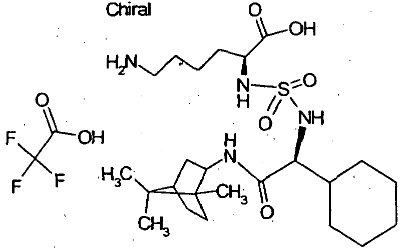 | C | 1,60 | 501,31 | 501,29 |
| 182 | 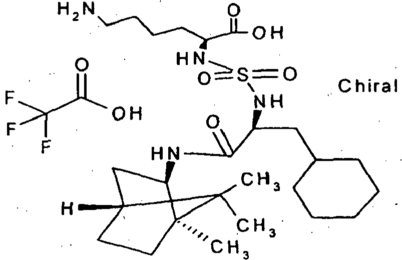 | F | 1,69 | 515,33 | 515,51 |
| 183 | 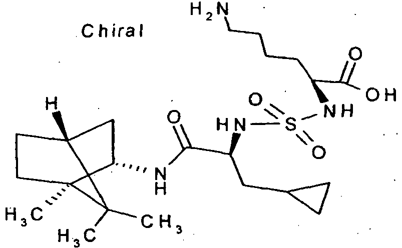 | B | 1,31 | 473,28 | 473,39 |
| 184 | 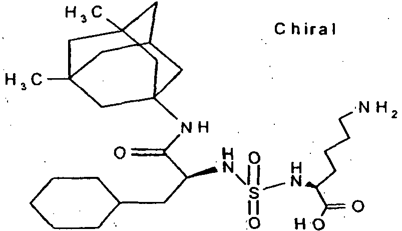 | B | 1,61 | 541,34 | 541,39 |
| 185 | 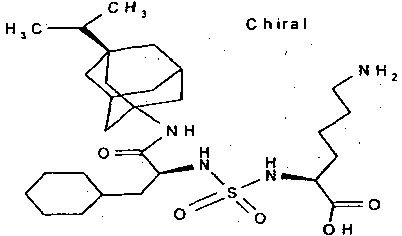 | B | 1,66 | 555,36 | 555,36 |
| 186 | 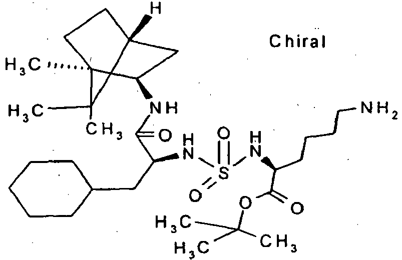 | B | 1,67 | 571, 39 | 571,50 |

### Pharmakologische Beispiele

Die hergestellten Substanzen wurden mit dem Actichrome Plasma TAFI Activity Kit der Firma American Diagnostica (Pr.Nr. 874) auf Inhibition von TAFla geprüft. Dabei wurden zu 2 µl 2,5 mM DMSO-Lösung der Substanz 28 µL Testpuffer (20mM Hepes, 150mM NaCl, pH 7,4) und 10 µL TAFla (American Diagnostica Pr.Nr. 874TAFIA; 2,5 /ml) gegeben und 15 Minuten bei RT in einer 96 half-well Mikrotiterplatte inkubiert. Die Enzymreaktion wurde durch Zugabe von 10 µL TAFla "Developer" (1:2 mit Testpuffer vorverdünnt) gestartet. Der Zeitverlauf der Reaktion wurde bei 420 nm in einem Mikrotiterplattenreader (SpectraMax plus 384; Fa. Molecular Devices) über 15 Minuten verfolgt.

Die IC₅₀-Werte wurden aus den gemittelten Werten (Doppelbestimmung) einer Verdünnungsreihe der Substanz mit Hilfe der Software Softmax Pro (Version 4.8; Fa. Molecular Devices) berechnet.
Tabelle 1 zeigt die Ergebnisse.

**Tabelle 1:**

| Beispiel Nr. | IC₅₀-Wert [µM] | Beispiel Nr. | IC₅₀-Wert [µM] |
|---|---|---|---|
| | | 146 | 0,071 |
| | | 149 | 0,049 |
| | | 150 | 0,357 |
| | | 153 | 1,087 |
| | | 154 | 0,220 |
| | | 155 | 0,669 |
| | | 156 | 0,492 |
| | | 157 | 0,2 |
| | | 159 | 0,131 |
| | | 163 | 0,012 |
| | | 164 | 0,026 |
| | | 165 | 0,882 |
| | | 169 | 0,770 |
| | | 172 | 0,420 |
| | | 173 | 0,012 |
| | | 174 | 0,326 |
| | | 175 | 0,168 |
| | | 177 | 2,117 |
| | | 180 | 0,168 |
| | | 182 | 0,069 |
| | | 183 | 0,805 |
| | | 184 | 1,069 |
| | | 185 | 0,4 |
| | | 186 | 22,943 |
| | | 187 | 10,1176 |
| | | | |
| 143 | 9,756 | | |
| 145 | 10,601 | | |

## Patentansprüche

1. Verbindung der Formel I 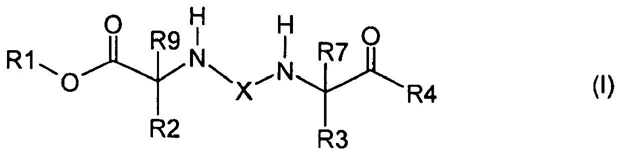 und/oder einer stereoisomeren Form der Verbindung der Formel I und/oder Gemische dieser Formen in jedem Verhältnis, und/oder ein physiologisch verträgliches Salz der Verbindung der Formel I, wobei
X für -SO₂- steht,
R1 für
1) Wasserstoffatom,
2) -(C₁-C₆)-Alkyl,
3) -(C₀-C₄)-Alkylen-(C₃-C₁₂)-Cycloalkyl oder
4) -(C₁-C₆)-Alkylen-(C₆-C₁₄)-Aryl steht,
R2 für den Rest der Formel II
-(A1)ₘ-A2 (II)
steht, worin
m die ganze Zahl Null oder 1 bedeutet,
A1 für
1) -(CH₂)ₙ-, worin n die ganze Zahl Null, 1, 2 oder 3 bedeutet,
2) -NH-(CH₂)ₙ-, worin n die ganze Zahl Null, 1, 2 oder 3 bedeutet,
3) -NH(C₁-C₆)-Alkyl)-(CH₂)ₙ-, worin n die ganze Zahl Null, 1, 2 oder 3 bedeutet,
4) -NH((C₃-C₆)-Cycloalkyl)-(CH₂)ₙ-, worin n die ganze Zahl Null, 1, 2 oder 3 bedeutet,
5) -O-(CH₂)ₙ-, worin n die ganze Zahl Null, 1, 2 oder 3 bedeutet, oder
6) -(CH₂)ₙ-SOₓ-, worin n die ganze Zahl Null, 1, 2 oder 3 bedeutet und x die ganze Zahl Null, 1 oder 2 bedeutet, steht,
A2 für
1) Het, wobei unter Het ein 4- bis 15-gliedriges heterocyclisches Ringsystem mit 4 bis 15 Ringatomen verstanden wird, die in ein, zwei oder drei miteinander verbundenen Ringsystemen vorliegen und die ein, zwei, drei oder vier gleiche oder verschiedene Heteroatome aus der Reihe Sauerstoff, Stickstoff oder Schwefel enthalten und unsubstituiert oder unabhängig voneinander ein-, zwei- oder dreifach durch ein -(C₁-C₃)-Alkyl, Halogen, -NH₂, -CF₃ oder -O-CF₃ substituiert ist,
2) -(C₀-C₆)-Alkylen-NH₂,
3) -(C₁-C₆)-Alkylen-NH-C(=NH)-NH₂,
4) -(C₁-C₆)-Alkylen-NH-C(=NH)-(C₁-C₄)-Alkyl,
5) -(C₀-C₄)-Alkylen-O-NH-C(=NH)-NH₂,
6) -(C₀-C₄)-Alkylen-NH-C(O)-(C₁-C₆)-Alkyl,
7) -(C₁-C₆)-Alkylen-NH-C(O)-O-(C₁-C₄)-Alkylen-Aryl, wobei Aryl unsubstituiert oder durch -NH₂ substituiert ist oder durch -NH₂ und ein-, zwei- oder dreifach durch R15 substituiert ist,
8) -(C₃-C₈)-Cycloalkyl-NH₂, oder
9) -(C₀-C₄)-Alkylen-(C₆-C₁₄)-Aryl, wobei Aryl unsubstituiert oder durch -NH₂ substituiert ist oder durch -NH₂ und ein-, zwei- oder dreifach durch R15 substituiert ist, steht,
R3 für
1) -(C₁-C₆)-Alkyl,
2) -(C₀-C₄)-Alkylen-(C₃-C₁₂)-Cycloalkyl,
3) -(C₁-C₆)-Alkylen-(C₆-C₁₄)-Aryl, wobei Aryl unabhängig voneinander ein-, zwei- oder dreifach durch R15 substituiert ist,
4) -(C₀-C₈)-Alkylen-N(R5)-PG,
5) -(C₁-C₆)-Alkylen-NH-C(O)-O-(C₁-C₄)-Alkylen-Aryl, wobei Aryl unabhängig voneinander ein-, zwei- oder dreifach durch R15 substituiert ist,
6) -(C₀-C₄)-Alkylen-(C₆-C₁₄)-Aryl-(C₀-C₄)-Alkylen-N(R5)-PG,
7) -(C₀-C₈)-Alkylen-O-PG,
8) -(C₀-C₄)-Alkylen-(C₆-C₁₄)-Aryl-(C₀-C₄)-Alkylen-O-PG,
9) -(C₀-C₈)-Alkylen-C(O)-O-PG,
10) -(C₀-C₄)-Alkylen-(C₆-C₁₄)-Aryl-(C₀-C₄)-Alkylen-C(O)-O-PG oder
11) Wasserstoffatom, steht,
R4 für-N(R6)₂, steht,
wobei R6 gleich oder verschieden sind und unabhängig voneinander für
1) Wasserstoffatom,
2) -(C₁-C₆)-Alkyl,
3) -(C₀-C₄)-Alkylen-(C₃-C₁₂)-Cycloalkyl, wobei Cycloalkyl unsubstituiert oder unabhängig voneinander ein-, zwei-, drei- oder vierfach durch R11, Halogen, -C(O)-O-R11, -(C₁-C₄)-Alkyl-O-R11 oder-O-(C₁-C₄)-Alkyl substituiert ist,
4) -(C₀-C₆)-Alkylen-(C₆-C₁₄)-Aryl, wobei Aryl und Alkylen unsubstituiert oder unabhängig voneinander ein-, zwei-, drei- oder vierfach durch R11, Halogen, -C(O)-O-R11, -(C₁-C₄)-Alkyl-O-R11, -C(O)-N(R8)₂ oder -O-(C₁-C₄)-Alkyl substituiert sind,
5) -(C₀-C₈)-Alkylen-N(R5)-PG,
6) -(C₀-C₄)-Alkylen-(C₆-C₁₄)-Aryl-(C₀-C₄)-Alkyl-N(R5)-PG,
7) -(C₀-C₈)-Alkylen-O-PG,
8) -(C₀-C₄)-Alkylen-(C₆-C₁₄)-Aryl-(C₀-C₄)-Alkyl-O-PG,
9) -(C₀-C₈)-Alkylen-C(O)-O-R11,
10) -(C₀-C₄)-Alkylen-(C₆-C₁₄)-Aryl-(C₀-C₄)-Alkyl-C(O)-O-PG,
11) -(C₀-C₄)-Alkylen-Het, wobei unter Het ein 4- bis 15-gliedriges heterocyclisches Ringsystem verstanden wird mit 4 bis 15 Ringatomen, die in ein, zwei oder drei miteinander verbundenen Ringsystemen vorliegen und die ein, zwei, drei oder vier gleiche oder verschiedene Heteroatome aus der Reihe Sauerstoff, Stickstoff oder Schwefel enthalten, wobei Het oder Alkylen unsubstituiert oder unabhängig voneinander ein-, zwei- oder dreifach durch R11, Halogen, -C(O)-O-R11, -(C₁-C₄)-Alkyl-O-R11 oder-O-(C1-C4)-Alkyl substituiert sind,
12) -(C₁-C₃)-Fluoralkyl,
13) -(C₀-C₄)-Alkylen-CH(R11)-C(O)-NH₂,
14) -(C₀-C₄)-Alkylen-CH(R11)-C(O)-NH-(C₁-C₄)-Alkyl, oder
15) -(C₀-C₄)-Alkylen-CH(R11)-C(O)-NH-CH(R12)-R13 stehen,
oder die beiden Reste R6 zusammen mit dem N-Atom, an das sie gebunden sind, einen mono- oder bicyclischen Ring mit 4 bis 9 Ringatomen bilden, der gesättigt, teilweise gesättigt oder aromatisch ist, wobei der Ring unsubstituiert oder ein- oder zweifach durch -(C₁-C₄)-Alkyl, -C(O)-O-R11, Halogen, -(C₁-C₄)-Alkyl-O-R11 oder Phenyl substituiert ist,
R5 für Wasserstoffatom oder-(C₁-C₆)-Alkyl steht,
PG für eine Schutzgruppe für die Amino-, Carboxyl- oder für die Hydroxyfunktion steht,
R7 für Wasserstoffatom oder -(C₁-C₆)-Alkyl steht,
R8 für Wasserstoffatom oder -(C₁-C₆)-Alkyl steht,
R9 für Wasserstoffatom oder-(C₁-C₆)-Alkyl steht,
R11 und R12 gleich oder verschieden sind und unabhängig voneinander für
1) Wasserstoffatom,
2) -(C₁-C₆)-Alkyl,
3) -(C₀-C₄)-Alkylen-Phenyl, wobei Phenyl unsubstituiert oder unabhängig voneinander ein-, zwei- oder dreifach durch Halogen, -OH oder -O-(C₁-C₄)-Alkyl substituiert ist,
4) -(C₀-C₄)-Alkylen-(C₃-C₁₂)-Cycloalkyl, wobei Cycloalkyl unsubstituiert oder unabhängig voneinander ein-, zwei-, drei- oder vierfach durch R13, Halogen, -C(O)-O-R13, -(C₁-C₄)-Alkyl-O-R13, -O-(C₁-C₄)-Alkyl oder -(C₀-C₄)-Alkylen-Phenyl substituiert ist,
5) -(C₀-C₄)-Alkylen-C(O)-N(R13)₂ oder
6) -(C₀-C₄)-Alkylen-Indolyl, stehen,
R13 für
1) Wasserstoffatom,
2) -(C₁-C₄)-Alkyl,
3) -(C₀-C₄)-Alkylen-C(O)-O-R14,
4) -(C₀-C₄)-Alkylen-C(O)-R14 oder
5) -(C₀-C₄)-Alkylen-O-R14 steht,
R14 für Wasserstoffatom, -(C₁-C₄)-Alkyl, -NH₂ oder -OH, steht und
R15 für Wasserstoffatom, -(C₁-C₄)-Alkyl, -O-CF₃, -NH₂, -OH, -CF₃ oder Halogen steht.

2. Verbindung der Formel I gemäß Anspruch 1, wobei
R1 für
1) Wasserstoffatom oder
2) -(C₁-C₄)-Alkyl, steht,
R2 für
1) -(C₁-C₆)-Alkylen-NH₂,
2) -(C₀-C₄)-Alkylen-Pyridyl-NH₂,
3) -(C₀-C₄)-Alkylen-Piperidinyl-NH₂,
4) -(C₀-C₄)-Alkylen-Thiazolyl-NH2,
5) -(C₁-C₆)-Alkylen-NH-C(=NH)-NH₂,
6) -(C₀-C₄)-Alkylen-(C₃-C₈)-Cycloalkyl-NH₂,
7) -(C₁-C₆)-Alkylen-NH-C(=NH)-(C₁-C₄)-Alkyl,
8) -(C₀-C₄)-Alkylen-O-NH-C(=NH)-NH₂,
9) -(C₁-C₆)-Alkylen-NH-C(O)-O-(C₁-C₄)-Alkylen-Aryl, wobei Aryl unsubstituiert oder durch -NH₂ substituiert ist oder durch -NH₂ und ein-, zwei- oder dreifach durch R15 substituiert ist,
10) -(C₀-C₄)-Alkylen-NH-C(O)-(C₁-C₄)-Alkyl,
11) -(C₀-C₄)-Alkylen-(C₆-C₁₄)-Aryl, wobei Aryl unsubstituiert oder durch -NH₂ substituiert ist oder durch -NH₂ und ein-, zwei- oder dreifach durch R15 substituiert ist, oder
12) -(C₁-C₄)-Alkylen-SOₓ-(C₁-C₄)-Alkylen-NH₂ worin x die ganze Zahl Null, 1 oder 2 bedeutet, steht,
R3 für
1) -(C₁-C₄)-Alkyl,
2) -(C₀-C₄)-Alkylen-(C₃-C₈)-Cycloalkyl,
3) -(C₁-C₆)-Alkylen-Aryl, wobei Aryl unabhängig voneinander ein-, zwei- oder dreifach durch R15 substituiert ist,
4) -(C₁-C₆)-Alkylen-NH-C(O)-O-(C₁-C₄)-Alkylen-Aryl, wobei Aryl unabhängig voneinander ein-, zwei- oder dreifach durch R15 substituiert ist,
5) -(C₁-C₆)-Alkylen-NH-PG,
6) -(C₁-C₆)-Alkylen-O-PG,
7) -(C₁-C₆)-Alkyl, oder
8) Wasserstoffatom, steht,
wobei PG für t-Butyl-, t-Butyloxycarbonyl oder Benzyloxycarbonyl steht,
R4 für-N(R6)₂, steht,
wobei R6 gleich oder verschieden sind und unabhängig voneinander für
1) Wasserstoffatom,
2) -(C₁-C₆)-Alkyl,
3) -(C₀-C₄)-Alkylen-(C₃-C₁₂)-Cycloalkyl, wobei Cycloalkyl unsubstituiert oder unabhängig voneinander ein-, zwei-, drei- oder vierfach durch R11, Halogen, -C(O)-O-R11, -(C₁-C₄)-Alkyl-O-R11 oder -O-(C₁-C₄)-Alkyl substituiert ist,
4) -(C₀-C₄)-Alkylen-C(R11)(R12)-(C₃-C₁₂)-Cycloalkyl, wobei Cycloalkyl unsubstituiert oder unabhängig voneinander ein-, zwei- oder dreifach durch R11, Halogen, -C(O)-O-R11, -(C₁-C₄)-Alkyl-O-R11 oder -O-(C₁-C₄)-Alkyl substituiert ist,
5) -(C₀-C₄)-Alkylen-Het, wobei unter Het ein 4- bis 15-gliedriges heterocyclisches Ringsystem verstanden wird mit 4 bis 15 Ringatomen, die in ein, zwei oder drei miteinander verbundenen Ringsystemen vorliegen und die ein, zwei, drei oder vier gleiche oder verschiedene Heteroatome aus der Reihe Sauerstoff, Stickstoff oder Schwefel enthalten, wobei Het oder Alkylen unsubstituiert oder unabhängig voneinander ein-, zwei- oder dreifach durch R11, Halogen, -C(O)-O-R11, -(C₁-C₄)-Alkyl-O-R11 oder -O-(C₁-C₄)-Alkyl substituiert sind,
6) -(C₀-C₆)-Alkylen-Aryl, wobei Aryl oder Alkylen unsubstituiert oder unabhängig voneinander ein-, zwei- oder dreifach durch R11, Halogen, -C(O)-O-R11, -(C₀-C₄)-Alkyl-O-R11 oder-O-(C₁-C₄)-Alkyl substituiert sind,
7) -(C₀-C₄)-Alkylen-C(R11)(R12)-Aryl, wobei Aryl oder Alkylen unsubstituiert oder unabhängig voneinander ein-, zwei- oder dreifach durch R11, Halogen, -C(O)-O-R11, -(C₀-C₄)-Alkyl-O-R11 oder -O-(C₁-C₄)-Alkyl substituiert sind,
8) 1,2,3,4-Tetrahydro-naphthalenyl,
9) -(C₀-C₄)-Alkylen-CH(R11)-C(O)-NH₂,
10) -(C₀-C₄)-Alkylen-CH(R11)-C(O)-NH-(C₁-C₄)-Alkyl,
11) -(C₀-C₄)-Alkylen-CH(R11)-C(O)-NH-CH(R12)-R13,
12) -(C₀-C₆)-Alkylen-C(O)-O-R11, wobei Alkylen unsubstituiert oder unabhängig voneinander ein- oder zweifach durch R11, Halogen, -C(O)-O-R11, -(C₁-C₄)-Alkyl-O-R11 oder -O-(C₁-C₄)-Alkyl substituiert ist,
13) -(C₀-C₄)-Alkylen-C(R11)(R12)-C(O)-O-R11, oder
14) -(C₁-C₃)-Fluoralkyl, stehen,
oder die beiden Reste R6 zusammen mit dem N-Atom, an das sie gebunden sind, einen mono- oder bicyclischen Ring mit 4 bis 9 Ringatomen bilden, der gesättigt, teilweise gesättigt oder aromatisch ist, wobei der Ring unsubstituiert oder ein- oder zweifach durch -(C₁-C₄)-Alkyl, -C(O)-O-R11, Halogen, -(C₁-C₄)-Alkyl-O-R11 oder Phenyl substituiert ist,
R7 für Wasserstoffatom oder -(C₁-C₄)-Alkyl steht,
R9 für Wasserstoffatom oder -(C₁-C₄)-Alkyl steht,
R11 und R12 gleich oder verschieden sind und unabhängig voneinander für
1) Wasserstoffatom,
2) -(C₁-C₄)-Alkyl,
3) -(C₀-C₄)-Alkylen-Phenyl, wobei Phenyl unsubstituiert oder unabhängig voneinander ein-, zwei- oder dreifach durch Halogen, -OH oder -O-(C₁-C₄)-Alkyl substituiert ist,
4) -(C₀-C₄)-Alkylen-(C₃-C₁₂)-Cycloalkyl, wobei Cycloalkyl unsubstituiert oder unabhängig voneinander ein-, zwei-, drei- oder vierfach durch R13, Halogen, -C(O)-O-R13, -(C₁-C₄)-Alkyl-O-R13, -O-(C₁-C₄)-Alkyl oder -(C₀-C₄)-Alkylen-Phenyl substituiert ist,
5) -(C₀-C₄)-Alkylen-C(O)-N(R13)₂ oder
6) -(C₀-C₄)-Alkylen-Indolyl, stehen,
R13 für
1) Wasserstoffatom,
2) -(C₁-C₄)-Alkyl,
3) -(C₀-C₄)-Alkylen-C(O)-O-R14,
4) -(C₀-C₄)-Alkylen-C(O)-R14 oder
5) -(C₀-C₄)-Alkylen-O-R14 steht,
R14 für Wasserstoffatom, -(C₁-C₄)-Alkyl, -NH₂ oder-OH, steht und
R15 für Wasserstoffatom, -(C₁-C₄)-Alkyl, -O-CF₃, -NH₂, -OH, -CF₃ oder Halogen steht.

3. Verbindung der Formel I gemäß Anspruch 1 oder 2, wobei
R1 für
1) Wasserstoffatom oder
2) -(C₁-C₄)-Alkyl, steht,
R2 für
1) -(C₁-C₆)-Alkylen-NH₂,
2) -(C₁-C₄)-Alkylen-Pyridyl-NH₂,
3) -(C₁-C₄)-Alkylen-Piperidinyl-NH₂,
4) -(C₁-C₆)-Alkylen-NH-C(=NH)-NH₂,
5) -(C₀-C₄)-Alkylen-(C₃-C₆)-Cycloalkyl-NH₂,
6) -(C₁-C₆)-Alkylen-NH-C(=NH)-(C₁-C₄)-Alkyl,
7) -(C₁-C₄)-Alkylen-O-NH-C(=NH)-NH₂,
8) -(C₁-C₆)-Alkylen-NH-C(O)-O-(C₁-C₄)-Alkylen-Phenyl, wobei Phenyl unsubstituiert oder durch -NH₂ substituiert ist oder durch -NH₂ und ein-, zwei- oder dreifach durch R15 substituiert ist,
9) -(C₁-C₄)-Alkylen-NH-C(O)-(C₁-C₆)-Alkyl,
10) -(C₁-C₄)-Alkylen-Phenyl, wobei Phenyl unsubstituiert oder durch -NH₂ substituiert ist oder durch -NH₂ und ein-, zwei- oder dreifach durch R15 substituiert ist,
11) -(C₁-C₄)-Alkylen-SO₂-(C₁-C₄)-Alkylen-NH₂, oder
12) -(C₁-C₄)-Alkylen-S-(C₁-C₄)-Alkylen-NH₂, steht,
R3 für
1) -(C₁-C₄)-Alkyl,
2) -(C₁-C₄)-Alkylen-(C₃-C₆)-Cycloalkyl,
3) -(C₁-C₄)-Alkylen-Phenyl, wobei Phenyl unabhängig voneinander ein-, zwei- oder dreifach durch R15 substituiert ist,
4) -(C₁-C₆)-Alkylen-NH-C(O)-O-(C₁-C₄)-Alkylen-Phenyl, wobei Phenyl unabhängig voneinander ein-, zwei- oder dreifach durch R15 substituiert ist,
5) Wasserstoffatom steht,
R4 für-N(R6)₂, steht,
wobei R6 gleich oder verschieden sind und unabhängig voneinander für
1) Wasserstoffatom,
2) -(C₁-C₄)-Alkyl,
3) -(C₀-C₄)-Alkylen-(C₃-C₁₂)-Cycloalkyl, wobei Cycloalkyl ausgewählt ist aus der Gruppe Cyclohexyl, Cyclopentyl, Cyclobutyl, Cyclopropyl, Adamantanyl, 1,7,7-Trimethyl-bicyclo[3.1.1]heptanyl, Decahydro-naphthalenyl, Tetrahydronaphthalenyl, Octahydro-4,7-methano-indenyl oder Bicyclo[2.2.1]heptanyl und worin Cycloalkyl unsubstituiert oder unabhängig voneinander ein-, zwei-, drei- oder vierfach substituiert ist durch -(C₁-C₄)-Alkyl, -C(O)-O-R11 oder -(C₁-C₄)-Alkylen-Phenyl, wobei Phenyl unsubstituiert oder durch Halogen substituiert ist,
4) -(C₀-C₄)-Alkylen-C(R11)(R12)-(C₃-C₁₂)-Cycloalkyl, wobei Cycloalkyl ausgewählt ist aus der Gruppe Cyclohexyl, Cyclopentyl, Cyclobutyl, Cyclopropyl, Adamantanyl, 1,7,7-Trimethyl-bicyclo[3.1.1]heptanyl, Decahydronaphthalen, Tetrahydronaphthalenyl, Octahydro-4,7-methano-indenyl oder Bicyclo[2.2.1]heptanyl und worin Cycloalkyl unsubstituiert oder unabhängig voneinander ein-, zwei-, drei- oder vierfach substituiert ist durch -(C₁-C₄)-Alkyl, -C(O)-O-R11 oder-(C₁-C₄)-Alkylen-Phenyl, wobei Phenyl unsubstituiert oder durch Halogen substituiert ist,
5) -(C₀-C₄)-Alkylen-Het, wobei Het ausgewählt ist aus der Gruppe Acridinyl, Azepinyl, Azetidinyl, Aziridinyl, Benzimidazalinyl, Benzimidazolyl, Benzo[1,3]dioxolyl, Benzofuranyl, Benzothiofuranyl, Benzothiophenyl, Benzoxazolyl, Benzthiazolyl, Benztriazolyl, Benztetrazolyl, Benzisoxazolyl, Benzisothiazolyl, Carbazolyl, 4aH-Carbazolyl, Carbolinyl, Chinazolinyl, Chinolinyl, 4H-Chinolizinyl, Chinoxalinyl, Chinuclidinyl, Chromanyl, Chromenyl, Cinnolinyl, Deca-hydrochinolinyl, Dibenzofuranyl, Dibenzothiophenyl, Dihydrofuran[2,3-b]-tetrahydrofuranyl, Dihydrofuranyl, Dioxolyl, Dioxanyl, 2H, 6H-1,5,2-Dithiazinyl, Furanyl, Furazanyl, Imidazolidinyl, Imidazolinyl, Imidazolyl, 1H-Indazolyl, Indolinyl, Indolizinyl, Indolyl, 3H-Indolyl, Isobenzofuranyl, Isochromanyl, Isoindazolyl, Isoindolinyl, Isoindolyl, Isochinolinyl (Benzimidazolyl), Isothiazolidinyl, 2-Isothiazolinyl, Isothiazolyl, Isoxazolyl, Isoxazolidinyl, 2-Isoxazolinyl, Morpholinyl, Naphthyridinyl, Octahydroisochinolinyl, Oxadiazolyl, 1,2,3-Oxadiazolyl, 1,2,4-Oxadiazolyl, 1,2,5-Oxadiazolyl, 1,3,4-Oxadiazolyl, Oxazolidinyl, Oxazolyl, Oxazolidinyl, Oxothiolanyl, Pyrimidinyl, Phenanthridinyl, Phenanthrolinyl, Phenazinyl, Phenothiazinyl, Phenoxathünyl, Phenoxazinyl, Phthalazinyl, Piperazinyl, Piperidinyl, Pteridinyl, Purynyl, Pyranyl, Pyrazinyl, Pyroazolidinyl, Pyrazolinyl, Pyrazolyl, Pyridazinyl, Pryidooxazolyl, Pyridoimidazolyl, Pyridothiazolyl, Pyridothiophenyl, Pyridinyl, Pyridyl, Pyrimidinyl, Pyrrolidinyl, Pyrrolinyl, 2H-Pyrrolyl, Pyrrolyl, Tetrahydrofuranyl, Tetrahydroisochinolinyl, Tetrahydrochinolinyl, Tetrahydropyridinyl, 6H-1,2,5-Thiadazinyl, 1,2,3-Thiadiazolyl, 1,2,4-Thiadiazolyl, 1,2,5-Thiadiazolyl, 1,3,4-Thiadiazolyl, Thianthrenyl, Thiazolyl, Thienyl, Thienoimidazolyl, Thienooxazolyl, Thienopyridin, Thienothiazolyl, Thiomorpholinyl, Thiophenyl, Triazinyl, 1,2,3-Triazolyl, 1,2,3-Triazolyl, 1,2,4-Triazolyl, 1,2,5-Triazolyl, 1,3,4-Triazolyl und Xanthenyl, wobei Het oder Alkylen unsubstituiert oder unabhängig voneinander ein- oder zweifach durch -(C₁-C₄)-Alkyl substituiert ist
6) -(C₁-C₆)-Alkylen-Phenyl, wobei Phenyl oder Alkylen unsubstituiert oder unabhängig voneinander ein- oder zweifach durch Halogen, Phenyl, -C(O)-O-R11, -(C₁-C₄)-Alkyl-O-R11, -O-(C₁-C₄)-Alkyl oder -(C₁-C₄)-Alkyl substituiert sind,
7) -(C₀-C₄)-Alkylen-C(R11)(R12)-Phenyl, wobei Phenyl unsubstituiert oder unabhängig voneinander ein-, zwei- oder dreifach durch Phenyl oder Fluor substituiert ist,
8) 1,2,3,4-Tetrahydro-naphthalenyl,
9) -(C₀-C₄)-Alkylen-CH(R11)-C(O)-NH2,
10) -(C₀-C₄)-Alkylen-CH(R11)-C(O)-NH-(C1-C4)-Alkyl,
11) -(C₀-C₄)-Alkylen-CH(R11)-C(O)-NH-CH(R12)-R13,
12) -(C₁-C₆)-Alkylen-C(O)-O-R11, wobei Alkylen unsubstituiert oder unabhängig voneinander ein- oder zweifach durch Halogen, Phenyl, -C(O)-O-R11, -(C₁-C₄)-Alkyl-O-R11, -O-(C₁-C₄)-Alkyl oder-(C₁-C₄)-Alkyl substituiert ist,
13) -(C₀-C₄)-Alkylen-C(R11)(R12)-C(O)-O-R11, oder
14) -(C₁-C₃)-Fluoralkyl, stehen,
oder die beiden Reste R6 zusammen mit dem N-Atom an das sie gebunden sind einen Mono- oder Bicyclischen Ring bilden ausgewählt aus der Gruppe Pyrrolidin, Piperidin, 2-Aza-bicyclo[3.2.2]nonan und 7-Aza-bicyclo[2.2.1]heptan, wobei der Ring unsubstituiert oder ein- oder zweifach durch -(C₁-C₄)-Alkyl, -C(O)-O-R11, -(C₁-C₄)-Alkyl-O-R11 oder Phenyl substituiert ist,
R7 für Wasserstoffatom oder -(C₁-C₄)-Alkyl steht,
R9 für Wasserstoffatom oder -(C₁-C₄)-Alkyl steht,
R11 und R12 gleich oder verschieden sind und unabhängig voneinander für
1) Wasserstoffatom,
2) -(C₁-C₄)-Alkyl,
3) -(C₀-C₄)-Alkylen-Phenyl, wobei Phenyl unsubstituiert oder unabhängig voneinander ein-, zwei- oder dreifach durch -OH, Halogen oder -O-(C₁-C₄)-Alkyl substituiert ist,
4) -(C₀-C₄)-Alkylen-(C₃-C₁₂)-Cycloalkyl, wobei Cycloalkyl ausgewählt ist aus der Gruppe Cyclohexyl, Cyclopentyl, Cyclobutyl, Cyclopropyl, Adamantanyl, 1,7,7-Trimethyl-bicyclo[3.1.1]heptanyl, Decahydro-naphthalenyl, Tetrahydronaphthalenyl, Octahydro-4,7-methano-indenyl oder Bicyclo[2.2.1]heptanyl und worin Cycloalkyl unsubstituiert oder unabhängig voneinander ein-, zwei-, drei- oder vierfach durch -(C₁-C₄)-Alkyl, -C(O)-O-R13 oder Phenyl substituiert ist, oder
5) -(C₀-C₄)-Alkylen-Indolyl, stehen,
R13 für
1) Wasserstoffatom,
2) -(C₁-C₄)-Alkyl,
3) -(C₀-C₄)-Alkylen-C(O)-O-R14,
4) -(C₀-C₄)-Alkylen-C(O)-R14 oder
5) -(C₀-C₄)-Alkylen-O-R14 steht, und
R14 für Wasserstoffatom, -(C₁-C₄)-Alkyl, -NH₂ oder -OH steht und R15 für Wasserstoffatom, -(C₁-C₄)-Alkyl, -O-CF₃, -NH₂, -OH, -CF₃ oder Halogen steht.

4. Verbindung der Formel I gemäß der Ansprüche 1 bis 3, wobei
R1 für
1) Wasserstoffatom oder
2) -(C₁-C₄)-Alkyl, steht,
R2 für
1) -(C₁-C₆)-Alkylen-NH₂,
2) -(C₁-C₄)-Alkylen-Pyridyl-NH₂,
3) -(C₁-C₄)-Alkylen-Piperidinyl-NH₂,
4) -(C₁-C₄)-Alkylen-NH-C(=NH)-NH₂,
5) -(C₁-C₆)-Alkylen-NH-C(=NH)-(C₁-C₄)-Alkyl,
6) -(C₁-C₄)-Alkylen-(C₃-C₆)-Cycloalkyl-NH₂,
7) -(C₁-C₄)-Alkylen-O-NH-C(=NH)-NH₂,
8) -(C₁-C₆)-Alkylen-NH-C(O)-O-(C₁-C₄)-Alkylen-Phenyl,
9) -(C₁-C₄)-Alkylen-NH-C(O)-(C₁-C₆)-Alkyl,
10) -(C₁-C₄)-Alkylen-Phenyl-NH₂,
11) -(C₁-C₄)-Alkylen-SO₂-(C₁-C₄)-Alkylen-NH₂, oder
12) -(C₁-C₄)-Alkylen-S-(C₁-C₄)-Alkylen-NH₂, steht,
R3 für
1) -(C₁-C₄)-Alkyl,
2) -(C₁-C₄)-Alkylen-(C₃-C₆)-Cycloalkyl,
3) -(C₁-C₄)-Alkylen-Phenyl, wobei Phenyl unsubstituiert oder durch -OH substituiert ist,
4) -(C₁-C₆)-Alkylen-NH-C(O)-O-(C₁-C₄)-Alkylen-Phenyl,
5) Wasserstoffatom steht,
R4 für-N(R6)₂, steht,
wobei R6 gleich oder verschieden sind und unabhängig voneinander für
1) Wasserstoffatom,
2) -(C₁-C₆)-Alkyl,
3) -(C₀-C₄)-Alkylen-(C₃-C₈)-Cycloalkyl, wobei Cycloalkyl ausgewählt ist aus der Gruppe Cyclohexyl, Cyclopentyl, Cyclopropyl, Adamantanyl, 1,7,7-Trimethyl-bicyclo[3.1.1]heptanyl, Decahydro-naphthalen, Octahydro-4,7-methano-indenyl oder Bicyclo[2.2.1]heptanyl und worin Cycloalkyl unsubstituiert oder unabhängig voneinander ein-, zwei- oder dreifach durch-(C₁-C₄)-Alkyl oder Phenyl substituiert ist,
4) -C(R11)(R12)-Adamantanyl,
5) -CH(R11)-C(O)-NH-CH(R12)-R13,
6) -(C₀-C₄)-Alkylen-Het, wobei Het ausgewählt ist aus der Gruppe Benzimidazolyl, Isoxazolyl, Piperidin, Pyridin, Pyrrolidinyl, Thiophenyl und Benzo[1,3]dioxol,
7) 1,2,3,4-Tetrahydro-naphthalenyl,
8) -(C₀-C₄)-Alkylen-C(R11)(R12)-Phenyl, wobei Phenyl unsubstituiert oder unabhängig voneinander ein-, zwei- oder dreifach durch Phenyl oder Fluor substituiert ist,
9) -CH(R11)-C(O)-NH₂,
10) -CH(R11)-C(O)-NH-CH(R12)-CH₂-OH,
11) -(C₁-C₆)-Alkylen-Phenyl, wobei Phenyl oder Alkylen unsubstituiert oder unabhängig voneinander ein- oder zweifach durch Chlor, Fluor, -C(O)-O-R11, -(C₁-C₄)-Alkyl-O-R11, -O-(C₁-C₄)-Alkyl, Phenyl oder -(C₁-C₄)-Alkyl substituiert sind,
12) -CH(R11)-C(O)-NH-(C₁-C₄)-Alkyl,
13) -(C₀-C₄)-Alkylen-C(R11)(R12)-Bicyclo[3.1.1]heptanyl, wobei Bicyclo[3.1.1]heptanyl unsubstituiert oder ein- bis vierfach durch -(C₁-C₄)-Alkyl substituiert ist,
14) -(C₁-C₆)-Alkylen-C(O)-O-R11, wobei Alkylen unsubstituiert oder unabhängig voneinander ein- oder zweifach durch Chlor, Fluor, -C(O)-O-R11, -(C₁-C₄)-Alkyl-O-R11, -O-(C₁-C₄)-Alkyl, Phenyl oder -(C₁-C₄)-Alkyl substituiert ist,
15) -(C₀-C₄)-Alkylen-C(R11)(R12)-C(O)-O-R11, oder
16) -CH₂-CF₂-CF₃, stehen,
oder die beiden Reste R6 zusammen mit dem N-Atom an das sie gebunden sind einen Mono- oder Bicyclischen Ring bilden, ausgewählt aus der Gruppe Pyrrolidine, 2-aza-bicyclo[3.2.2]nonan und 7-aza-bicyclo[2.2.1]heptan, wobei der Ring unsubstituiert oder ein- oder zweifach durch -(C₁-C₄)-Alkyl, -C(O)-O-R11, -(C₁-C₄)-Alkyl-O-R11 oder Phenyl substituiert ist,
R7 für Wasserstoffatom oder -(C₁-C₄)-Alkyl steht,
R9 für Wasserstoffatom oder -(C₁-C₄)-Alkyl steht,
R11 und R12 gleich oder verschieden sind und unabhängig voneinander für
1) Wasserstoffatom,
2) -(C₁-C₄)-Alkyl,
3) -(C₀-C₄)-Alkylen-Phenyl, wobei Phenyl unsubstituiert oder unabhängig voneinander ein-, zwei- oder dreifach durch -OH, Halogen oder -O-(C₁-C₄)-Alkyl substituiert ist,
4) -(C₀-C₄)-Alkylen-(C₃-C₁₂)-Cycloalkyl, wobei Cycloalkyl ausgewählt ist aus der Gruppe Cyclohexyl, Cyclopentyl, Cyclobutyl, Cyclopropyl, Adamantanyl, 1,7,7-Trimethyl-bicyclo[3.1.1]heptanyl, Decahydro-naphthalenyl, Octahydro-4,7-methano-indenyl oder Bicyclo[2.2.1]heptanyl und worin Cycloalkyl unsubstituiert oder unabhängig voneinander ein-, zwei-, drei- oder vierfach durch -(C₁-C₄)-Alkyl, -C(O)-O-R13 oder Phenyl substituiert ist, oder
5) -(C₀-C₄)-Alkylen-Indolyl, stehen,
R13 für
1) Wasserstoffatom,
2) -(C₁-C₄)-Alkyl,
3) -(C₀-C₄)-Alkylen-C(O)-O-R14,
4) -(C₀-C₄)-Alkylen-C(O)-R14 oder
5) -(C₀-C₄)-Alkylen-O-R14 steht,
R14 für Wasserstoffatom, -(C₁-C₄)-Alkyl, -NH₂ oder -OH steht und R15 für Wasserstoffatom, -(C₁-C₄)-Alkyl, -O-CF₃, -NH₂, -OH, -CF₃ oder Halogen steht.

5. Verbindung der Formel I gemäß einem oder mehreren der Ansprüche 1 bis 4, **dadurch gekennzeichnet, dass** es die Verbindung der Formel I (S)-6-Amino-2-(3-{(S)-1-[(S)-1-((S)-1-methoxycarbonyl-2-methyl-propylcarbamoyl)-2-methyl-propylcarbamoyl]-2-phenyl-ethyl}-sulfamidyl)-hexansäure, (S)-6-Amino-2-{3-[(R)-1-(bicyclo[2.2.1]hept-2-ylcarbamoyl)-2-cyclohexyl-ethyl]-sulamidyl}-hexansäure, (S)-6-Amino-2-[3-((S)-1-cyclohexylcarbamoyl-2-phenyl-ethyl)-sulfamidyl]-hexansäure, (S)-6-Acetimidoylamino-2-{[(S)-2-cyclohexyl-1-((1R,2S,4R)-1,7,7-trimethyl-bicyclo[2.2.1]hept-2-ylcarbamoyl)-ethylsulfamidyl]}-hexansäure, 3-(6-Amino-pyridin-3-yl)-2-[(S)-2-cyclohexyl-1-((1R,2S,4R)-1,7,7-trimethyl-bicyclo[2.2.1]hept-2-ylcarbamoyl)-ethylsulfamid-yl]-propionsäureethylester, (S)-6-Amino-2-{[(S)-1-((1R,2S,4R)-1,7,7-trimethyl-bicyclo[2.2.1]hept-2-ylcarbamoyl)-propylsulfamidyl]-}-hexansäure, (S)-2-{[(S)-2-Cyclohexyl-1-((1R,2S,4R)-1,7,7-trimethyl-bicyclo[2.2.1]hept-2-ylcarbamoyl)-ethylsulfamidyl]-}-5-guanidino-pentansäure, (S)-6-Amino-2-{[(S)-2-cyclobutyl-1-((1R,2S,4R)-1,7,7-trimethyl-bicyclo[2.2.1]hept-2-ylcarbamoyl)-ethylsulf-amidyl]}-hexansäure, (S)-5-Amino-2-({2-cyclohexyl-1-[(R)-(1,2,3,4-tetrahydronaphthalen-2-yl)carbamoyl]-ethylsulfamidyl})-pentansäure, (S)-2-{[(S)-2-Cyclohexyl-1-((1R,2S,4R)-1,7,7-trimethyl-bicyclo[2.2.1]hept-2-ylcarbamoyl)-ethylsulfamidyl]}-6-hexanoylamino-hexansäure-ethylester, (S)-6-Amino-2-{[(S)-1-((1R,2S,4R)-1,7,7-trimethyl-bicyclo[2.2.1]hept-2-ylcarbamoyl)-butylsulfamidyl]}-hexansäure, (S)-6-Amino-2-{[(S)-3-cyclohexyl-1-((1R,2S,4R)-1,7,7-trimethyl-bicyclo[2.2.1]hept-2-ylcarbamoyl)-propylsulf-amidyl]-methyl}-hexansäure, (S)-6-Amino-2-{[(S)-2-cyclohexyl-1-(decahydro-naphthalen-2-ylcarbamoyl)-ethylsulfamidyl]}-hexansäure, (S)-2-{[(S)-1-(Adamantan-1-ylcarbamoyl)-2-cyclohexyl-ethylsulfamidyl]}-6-amino-hexan-säure, (S)-2-[(S)-2-Cyclohexyl-1-((1R,2S,4R)-1,7,7-trimethyl-bicyclo[2.2.1]-hept-2-ylcarbamoyl)-ethylsulfamidyl]-3-pyridin-3-yl-propionsäure, (S)-2-[(S)-2-Cyclohexyl-1-((1R,2S,4R)-1,7,7-trimethyl-bicyclo[2.2.1]hept-2-ylcarbamoyl)-ethylsulfamidyl]-3-pyridin-4-yl-propionsäure, (S)-6-Amino-2-{[(S)-2-cyclohexyl-1-((1S,2S,3S,5R)-2,6,6-trimethyl-bicyclo[3.1.1]hept-3-ylcarbamoyl)-ethylsulfamidyl]}-hexansäure, (S)-6-Amino-2-{[(S)-2-cyclohexyl-1-(3,3,5-trimethyl-cyclohexylcarbamoyl)-ethylsulfamidyl]}-hexansäure, (S)-6-Amino-2-{[(S)-1-(4-tert-butyl-cyclohexylcarbamoyl)-2-cyclohexyl-ethylsulfamidyl]}-hexansäure, (S)-6-Amino-2-{[(S)-2-cyclohexyl-1-(3-methyl-cyclohexylcarbamoyl)-ethylsulfamidyl]}-hexansäure, (S)-6-Amino-2-{[(S)-2-cyclohexyl-1-((1R,2S,4R)-1,7,7-trimethyl-bicyclo[2.2.1]hept-2-ylcarbamoyl)-ethylsulfamidyl]}-hexansäure, 3-(6-Amino-pyridin-3-ylmethyl)-2-[(S)-2-cyclohexyl-1-((1R,2S,4R)-1,7,7-trimethyl-bicyclo[2.2.1]hept-2-yl-carbamoyl)-ethylsulfamidyl]-propionsäure, 2-[(S)-2-Cyclohexyl-1-((1R,2S,4R)-1,7,7-trimethyl-bicyclo[2.2.1]hept-2-ylcarbamoyl)-ethylsulfamidyl]-3-piperidin-4-yl-propionsäure, (S)-3-(4-Amino-phenyl)-2-[(S)-2-cyclohexyl-1-((1R,2S,4R)-1,7,7-trimethyl-bicyclo[2.2.1]hept-2-ylcarbamoyl)-ethylsulfamidyl]-propionsäure, (S)-6-Amino-2-[((S)-1-cyclohexylmethyl-2-oxo-2-piperidin-1-yl-ethylsulfamidyl)]-hexansäure, (S)-5-Amino-2-{[(S)-2-cyclohexyl-1-((1R,2S,4R)-1,7,7-trimethyl-bicyclo[2.2.1]hept-2-ylcarbamoyl)-ethylsulfamidyl]}-pentansäure, (S)-6-Amino-2-{[(S)-2-cyclohexyl-1-((S)-1-isobutylcarbamoyl-2-methyl-propylcarbamoyl)-ethylsulfamidyl]}-hexansäure (S)-6-Amino-2-{[(S)-1-((S)-1-isobutylcarbamoyl-2-methyl-propylcarbamoyl)-2-phenyl-ethylsulfamidyl]}-hexansäure, (S)-6-Amino-2-[((S)-2-cyclohexyl-1-isobutylcarbamoyl-ethylsulfamidyl)]-hexansäure, (S)-6-Amino-2-{[(S)-1-((1R,2R,4S)-bicyclo[2.2.1]hept-2-ylcarbamoyl)-2-cyclohexyl-ethylsulfamidyl]}-hexansäure, (S)-6-Amino-2-{[(S)-2-cyclohexyl-1-((1R,2S,4R)-1,7,7-trimethyl-bicyclo[2.2.1]hept-2-ylcarbamoyl)-ethylsulfamidyl]}-hexansäure, (S)-6-Amino-2-{[(S)-1-((1S,4R)-bicyclo[2.2.1]hept-2-ylcarbamoyl)-2-cyclohexyl-ethylsulf-amidyl]}-hexansäure, (S)-6-Amino-2-{[(S)-2-cyclohexyl-1-(octahydro-4,7-methano-inden-5-ylcarbamoyl)-ethylsulfamidyl]}-hexansäure, (S)-6-Amino-2-[((S)-1-tert-butylcarbamoyl-2-cyclohexyl-ethylsulfamidyl)]-hexansäure, (S)-2-{[(S)-1-(Adamantan-1-ylcarbamoyl)-2-phenyl-ethylsulfamidyl]}-6-amino-hexansäure, (S)-6-Amino-2-{[(S)-1-((1S,4R)-bicyclo[2.2.1]hept-2-ylcarbamoyl)-2-phenyl-ethylsulfamidyl]}-hexansäure, (S)-2-{[(S)-1-(Adamantan-1-ylcarbamoyl)-2-(4-hydroxy-phenyl)-ethylsulfamidyl]}-6-amino-hexansäure, (S)-6-Amino-2-{[(S)-2-phenyl-1-((1R,2S,4R)-1,7,7-trimethyl-bicyclo[2.2.1]hept-2-ylcarbamoyl)-ethylsulfamidyl]}-hexansäure, (S)-6-Amino-2-({[(S)-cyclohexyl-(1,7,7-trimethyl-bicyclo[2.2.1]hept-2-ylcarbamoyl)-methyl]-sulfamidyl})-hexansäure, (S)-6-Amino-2-{[(S)-2-cyclohexyl-1-((1R,2R,4R)-1,7,7-trimethyl-bicyclo[2.2.1]hept-2-ylcarbamoyl)-ethylsulfamidyl]}-hexansäure, (S)-6-Amino-2-{[(S)-2-cyclopropyl-1-((1R,2R,4R)-1,7,7-trimethyl-bicyclo[2.2.1]hept-2-ylcarbamoyl)-ethylsulfamidyl]}-hexansäure, (S)-6-Amino-2-{[(S)-2-cyclohexyl-1-(3,5-dimethyl-adamantan-1-ylcarbamoyl)-ethylsulf-amidyl]}-hexansäure, (S)-6-Amino-2-{[(S)-2-cyclohexyl-1-(3-isopropyl-adamantan-1-ylcarbamoyl)-ethylsulfamidyl]}-hexansäure, (S)-6-Amino-2-{[(S)-2-cyclohexyl-1 -((1R,2R,4R)-1,7,7-trimethyl-bicyclo[2.2.1]hept-2-ylcarbamoyl)-ethylsulfamidyl]}-hexansäure-tert-butylester oder (S)-2-{[(S)-1-(Adamantan-1-ylcarbamoyl)-3-methylbutylsulfamidyl]}-6-amino-hexansäure ist.

6. Verfahren zur Herstellung der Verbindung der Formel I gemäß einem oder mehreren der Ansprüche 1 bis 5, **dadurch gekennzeichnet, dass** man
a) eine Verbindung der Formel II 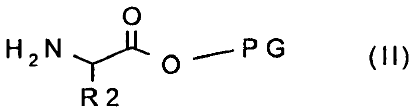 wobei R2 und PG die in der Verbindung der Formel I genannten Bedeutungen haben, mit einer Verbindung der Formel IX, 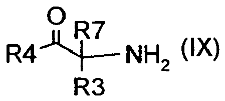 wobei R3, R4, R7 und PG die in der Verbindung der Formel I genannten Bedeutungen haben, zu einer Verbindung der Formel X umgesetzt, 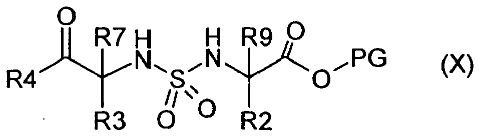 wobei R2, R3, R4, R7, R9 und PG die in der Verbindung der Formel I genannten Bedeutungen haben, und dann in eine Verbindung der Formel I überführt, oder
b) eine nach den Verfahren a) hergestellte Verbindung der Formel I, oder eine geeignete Vorstufe der Formel I, die aufgrund ihrer chemischen Struktur in enantiomeren Formen auftritt, durch Salzbildung mit enantiomerenreinen Säuren oder Basen, Chromatographie an chiralen Stationärphasen oder Derivatisierung mittels chiraler enantiomerenreinen Verbindungen wie Aminosäuren, Trennung der somit erhaltenen Diastereomeren, und Abspaltung der chiralen Hilfsgruppen in die reinen Enantiomeren auftrennt, oder
c) die nach den Verfahren a) oder b) hergestellte Verbindung der Formel I entweder in freier Form isoliert oder im Falle des Vorliegens von sauren oder basischen Gruppen in physiologisch verträgliche Salze umwandelt.

7. Arzneimittel, **gekennzeichnet durch** einen wirksamen Gehalt an mindestens einer Verbindung der Formel I gemäß einem oder mehreren der Ansprüche 1 bis 5 zusammen mit einem pharmazeutisch geeigneten und physiologisch verträglichen Trägerstoff, Zusatzstoff und/oder anderen Wirk- und Hilfsstoffen.

8. Verwendung der Verbindung der Formel I 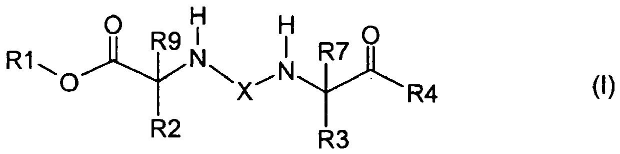 und/oder einer stereoisomeren Form der Verbindung der Formel I und/oder Gemische dieser Formen in jedem Verhältnis, und/oder ein physiologisch verträgliches Salz der Verbindung der Formel I gemäß der Ansprüche 1 bis 5, zur Herstellung eines Arzneimittels zur Prophylaxe, Sekundärprevention und Therapie einer oder mehrerer Erkrankungen, die mit Thrombosen, Embolien, Hyperkoagulabilität oder fibrotischen Veränderungen einhergehen ausgewählt aus der Reihe Myokardinfarkt, Angina pectoris und andere Formen des akuten Koronarsyndroms, den Schlaganfall, die peripher vaskulären Erkrankungen, die tiefe Venenthrombose, die Lungenembolie, embolische oder thrombotische Ereignisse bedingt durch kardiale Arrhythmien, kardiovaskuläre Ereignisse wie Restenose nach Revaskularisierung und Angioplastie und ähnlichen Eingriffen wie Stentimplantationen und Bypass-Operationen handelt, oder die Reduktion der Thrombosegefahr nach chirurgischen Eingriffen wie bei Knie- und Hüftgelenksoperationen, oder die disseminierte intravaskuläre Koagulation, Sepsis und anderen intravaskulären Ereignissen, die mit einer Entzündung einhergehen, Atherosklerose, Diabetes und dem metabolischen Syndrom und deren Folgen, Tumorwachstum und Tumormetastasierung, entzündliche und degenerative Gelenkserkrankungen wie der rheumatoiden Arthritis und der Arthrose, Störungen des hämostatischen Systems wie Fibrinablagerungen, fibrotische Veränderungen der Lunge wie die chronische obstruktive Lungenerkrankung, das adult respiratory distress syndrom oder Fibrinablagerungen des Auges nach Augenoperationen oder Verhinderung oder Behandlung von Narbenbildung.

## Claims

1. A compound of the formula I 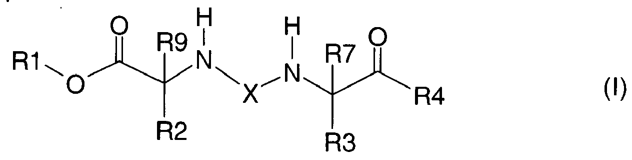 and/or of a stereoisomeric form of the compound of the formula I and/or mixtures of these forms in any ratio, and/or a physiologically tolerated salt of the compound of the formula I, where
X is -S(O)₂-,
R1 is
1) hydrogen atom,
2) -(C₁-C₆)-alkyl,
3) -(C₀-C₄)-alkylene-(C₃-C₁₂)-cycloalkyl or
4) -(C₁-C₆)-alkylene-(C₆-C₁₄)-aryl,
R2 is a radical of the formula II
-(A1)ₘ-A2 (II)
in which
m is the integer zero or 1,
A1 is
1) -(CH₂)ₙ- in which n is the integer zero, 1, 2 or 3,
2) -NH-(CH₂)ₙ- in which n is the integer zero, 1, 2 or 3,
3) -NH(C₁-C₆)-alkyl)-(CH₂)ₙ- in which n is the integer zero, 1, 2 or 3,
4) -NH((C₃-C₆)-cycloalkyl)-(CH₂)ₙ- in which n is the integer zero, 1, 2 or 3,
5) -O-(CH₂)ₙ- in which n is the integer zero, 1, 2 or 3, or
6) -(CH₂)ₙ-SOₓ- in which n is the integer zero, 1,2 or 3 and x the integer zero, 1 or 2,
A2 is
1) Het, where Het means a 4- to 15-membered heterocyclic ring system having 4 to 15 ring atoms which are present in one, two or three ring systems connected together, and which comprise one, two, three or four identical or different heteroatoms from the series oxygen, nitrogen or sulfur, and are unsubstituted or substituted independently of one another once, twice or three times by -(C₁-C₃)-alkyl, halogen, -NH2, -CF₃ or -O-CF₃,
2) -(C₀-C₆)-alkylene-NH₂ ,
3) -(C₁-C₆)-alkylene-NH-C(=NH)-NH₂,
4) -(C₁-C₆)-alkylene-NH-C(=NH)-(C₁-C₄)-alkyl,
5) -(C₀-C₄)-alkylene-O-NH-C(=NH)-NH₂,
6) -(C₀-C₄)-alkylene-NH-C(O)-(C₁-C₆)-alkyl,
7) -(C₁-C₆)-alkylene-NH-C(O)-O-(C₁-C₄)-alkylene-aryl, where aryl is unsubstituted or substituted by -NH₂ or is substituted by -NH₂ and once, twice or three times by R15,
8) -(C₃-C₈)-cycloalkyl-NH₂, or
9) -(C₀-C₄)-alkylene-(C₆-C₁₄)-aryl, where aryl is unsubstituted or substituted by -NH₂ or is substituted by -NH₂ and once, twice or three times by R15,
R3 is
1) -(C₁-C₆)-alkyl,
2) -(C₀-C₄)-alkylene-(C₃-C₁₂)-cycloalkyl,
3) -(C₁-C₆)-alkylene-(C₆-C₁₄)-aryl, where aryl is substituted independently of one another once, twice or three times by R15,
4) -(C₀-C₈)-alkylene-N(R5)-PG,
5) -(C₁-C₆)-alkylene-NH-C(O)-O-(C₁-C₄)-alkylene-aryl, where aryl is substituted independently of one another once, twice or three times by R15,
6) -(C₀-C₄)-alkylene-(C₆-C₁₄)-aryl-(C₀-C₄)-alkylene-N(R5)-PG,
7) -(C₀-C₈)-alkylene-O-PG,
8) -(C₀-C₄)-alkylene-(C₆-C₁₄)-aryl-(C₀-C₄)-alkylene-O-PG,
9) -(C₀-C₈)-alkylene-C(O)-O-PG,
10) -(C₀-C₄)-alkylene-(C₆-C₁₄)-aryl-(C₀-C₄)-alkylene-C(O)-O-PG or
11) hydrogen atom,
R4 is -N(R6)₂,
where R6 are identical or different and are independently of one another
1) hydrogen atom,
2) -(C₁-C₆)-alkyl,
3) -(C₀-C₄)-alkylene-(C₃-C₁₂)-cycloalkyl, where cycloalkyl is unsubstituted or substituted independently of one another once, twice, three or four times by R11, halogen, -C(O)-O-R11, -(C₁-C₄)-alkyl-O-R11 or -O-(C₁-C₄)-alkyl,
4) -(C₀-C₆)-alkylene-(C₆-C₁₄)-aryl, where aryl and alkylene are unsubstituted or substituted independently of one another once, twice, three or four times by R11, halogen, -C(O)-O-R11, -(C₁-C₄)-alkyl-O-R11, -C(O)-N(R8)₂ or -O-(C₁-C₄)-alkyl,
5) -(C₀-C₈)-alkylene-N(R5)-PG,
6) -(C₀-C₄)-alkylene-(C₆-C₁₄)-aryl-(C₀-C₄)-alkyl-N(R5)-PG,
7) -(C₀-C₈)-alkylene-O-PG,
8) -(C₀-C₄)-alkylene-(C₆-C₁₄)-aryl-(C₀-C₄)-alkyl-O-PG,
9) -(C₀-C₈)-alkylene-C(O)-O-R11,
10) -(C₀-C₄)-alkylene-(C₆-C₁₄)-aryl-(C₀-C₄)-alkyl-C(O)-O-PG,
11) -(C₀-C₄)-alkylene-Het, where Het means a 4- to 15-membered heterocyclic ring system having 4 to 15 ring atoms which are present in one, two or three ring systems connected together, and which comprise one, two, three or four identical or different heteroatoms from the series oxygen, nitrogen or sulfur, where Het or alkylene are unsubstituted or substituted independently of one another once, twice or three times by R11, halogen, -C(O)-O-R11,-(C₁-C₄)-alkyl-O-R11 or -O-(C₁-C₄)-alkyl,
12) -(C₁-C₃)-fluoroalkyl,
13) -(C₀-C₄)-alkylene-CH(R11)-C(O)-NH₂,
14) -(C₀-C₄)-alkylene-CH(R11)-C(O)-NH-(C₁-C₄)-alkyl, or
15) -(C₀-C₄)-alkylene-CH(R11)-C(O)-NH-CH(R12)-R13,
or the two R6 radicals form together with the N atom to which they are bonded a mono- or bicyclic ring having 4 to 9 ring atoms which is saturated, partly saturated or aromatic, where the ring is unsubstituted or substituted once or twice by -(C₁-C₄)-alkyl, -C(O)-O-R11, halogen, -(C₁-C₄)-alkyl-O-R11 or phenyl,
R5 is hydrogen atom or-(C₁-C₆)-alkyl,
PG is a protective group for the amino, carboxyl or for the hydroxy function,
R7 is hydrogen atom or -(C₁-C₆)-alkyl,
R8 is hydrogen atom or -(C₁-C₆)-alkyl,
R9 is hydrogen atom or -(C₁-C₆)-alkyl,
R11 and R12 are identical or different and are independently of one another
1) hydrogen atom,
2) -(C₁-C₆)-alkyl,
3) -(C₀-C₄)-alkylene-phenyl, where phenyl is unsubstituted or substituted independently of one another once, twice or three times by halogen, -OH or -O-(C₁-C₄)-alkyl,
4) -(C₀-C₄)-alkylene-(C₃-C₁₂)-cycloalkyl, where cycloalkyl is unsubstituted or substituted independently of one another once, twice, three or four times by R13, halogen, -C(O)-O-R13, -(C₁-C₄)-alkyl-O-R13, -O-(C₁-C₄)-alkyl or -(C₀-C₄)-alkylene-phenyl,
5) -(C₀-C₄)-alkylene-C(O)-N(R13)₂ or
6) -(C₀-C₄)-alkylene-indolyl,
R13 is
1) hydrogen atom,
2) -(C₁-C₄)-alkyl,
3) -(C₀-C₄)-alkylene-C(O)-O-R14,
4) -(C₀-C₄)-alkylene-C(O)-R14 or
5) -(C₀-C₄)-alkylene-O-R14,
R14 is hydrogen atom, -(C₁-C₄)-alkyl, -NH₂ or -OH, and
R15 is hydrogen atom, -(C₁-C₄)-alkyl, -O-CF₃, -NH₂, -OH,-CF₃ or halogen.

2. A compound of the formula I as claimed in claim 1, where
R1 is
1) hydrogen atom or
2) -(C₁-C₄)-alkyl,
R2 is
1) -(C₁-C₆)-alkylene-NH₂,
2) -(C₀-C₄)-alkylene-pyridyl-NH₂,
3) -(C₀-C₄)-alkylene-piperidinyl-NH₂,
4) -(C₀-C₄)-alkylene-thiazolyl-NH₂,
5) -(C₁-C₆)-alkylene-NH-C(=NH)-NH₂,
6) -(C₀-C₄)-alkylene-(C₃-C₈)-cycloalkyl-NH₂,
7) -(C₁-C₆)-alkylene-NH-C(=NH)-(C₁-C₄)-alkyl,
8) -(C₀-C₄)-alkylene-O-NH-C(=NH)-NH₂,
9) -(C₁-C₆)-alkylene-NH-C(O)-O-(C₁-C₄)-alkylene-aryl, where aryl is unsubstituted or substituted by -NH₂ or is substituted by -NH₂ and once, twice or three times by R15,
10) -(C₀-C₄)-alkylene-NH-C(O)-(C₁-C₄)-alkyl,
11) -(C₀-C₄)-alkylene-(C₆-C₁₄)-aryl, where aryl is unsubstituted or substituted by -NH₂ or is substituted by -NH₂ and once, twice or three times by R15, or
12) -(C₁-C₄)-alkylene-SOₓ-(C₁-C₄)-alkylene-NH₂ in which x is the integer zero, 1 or 2,
R3 is
1) -(C₁-C₄)-alkyl,
2) -(C₀-C₄)-alkylene-(C₃-C₈)-cycloalkyl,
3) -(C₁-C₆)-alkylene-aryl, where aryl is substituted independently of one another once, twice or three times by R15,
4) -(C₁-C₆)-alkylene-NH-C(O)-O-(C₁-C₄)-alkylene-aryl, where aryl is substituted independently of one another once, twice or three times by R15,
5) -(C₁-C₆)-alkylene-NH-PG,
6) -(C₁-C₆)-alkylene-O-PG,
7) -(C₁-C₆)-alkyl, or
8) hydrogen atom,
where PG is t-butyl-, t-butyloxycarbonyl or benzyloxycarbonyl,
R4 is -N(R6)₂,
where R6 are identical or different and are independently of one another
1) hydrogen atom,
2) -(C₁-C₆)-alkyl,
3) -(C₀-C₄)-alkylene-(C₀-C₁₂)-cycloalkyl, where cycloalkyl is unsubstituted or substituted independently of one another once, twice, three or four times by R11, halogen, -C(O)-O-R11, -(C₁-C₄)-alkyl-O-R11 or -O-(C₁-C₄)-alkyl,
4) -(C₀-C₄)-alkylene-C(R11)(R12)-(C₃-C₁₂)-cycloalkyl, where cycloalkyl is unsubstituted or substituted independently of one another once, twice or three times by R11, halogen, -C(O)-O-R11, -(C₁-C₄)-alkyl-O-R11 or -O-(C₁-C₄)-alkyl,
5) -(C₀-C₄)-alkylene-Het, where Het means a 4- to 15-membered heterocyclic ring system having 4 to 15 ring atoms which are present in one, two or three ring systems connected together, and which comprise one, two, three or four identical or different heteratoms from the series oxygen, nitrogen or sulfur, where Het or alkylene are unsubstituted or substituted independently of one another once, twice or three times by R11, halogen, -C(O)-O-R11, -(C₁-C₄)-alkyl-O-R11 or -O-(C₁-C₄)-alkyl,
6) -(C₀-C₆)-alkylene-aryl, where aryl or alkylene is unsubstituted or substituted independently of one another once, twice or three times by R11, halogen, -C(O)-O-R11, -(C₀-C₄)-alkyl-OR11 or -O-(C₁-C₄)-alkyl,
7) -(C₀-C₄)-alkylene-C(R11)(R12)-aryl, where aryl or alkylene is unsubstituted or substituted independently of one another once, twice or three times by R11, halogen, -C(O)-O-R11, - (C₀-C₄)-alkyl-O-R11 or -O-(C₁-C₄)-alkyl,
8) 1,2,3,4-tetrahydronaphthalenyl,
9) -(C₀-C₄)-alkylene-CH(R11)-C(O)-NH₂,
10) -(C₀-C₄)-alkylene-CH(R11)-C(O)-NH-(C₁-C₄)-alkyl,
11) -(C₀-C₄)-alkylene-CH(R11)-C(O)-NH-CH(R12)-R13,
12) -(C₀-C₆)-alkylene-C(O)-O-R11, where alkylene is unsubstituted or substituted independently of one another once or twice by R11, halogen, -C(O)-O-R11, -(C₁-C₄)-alkyl-O-R11 or -O-(C₁-C₄)-alkyl,
13) -(C₀-C₄)-alkylene-C(R11)(R12)-C(O)-O-R11, or
14) -(C₁-C₃)-fluoroalkyl,
or the two R6 radicals form together with the N atom to which they are bonded a mono- or bicyclic ring having 4 to 9 ring atoms which is saturated, partly saturated or aromatic, where the ring is unsubstituted or substituted once or twice by -(C₁-C₄)-alkyl, -C(O)-O-R11, halogen, -(C₁-C₄)-alkyl-O-R11 or phenyl,
R7 is hydrogen atom or -(C₁-C₄)-alkyl,
R9 is hydrogen atom or -(C₁-C₄)-alkyl,
R11 and R12 are identical or different and are independently of one another
1) hydrogen atom,
2) -(C₁-C₄)-alkyl,
3) -(C₀-C₄)-alkylene-phenyl, where phenyl is unsubstituted or substituted independently of one another once, twice or three times by halogen, -OH or -O-(C₁-C₄)-alkyl,
4) -(C₀-C₄)-alkylene-(C₃-C₁₂)-cycloalkyl, where cycloalkyl is unsubstituted or substituted independently of one another once, twice, three or four times by R13, halogen, -C(O)-O-R13, -(C₁-C₄)-alkyl-O-R13, -O-(C₁-C₄)-alkyl or -(C₀-C₄)-alkylene-phenyl,
5) -(C₀-C₄)-alkylene-C(O)-N(R13)₂ or
6) -(C₀-C₄)-alkylene-indolyl,
R13 is
1) hydrogen atom,
2) -(C₁-C₄)-alkyl,
3) -(C₀-C₄)-alkylene-C(O)-O-R14,
4) -(C₀-C₄)-alkylene-C(O)-R14 or
5) -(C₀-C₄)-alkylene-O-R14,
R14 is hydrogen atom, -(C₁-C₄)-alkyl, -NH₂ or -OH, and
R15 is hydrogen atom, -(C₁-C₄)-alkyl, -O-CF₃, -NH₂, -OH,-CF₃ or halogen.

3. A compound of the formula I as claimed in claim 1 or 2, where
R1 is
1) hydrogen atom or
2) -(C₁-C₄)-alkyl,
R2 is
1) -(C₁-C₆)-alkylene-NH₂,
2) -(C₁-C₄)-alkylene-pyridyl-NH₂,
3) -(C₁-C₄)-alkylene-piperidinyl-NH₂,
4) -(C₁-C₆)-alkylene-NH-C(=NH)-NH₂,
5) -(C₀-C₄)-alkylene-(C₃-C₆)-cycloalkyl-NH₂,
6) -(C₁-C₆)-alkylene-NH-C(=NH)-(C₁-C₄)-alkyl,
7) -(C₁-C₄)-alkylene-O-NH-C(=NH)-NH₂,
8) -(C₁-C₆)-alkylene-NH-C(O)-O-(C₁-C₄)-alkylene-phenyl, where phenyl is unsubstituted or substituted by -NH₂ or is substituted by -NH₂ and once, twice or three times by R15,
9) -(C₁-C₄)-alkylene-NH-C(O)-(C₁-C₆)-alkyl,
10) -(C₁-C₄)-alkylene-phenyl, where phenyl is unsubstituted or substituted by -NH₂ or is substituted by -NH₂ and once, twice or three times by R15,
11) -(C₁-C₄)-alkylene-SO₂-(C₁-C₄)-alkylene-NH₂ or
12) -(C₁-C₄)-alkylene-S-(C₁-C₄)-alkylene-NH₂,
R3 is
1) -(C₁-C₄)-alkyl,
2) -(C₁-C₄)-alkylene-(C₃-C₆)-cycloalkyl,
3) -(C₁-C₄)-alkylene-phenyl, where phenyl is substituted independently of one another once, twice or three times by R15,
4) -(C₁-C₆)-alkylene-NH-C(O)-O-(C₁-C₄)-alkylene-phenyl, where phenyl is substituted independently of one another once, twice or three times by R15,
5) hydrogen atom,
R4 is -N(R6)₂,
where R6 are identical or different and are independently of one another
1) hydrogen atom,
2) -(C₁-C₄)-alkyl,
3) -(C₀-C₄)-alkylene-(C₃-C₁₂)-cycloalkyl, where cycloalkyl is selected from the group of cyclohexyl, cyclopentyl, cyclobutyl, cyclopropyl, adamantanyl, 1,7,7-trimethylbicyclo[3.1.1]heptanyl, decahydronaphthalenyl, tetrahydronaphthalenyl, octahydro-4,7-methanoindenyl or bicyclo[2.2.1]heptanyl and in which cycloalkyl is unsubstituted or substituted independently of one another once, twice, three or four times by -(C₁-C₄)-alkyl, -C(O)-O-R11 or -(C₁-C₄)-alkylene-phenyl, where phenyl is unsubstituted or substituted by halogen,
4) -(C₀-C₄)-alkylene-C(R11)(R12)-(C₃-C₁₂)-cycloalkyl, where cycloalkyl is selected from the group of cyclohexyl, cyclopentyl, cyclobutyl, cyclopropyl, adamantanyl, 1,7,7-trimethylbicyclo[3.1.1]heptanyl, decahydronaphthalenyl, tetrahydronaphthalenyl, octahydro-4,7-methanoindenyl or bicyclo[2.2.1]heptanyl and in which cycloalkyl is unsubstituted or substituted independently of one another once, twice, three or four times by -(C₁-C₄)-alkyl, -C(O)-O-R11 or -(C₁-C₄)-alkylene-phenyl, where phenyl is unsubstituted or substituted by halogen,
5) -(C₀-C₄)-alkylene-Het, where Het is selected from the group of acridinyl, azepinyl, azetidinyl, aziridinyl, benzimidazalinyl, benzimidazolyl, benzo[1,3]dioxolyl, benzofuranyl, benzothiofuranyl, benzothiophenyl, benzoxazolyl, benzthiazolyl, benztriazolyl, benztetrazolyl, benzisoxazolyl, benzisothiazolyl, carbazolyl, 4aH-carbazolyl, carbolinyl, quinazolinyl, quinolinyl, 4H-quinolizinyl, quinoxalinyl, quinuclidinyl, chromanyl, chromenyl, cinnolinyl, decahydroquinolinyl, dibenzofuranyl, dibenzothiophenyl, dihydrofuran[2,3-b]-tetrahydrofuranyl, dihydrofuranyl, dioxolyl, dioxanyl, 2H, 6H-1,5,2-dithiazinyl, furanyl, furazanyl, imidazolidinyl, imidazolinyl, imidazolyl, 1H-indazolyl, indolinyl, indolizinyl, indolyl, 3H-indolyl, isobenzofuranyl, isochromanyl, isoindazolyl, isoindolinyl, isoindolyl, isoquinolinyl (benzimidazolyl), isothiazolidinyl, 2-isothiazolinyl, isothiazolyl, isoxazolyl, isoxazolidinyl, 2-isoxazolinyl, morpholinyl, naphthyridinyl, octahydroisoquinolinyl, oxadiazolyl, 1,2,3-oxadiazolyl, 1,2,4-oxadiazolyl, 1,2,5-oxadiazolyl, 1,3,4-oxadiazolyl, oxazolidinyl, oxazolyl, oxazolidinyl, oxothiolanyl, pyrimidinyl, phenanthridinyl, phenanthrolinyl, phenazinyl, phenothiazinyl, phenoxathiinyl, phenoxazinyl, phthalazinyl, piperazinyl, piperidinyl, pteridinyl, purynyl, pyranyl, pyrazinyl, pyroazolidinyl, pyrazolinyl, pyrazolyl, pyridazinyl, pryidooxazolyl, pyridoimidazolyl, pyridothiazolyl, pyridothiophenyl, pyridinyl, pyridyl, pyrimidinyl, pyrrolidinyl, pyrrolinyl, 2H-pyrrolyl, pyrrolyl, tetrahydrofuranyl, tetrahydroisoquinolinyl, tetrahydroquinolinyl, tetrahydropyridinyl, 6H-1,2,5-thiadazinyl, 1,2,3-thiadiazolyl, 1,2,4-thiadiazolyl, 1,2,5-thiadiazolyl, 1,3,4-thiadiazolyl, thianthrenyl, thiazolyl, thienyl, thienoimidazolyl, thienooxazolyl, thienopyridine, thienothiazolyl, thiomorpholinyl, thiophenyl, triazinyl, 1,2,3-triazolyl, 1,2,3-triazolyl, 1,2,4-triazolyl, 1,2,5-triazolyl, 1,3,4-triazolyl and xanthenyl, where Het or alkylene is unsubstituted or substituted independently of one another once or twice by -(C₁-C₄)-alkyl,
6) -(C₁-C₆)-alkylene-phenyl, where phenyl or alkylene are unsubstituted or substituted independently of one another once or twice by halogen, phenyl, -C(O)-O-R11, -(C₁-C₄)-alkyl-O-R11, -O-(C₁-C₄)-alkyl or -(C₁-C₄)-alkyl,
7) -(C₀-C₄)-alkylene-C(R11)(R12)-phenyl, where phenyl is unsubstituted or substituted independently of one another once, twice or three times by phenyl or fluorine,
8) 1,2,3,4-tetrahydronaphthalenyl,
9) -(C₀-C₄)-alkylene-CH(R11)-C(O)-NH₂,
10) -(C₀-C₄)-alkylene-CH(R11)-C(O)-NH-(C1-C4)-alkyl,
11) -(C₀-C₄)-alkylene-CH(R11)-C(O)-NH-CH(R12)-R13,
12) -(C₁-C₆)-alkylene-C(O)-O-R11, where alkylene is unsubstituted or substituted independently of one another once or twice by halogen, phenyl, -C(O)-O-R11, -(C₁-C₄)-alkyl-O-R11, -O-(C₁-C₄)-alkyl or -(C₁-C₄)-alkyl,
13) -(C₀-C₄)-alkylene-C(R11)(R12)-C(O)-O-R11, or
14) -(C₁-C₃)-fluoroalkyl,
or the two R6 radicals form together with the N atom to which they are bonded a mono- or bicyclic ring selected from the group of pyrrolidine, piperidine, 2-aza-bicyclo[3.2.2]nonane and 7-azabicyclo[2.2.1]heptane, where the ring is unsubstituted or substituted once or twice by -(C₁-C₄)-alkyl, -C(O)-O-R11, -(C₁-C₄)-alkyl-O-R11 or phenyl,
R7 is hydrogen atom or -(C₁-C₄)-alkyl,
R9 is hydrogen atom or -(C₁-C₄)-alkyl,
R11 and R12 are identical or different and are independently of one another
1) hydrogen atom,
2) -(C₁-C₄)-alkyl,
3) -(C₀-C₄)-alkylene-phenyl, where phenyl is unsubstituted or substituted independently of one another once, twice or three times by -OH, halogen or -O-(C₁-C₄)-alkyl,
4) -(C₀-C₄)-alkylene-(C₃-C₁₂)-cycloalkyl, where cycloalkyl is selected from the group of cyclohexyl, cyclopentyl, cyclobutyl, cyclopropyl, adamantanyl, 1,7,7-trimethylbicyclo[3.1.1]heptanyl, decahydronaphthalenyl, tetrahydronaphthalenyl, octahydro-4,7-methanoindenyl or bicyclo[2.2.1]heptanyl and in which cycloalkyl is unsubstituted or substituted independently of one another once, twice, three or four times by -(C₁-C₄)-alkyl, -C(O)-O-R13 or phenyl, or
5) -(C₀-C₄)-alkylene-indolyl,
R13 is
1) hydrogen atom,
2) -(C₁-C₄)-alkyl,
3) -(C₀-C₄)-alkylene-C(O)-O-R14,
4) -(C₀-C₄)-alkylene-C(O)-R14 or
5) -(C₀-C₄)-alkylene-O-R14, and
R14 is hydrogen atom, -(C₁-C₄)-alkyl, -NH₂ or -OH and
R15 is hydrogen atom, -(C₁-C₄)-alkyl, -O-CF₃, -NH₂, -OH, - CF₃ or halogen.

4. A compound of the formula I as claimed in claims 1 to 3, where
R1 is
1) hydrogen atom or
2) -(C₁-C₄)-alkyl,
R2 is
1) -(C₁-C₆)-alkylene-NH₂,
2) -(C₁-C₄)-alkylene-pyridyl-NH₂,
3) -(C₁-C₄)-alkylene-piperidinyl-NH₂,
4) -(C₁-C₄)-alkylene-NH-C(=NH)-NH₂,
5) -(C₁-C₆)-alkylene-NH-C(=NH)-(C₁-C₄)-alkyl,
6) -(C₁-C₄)-alkylene-(C₃-C₆)-cycloalkyl-NH₂,
7) -(C₁-C₄)-alkylene-O-NH-C(=NH)-NH₂,
8) -(C₁-C₆)-alkylene-NH-C(O)-O-(C₁-C₄)-alkylene-phenyl,
9) -(C₁-C₄)-alkylene-NH-C(O)-(C₁-C₆)-alkyl,
10) -(C₁-C₄)-alkylene-phenyl-NH₂,
11) -(C₁-C₄)-alkylene-SO₂-(C₁-C₄)-alkylene-NH₂ or,
12) -(C₁-C₄)-alkylene-S-(C₁-C₄)-alkylene-NH₂,
R3 is
1) -(C₁-C₄)-alkyl,
2) -(C₁-C₄)-alkylene-(C₃-C₆)-cycloalkyl,
3) -(C₁-C₄)-alkylene-phenyl, where phenyl is unsubstituted or substituted by -OH,
4) -(C₁-C₆)-alkylene-NH-C(O)-O-(C₁-C₄)-alkylene-phenyl,
5) hydrogen atom,
R4 is -N(R6)₂,
where R6 are identical or different and are independently of one another
1) hydrogen atom,
2) -(C₁-C₆)-alkyl,
3) -(C₀-C₄)-alkylene-(C₃-C₈)-cycloalkyl, where cycloalkyl is selected from the group of cyclohexyl, cyclopentyl, cyclopropyl, adamantanyl, 1,7,7-trimethylbicyclo[3.1.1]heptanyl, decahydronaphthalene, octahydro-4,7-methanoindenyl or bicyclo[2.2.1]heptanyl and in which cycloalkyl is unsubstituted or substituted independently of one another once, twice or three times by -(C₁-C₄)-alkyl or phenyl,
4) -C(R11)(R12)-adamantanyl,
5) -CH(R11)-C(O)-NH-CH(R12)-R13,
6) -(C₀-C₄)-alkylene-Het, where Het is selected from the group of benzimidazolyl, isoxazolyl, piperidine, pyridine, pyrrolidinyl, thiophenyl and benzo[1,3]dioxol,
7) 1,2,3,4-tetrahydronaphthalenyl,
8) -(C₀-C₄)-alkylene-C(R11)(R12)-phenyl, where phenyl is unsubstituted or substituted independently of one another once, twice or three times by phenyl or fluorine,
9) -CH(R11)-C(O)-NH₂,
10) -CH(R11)-C(O)-NH-CH(R12)-CH₂-OH,
11) -(C₁-C₆)-alkylene-phenyl, where phenyl or alkylene are unsubstituted or substituted independently of one another once or twice by chlorine, fluorine, -C(O)-O-R11, -(C₁-C₄)-alkyl-O-R11, -O-(C₁-C₄)-alkyl, phenyl or -(C₁-C₄)-alkyl,
12) -CH(R11)-C(O)-NH-(C₁-C₄)-alkyl,
13) -(C₀-C₄)-alkylene-C(R11)(R12)-bicyclo[3.1.1]heptanyl, where bicyclo[3.1.1]heptanyl is unsubstituted or substituted once to four times by -(C₁-C₄)-alkyl,
14) -(C₁-C₆)-alkylene-C(O)-O-R11, where alkylene is unsubstituted or substituted independently of one another once or twice by chlorine, fluorine, -C(O)-O-R11, -(C₁-C₄)-alkyl-O-R11, -O-(C₁-C₄)-alkyl, phenyl or -(C₁-C₄)-alkyl,
15) -(C₀-C₄)-alkylene-C(R11)(R12)-C(O)-O-R11, or
16) -CH₂-CF₂-CF₃,
or the two R6 radicals form together with the N atom to which they are bonded a mono- or bicyclic ring selected from the group of pyrrolidines, 2-azabicyclo[3.2.2]nonane and 7-azabicyclo[2.2.1]heptane, where the ring is unsubstituted or substituted once or twice by -(C₁-C₄)-alkyl, -C(O)-O-R11, -(C₁-C₄)-alkyl-O-R11 or phenyl,
R7 is hydrogen atom or -(C₁-C₄)-alkyl,
R9 is hydrogen atom or -(C₁-C₄)-alkyl,
R11 and R12 are identical or different and are independently of one another
1) hydrogen atom,
2) -(C₁-C₄)-alkyl,
3) -(C₀-C₄)-alkylene-phenyl, where phenyl is unsubstituted or substituted independently of one another once, twice or three times by -OH, halogen or -O-(C₁-C₄)-alkyl,
4) -(C₀-C₄)-alkylene-(C₃-C₁₂)-cycloalkyl, where cycloalkyl is selected from the group of cyclohexyl, cyclopentyl, cyclobutyl, cyclopropyl, adamantanyl, 1,7,7-trimethylbicyclo[3.1.1]heptanyl, decahydronaphthalenyl, octahydro-4,7-methanoindenyl or bicyclo[2.2.1]heptanyl and in which cycloalkyl is unsubstituted or substituted independently of one another once, twice, three or four times by -(C₁-C₄)-alkyl, -C(O)-O-R13 or phenyl or
5) -(C₀-C₄)-alkylene-indolyl,
R13 is
1) hydrogen atom,
2) -(C₁-C₄)-alkyl,
3) -(C₀-C₄)-alkylene-C(O)-O-R14,
4) -(C₀-C₄)-alkylene-C(O)-R14 or
5) -(C₀-C₄)-alkylene-O-R14,
R14 is hydrogen atom, -(C₁-C₄)-alkyl, -NH₂ or -OH and
R15 is hydrogen atom, -(C₁-C₄)-alkyl, -O-CF₃, -NH₂, -OH, - CF₃ or halogen.

5. A compound of the formula I as claimed in one or more of claims 1 to 4, which is the compound of the formula I (S)-6-amino-2-(3-{(S)-1-[(S)-1-((S)-1-methoxycarbonyl-2-methyl-propylcarbamoyl)-2-methyl-propylcarbamoyl]-2-phenyl-ethyl}-sulfamidyl)-hexanoic acid, (S)-6-amino-2-{3-[(R)-1-(bicyclo[2.2.1]hept-2-ylcarbamoyl)-2-cyclohexylethyl]-sulamidyl}- hexanoic acid, (S)-6-amino-2-[3-((S)-1-cyclohexylcarbamoyl-2-phenyl-ethyl)-sulfamidyl]- hexanoic acid, (S)-6-acetimidoylamino-2-{[(S)-2-cyclohexyl-1-((1R,2S,4R)-1,7,7-trimethyl-bicyclo[2.2.1]hept-2-ylcarbamoyl)-ethylsulfamidyl]}-hexanoic acid, ethyl 3-(6-amino-pyridin-3-yl)-2-[(S)-2-cyclohexyl-1-((1R,2S,4R)-1,7,7-trimethyl-bicyclo[2.2.1]hept-2-ylcarbamoyl)-ethylsulfamidyl]-propionate, (S)-6-amino-2-{[(S)-1-((1R,2S,4R)-1,7,7-trimethyl-bicyclo[2.2.1]hept-2-ylcarbamoyl)-propylsulfamidyl]-}-hexanoic acid, (S)-2-{[(S)-2-cyclohexyl-1-((1R,2S,4R)-1,7,7-trimethyl-bicyclo[2.2.1]hept-2-ylcarbamoyl)-ethylsulfamidyl]-}-5-guanidino-pentanoic acid, (S)-6-amino-2-{[(S)-2-cyclobutyl-1-((1R,2S,4R)-1,7,7-trimethyl-bicyclo[2.2.1]hept-2-ylcarbamoyl)-ethylsulfamidyl]}-hexanoic acid, (S)-5-amino-2-({2-cyclohexyl-1-[(R)-(1,2,3,4-tetrahydro-naphthalen-2-yl)carbamoyl]-ethylsulfamidyl})-pentanoic acid, ethyl (S)-2-{[(S)-2-cyclohexyl-1-((1R,2S,4R)-1,7,7-trimethyl-bicyclo[2.2.1]hept-2-ylcarbamoyl)-ethylsulfamidyl]}-6-hexanoylaminohexanoate, (S)-6-amino-2-{[(S)-1-((1R,2S,4R)-1,7,7-trimethyl-bicyclo[2.2.1]hept-2-ylcarbamoyl)-butylsulfamidyl]}-hexanoic acid, (S)-6-amino-2-{[(S)-3-cyclohexyl-1-((1R,2S,4R)-1,7,7-trimethyl-bicyclo[2.2.1]hept-2-ylcarbamoyl)-propylsulfamidyl]-methyl}-hexanoic acid, (S)-6-amino-2-{[(S)-2-cyclohexyl-1-(decahydro-naphthalen-2-ylcarbamoyl)-ethylsulfamidyl]}-hexanoic acid, (S)-2-{[(S)-1-(adamantan-1-ylcarbamoyl)-2-cyclohexyl-ethylsulfamidyl]}-6-amino- hexanoic acid, (S)-2-[(S)-2-cyclohexyl-1-((1R,2S,4R)-1,7,7-trimethyl-bicyclo[2.2.1]-hept-2-ylcarbamoyl)-ethylsulfamidyl]-3-pyridin-3-yl-propionic acid, (S)-2-[(S)-2-cyclohexyl-1-((1R,2S,4R)-1,7,7-trimethyl-bicyclo[2.2.1]hept-2-ylcarbamoyl)-ethylsulfamidyl]-3-pyridin-4-yl-propionic acid, (S)-6-amino-2-{[(S)-2-cyclohexyl-1-((1S,2S,3S,5R)-2,6,6-trimethyl-bicyclo[3.1.1]hept-3-ylcarbamoyl)-ethylsulfamidyl]}-hexanoic acid, (S)-6-amino-2-{[(S)-2-cyclohexyl-1-(3,3,5-trimethyl-cyclohexylcarbamoyl)-ethylsulfamidyl]}-hexanoic acid, (S)-6-amino-2-{[(S)-1-(4-tert-butyl-cyclohexylcarbamoyl)-2-cyclohexyl-ethylsulfamidyl]}-hexanoic acid, (S)-6-amino-2-{[(S)-2-cyclohexyl-1-(3-methyl-cyclohexylcarbamoyl)-ethylsulfamidyl]}-hexanoic acid, (S)-6-amino-2-{[(S)-2-cyclohexyl-1-((1R,2S,4R)-1,7,7-trimethyl-bicyclo[2.2.1]hept-2-ylcarbamoyl)-ethylsulfamidyl]}-hexanoic acid, 3-(6-amino-pyridin-3-ylmethyl)-2-[(S)-2-cyclohexyl-1-((1R,2S,4R)-1,7,7-trimethyl-bicyclo[2.2.1]hept-2-yl-carbamoyl)-ethylsulfamidyl]-propionic acid, 2-[(S)-2-cyclohexyl-1-((1R,2S,4R)-1,7,7-trimethyl-bicyclo[2.2.1]hept-2-ylcarbamoyl)-ethylsulfamidyl]-3-piperidin-4-yl-propionic acid, (S)-3-(4-amino-phenyl)-2-[(S)-2-cyclohexyl-1-((1R,2S,4R)-1,7,7-trimethyl-bicyclo[2.2.1]hept-2-ylcarbamoyl)-ethylsulfamidyl]-propionic acid, (S)-6-amino-2-[((S)-1-cyclohexylmethyl-2-oxo-2-piperidin-1-yl-ethylsulfamidyl)]-hexanoic acid, (S)-5-amino-2-{[(S)-2-cyclohexyl-1-((1R,2S,4R)-1,7,7-trimethyl-bicyclo[2.2.1]hept-2-ylcarbamoyl)-ethylsulfamidyl]}-pentanoic acid, (S)-6-amino-2-{[(S)-2-cyclohexyl-1-((S)-1-isobutylcarbamoyl-2-methyl-propylcarbamoyl)-ethylsulfamidyl]}-hexanoic acid, (S)-6-amino-2-{[(S)-1-((S)-1-isobutylcarbamoyl-2-methyl-propylcarbamoyl)-2-phenyl-ethylsulfamidyl]}-hexanoic acid, (S)-6-amino-2-[((S)-2-cyclohexyl-1-isobutylcarbamoyl-ethylsulfamidyl)]-hexanoic acid, (S)-6-amino-2-{[(S)-1-((1R,2R,4S)-bicyclo[2.2.1]hept-2-ylcarbamoyl)-2-cyclohexyl-ethylsulfamidyl]}-hexanoic acid, (S)-6-amino-2-{[(S)-2-cyclohexyl-1-((1R,2S,4R)-1,7,7-trimethyl-bicyclo[2.2.1]hept-2-ylcarbamoyl)-ethylsulfamidyl]}-hexanoic acid, (S)-6-amino-2-{[(S)-1-((1S,4R)-bicyclo[2.2.1]hept-2-ylcarbamoyl)-2-cyclohexyl-ethylsulfamidyl]}-hexanoic acid, (S)-6-amino-2-{[(S)-2-cyclohexyl-1-(octahydro-4,7-methano-inden-5-ylcarbamoyl)-ethylsulfamidyl]}-hexanoic acid, (S)-6-amino-2-[((S)-1-tert-butylcarbamoyl-2-cyclohexyl-ethylsulfamidyl)]-hexanoic acid, (S)-2-{[(S)-1-(adamantan-1-ylcarbamoyl)-2-phenyl-ethylsulfamidyl]}-6-amino-hexanoic acid, (S)-6-amino-2-{[(S)-1-((1S,4R)-bicyclo[2.2.1]hept-2-ylcarbamoyl)-2-phenyl-ethylsulfamidyl]}-hexanoic acid, (S)-2-{[(S)-1-(adamantan-1-ylcarbamoyl)-2-(4-hydroxy-phenyl)-ethylsulfamidyl]}-6-amino-hexanoic acid, (S)-6-amino-2-{[(S)-2-phenyl-1-((1R,2S,4R)-1,7,7-trimethylbicyclo[2.2.1]hept-2-ylcarbamoyl)-ethylsulfamidyl]}-hexanoic acid, (S)-6-amino-2-({[(S)-cyclohexyl-(1,7,7-trimethyl-bicyclo[2.2.1]hept-2-ylcarbamoyl)-methyl]-sulfamidyl})-hexanoic acid, (S)-6-amino-2-{[(S)-2-cyclohexyl-1-((1R,2R,4R)-1,7,7-trimethyl-bicyclo[2.2.1]hept-2-ylcarbamoyl)-ethylsulfamidyl]}-hexanoic acid, (S)-6-amino-2-{[(S)-2-cyclopropyl-1-((1R,2R,4R)-1,7,7-trimethyl-bicyclo[2.2.1]hept-2-ylcarbamoyl)-ethylsulfamidyl]}-hexanoic acid, (S)-6-amino-2-{[(S)-2-cyclohexyl-1-(3,5-dimethyl-adamantan-1-ylcarbamoyl)-ethylsulfamidyl]}-hexanoic acid, (S)-6-amino-2-{[(S)-2-cyclohexyl-1-(3-isopropyl-adamantan-1-ylcarbamoyl)-ethylsulfamidyl]}-hexanoic acid, tert-butyl (S)-6-amino-2-{[(S)-2-cyclohexyl-1-((1R,2R,4R)-1,7,7-trimethyl-bicyclo[2.2.1]hept-2-ylcarbamoyl)-ethylsulfamidyl]}-hexanoate or (S)-2-{[(S)-1-(adamantan-1-ylcarbamoyl)-3-methyl-butylsulfamidyl]}-6-amino-hexanoic acid.

6. A process for preparing the compound of the formula I as claimed in one or more of claims 1 to 5, which comprises
a) reacting a compound of the formula II 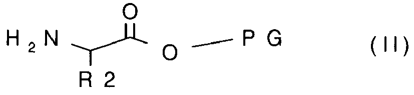 where R2 and PG have the meanings mentioned in the compound of the formula I,
with a compound of the formula IX 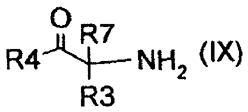 where R3, R4, R7 and PG have the meanings mentioned in the compound of the formula I, to give a compound of the formula X 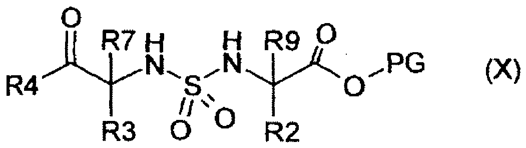 where R2, R3, R4, R7, R9 and PG have the meanings mentioned in the compound of the formula I, and then converting it into a compound of the formula I, or
b) fractionating a compound of the formula I prepared by process a), or a suitable precursor of the formula I which occurs in enantiomeric forms owing to its chemical structure, by salt formation with enantiopure acids or bases, chromatography on chiral stationary phases or derivatization by means of chiral enantiopure compounds such as amino acids, separation of the diastereomers obtained in this way, and elimination of the chiral auxiliary groups into the pure enantiomers, or
c) either isolating in free form the compound of the formula I prepared by processes a) or b), or converting into physiologically tolerated salts in the case where acidic or basic groups are present.

7. A medicament having an effective content of at least one compound of the formula I as claimed in one or more of claims 1 to 5 together with a pharmaceutically suitable and physiologically tolerated carrier, additive, and/or other active ingredients and excipients.

8. The use of the compound of the formula I 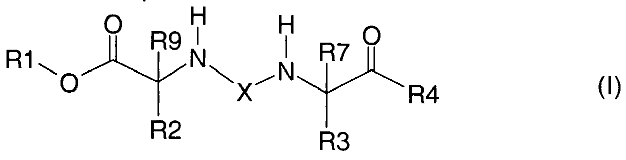 and/or of a stereoisomeric form of the compound of the formula I and/or mixtures of these forms in any ratio, and/or a physiologically tolerated salt of the compound of the formula I as claimed in claims 1 to 5, for the manufacture of a medicament for the prophylaxis, secondary prevention and therapy of one or more disorders associated with thromboses, embolisms, hypercoagulability or fibrotic changes selected from the series myocardial infarction, angina pectoris and other forms of acute coronary syndrome, stroke, peripherally vascular disorders, deep vein thrombosis, pulmonary embolism, embolic or thrombotic events caused by cardiac arrhythmias, cardiovascular events such as restenosis following revascularization and angioplasty and similar procedures such as stent implantations and bypass operations, or reducing the risk of thrombosis following surgical procedures such as operations on the knee and hip, or disseminated intravascular coagulation, sepsis and other intravascular events associated with inflammation, or atherosclerosis, diabetes and the metabolic syndrome and the sequelae thereof, tumor growth and tumor metastasis, inflammatory and degenerative articular disorders such as rheumatoid arthritis and arthrosis, impairments of the hemostatic systems such as fibrin deposits, fibrotic changes of the lung such as chronic obstructive pulmonary disease, adult respiratory distress syndrome or fibrin deposits in the eye following eye operations or prevention or treatment of scarring.

## Revendications

1. Composé de formule I 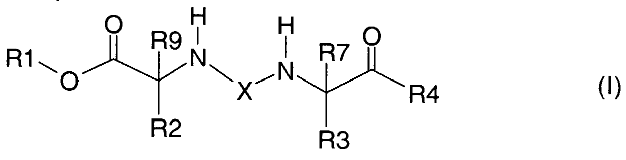 et/ou une forme stéréoisomère du composé de formule I et/ou des mélanges de ces formes en toute proportion, et/ou un sel physiologiquement toléré du composé de formule I, dans laquelle
X est -S(O)₂-,
R1 est
1) atome d'hydrogène,
2) -(C₁-C₆)-alkyle,
3) -(C₀-C₄)-alkylène-(C₃-C₁₂)-cycloalkyle ou
4) -(C₁-C₆)-alkylène-(C₆-C₁₄)-aryle,
R2 est un radical de formule II
-(A1)ₘ-A2 (II)
dans laquelle
m est l'entier zéro ou 1,
A1 est
1) -(CH₂)ₙ- dans lequel n est l'entier zéro, 1, 2 ou 3,
2) -NH-(CH₂)ₙ- dans lequel n est l'entier zéro, 1, 2 ou 3,
3) -NH(C₁-C₆)-alkyl)-(CH₂)ₙ-, dans lequel n est l'entier zéro, 1, 2 ou 3,
4) -NH((C₃-C₆)-cycloalkyl)-(CH₂)ₙ- dans lequel n est l'entier zéro, 1, 2 ou 3,
5) -O-(CH₂)ₙ- dans lequel n est l'entier zéro, 1, 2 ou 3, ou
6) -(CH₂)ₙ-SOₓ- dans lequel n est l'entier zéro, 1, 2 ou 3, et x est l'entier zéro, 1 ou 2,
A2 est
1) Het, où Het signifie un système de cycles hétérocycliques à 4 à 15 chaînons ayant de 4 à 15 atomes du cycle qui sont présents dans 1, 2 ou 3 systèmes de cycles liés les uns aux autres, et qui comprennent 1, 2, 3 ou 4 hétéroatomes identiques ou différents de la série oxygène, azote ou soufre, et sont non substitués ou substitués indépendamment les uns des autres une, deux ou trois fois par -(C₁-C₃)-alkyle, halogène, -NH₂, -CF₃ ou -O-CF₃,
2) -(C₀-C₆)-alkylène-NH₂ ,
3) -(C₁-C₆)-alkylène-NH-C(=NH)-NH₂,
4) -(C₁-C₆)-alkylène-NH-C(=NH)-(C₁-C₄)-alkyle,
5) -(C₀-C₄)-alkylène-O-NH-C(=NH)-NH₂,
6) -(C₀-C₄)-alkylène-NH-C(O)-(C₁-C₆)-alkyle,
7) -(C₁-C₆)-alkylène-NH-C(O)-O-(C₁-C₄)-alkylène-aryle, où aryle est non substitué ou substitué par -NH₂ ou est substitué par -NH₂ et une, deux ou trois fois par R15,
8) -(C₃-C₈)-cycloalkyl-NH₂, ou
9) -(C₀-C₄)-alkylène-(C₆-C₁₄)-aryle, où aryle est non substitué ou substitué par -NH₂ ou est substitué par -NH₂ et une, deux ou trois fois par R15,
R3 est
1) -(C₁-C₆)-alkyle,
2) -(C₀-C₄)-alkylène-(C₃-C₁₂)-cycloalkyle,
3) -(C₁-C₆)-alkylène-(C₆-C₁₄)-aryle, où aryle est substitué indépendamment les uns des autres une, deux ou trois fois par R15,
4) -(C₀-C₈)-alkylène-N(R5)-PG,
5) -(C₁-C₆)-alkylène-NH-C(O)-O-(C₁-C₄)-alkylène-aryle où aryle est substitué indépendamment les uns des autres une, deux ou trois fois par R15,
6) -(C₀-C₄)-alkylène-(C₆-C₁₄)-aryl-(C₀-C₄)-alkylène-N(R5)-PG,
7) -(C₀-C₈)-alkylène-O-PG,
8) -(C₀-C₄)-alkylène-(C₆-C₁₄)-aryl-(C₀-C₄)-alkylène-O-PG,
9) -(C₀-C₈)-alkylène-C(O)-O-PG,
10) -(C₀-C₄)-alkylène-(C₆-C₁₄)-aryl-(C₀-C₄)-alkylène-C(O)-O-PG ou
11) atome d'hydrogène,
R4 est -N(R6)₂,
où R6 sont identiques ou différents et sont indépendamment l'un de l'autre
1) atome d'hydrogène,
2) -(C₁-C₆)-alkyle,
3) -(C₀-C₄)-alkylène-(C₃-C₁₂)-cycloalkyle, où cycloalkyle est non substitué ou substitué indépendamment les uns des autres une, deux, trois ou quatre fois par R11, halogène, -C(O)-O-R11, -(C₁-C₄)-alkyl-O-R11 ou -O-(C₁-C₄)-alkyle,
4) -(C₀-C₆)-alkylène-(C₆-C₁₄)-aryle, où aryle et alkylène sont non substitués ou substitués indépendamment l'un de l'autre une, deux, trois ou quatre fois par R11, halogène, -C(O)-O-R11, -(C₁-C₄)-alkyl-O-R11, -C(O)-N(R8)₂ ou -O-(C₁-C₄)-alkyle,
5) -(C₀-C₈)-alkylène-N(R5)-PG,
6) -(C₀-C₄)-alkylène-(C₆-C₁₄)-aryl-(C₀-C₄)-alkyl-N(R5)-PG,
7) -(C₀-C₈)-alkylène-O-PG,
8) -(C₀-C₄)-alkylène-(C₆-C₁₄)-aryl-(C₀-C₄)-alkyl-O-PG,
9) -(C₀-C₈)-alkyène-C(O)-O-R11,
10) -(C₀-C₄)-alkylène-(C₀-C₁₄)-aryl-(C₀-C₄)-alkyl-C(O)-O-PG,
11) -(C₀-C₄)-alkylène-Het, où Het signifie un système de cycles hétérocycliques à 4 à 15 chaînons ayant de 4 à 15 atomes du cycle qui sont présents dans un, deux ou trois systèmes de cycles liés ensemble, et qui comprennent un, deux, trois ou quatre hétéroatomes identiques ou différents de la série oxygène, azote ou soufre, où Het ou alkylène sont non substitués ou substitués indépendamment l'un de l'autre une, deux ou trois fois par R11, halogène, -C(O)-O-R11, -(C₁-C₄)-alkyl-O-R11 ou -O-(C₁-C₄)-alkyle,
12) -(C₁-C₃)-fluoroalkyle,
13) -(C₀-C₄)-alkylène-CH(R11)-C(O)-NH₂,
14) -(C₀-C₄)-alkylène-CH(R11)-C(O)-NH-(C₁-C₄)-alkyle, ou
15) -(C₀-C₄)-alkylène-CH(R11)-C(O)-NH-CH(R12)-R13,
ou les deux radicaux R6 forment ensemble avec l'atome de N auquel ils sont liés un cycle mono- ou bicyclique ayant de 4 à 9 atomes du cycle qui est saturé, partiellement saturé ou aromatique, où le cycle est non substitué ou substitué une ou deux fois par -(C₁-C₄)-alkyle, -C(O)-O-R11, halogène, -(C₁-C₄)-alkyl-O-R11 ou phényle,
R5 est atome d'hydrogène ou -(C₁-C₆)-alkyle,
PG est un groupement protecteur de la fonction amino, carboxy ou hydroxy,
R7 est atome d'hydrogène ou -(C₁-C₆)-alkyle,
R8 est atome d'hydrogène ou -(C₁-C₆)-alkyle,
R9 est atome d'hydrogène ou -(C₁-C₆)-alkyle,
R11 et R12 sont identiques ou différents et sont indépendamment l'un de l'autre
1) atome d'hydrogène,
2) -(C₁-C₆)-alkyle,
3) -(C₀-C₄)-alkylène-phényle, où phényle est non substitué ou substitué indépendamment les uns des autres une, deux ou trois fois par halogène, -OH ou -O-(C₁-C₄)-alkyle,
4) -(C₀-C₄)-alkylène-(C₃-C₁₂)-cycloalkyle, où cycloalkyle est non substitué ou substitué indépendamment les uns des autres une, deux, trois ou quatre fois par R13, halogène, -C(O)-O-R13, -(C₁-C₄)-alkyl-O-R13, -O-(C₁-C₄)-alkyle ou -(C₀-C₄)-alkylène-phényle,
5) -(C₀-C₄)-alkylène-C(O)-N(R13)₂ ou
6) -(C₀-C₄)-alkylène-indolyle,
R13 est
1) atome d'hydrogène,
2) -(C₁-C₄)-alkyle,
3) -(C₀-C₄)-alkylène-C(O)-O-R14,
4) -(C₀-C₄)-alkylène-C(O)-R14 ou
5) -(C₀-C₄)-alkylène-O-R14,
R14 est atome d'hydrogène, -(C₁-C₄)-alkyle, -NH₂ ou -OH, et
R15 est atome d'hydrogène, -(C₁-C₄)-alkyle, -O-CF₃, -NH₂, - OH, -CF₃ ou halogène.

2. Composé de formule I selon la revendication 1, **caractérisé en ce que**
R1 est
1) atome d'hydrogène ou
2) -(C₁-C₄)-alkyle,
R2 est
1) -(C₁-C₆)-alkylène-NH₂,
2) -(C₀-C₄)-alkylène-pyridyl-NH₂,
3) -(C₀-C₄)-alkylène-pipéridinyl-NH₂,
4) -(C₀-C₄)-alkylène-thiazolyl-NH₂,
5) -(C₁-C₆)-alkylène-NH-C(=NH)-NH₂,
6) -(C₀-C₄)-alkylène-(C₃-C₈)-cycloalkyl-NH₂,
7) -(C₁-C₆)-alkylène-NH-C(=NH)-(C₁-C₄)-alkyle,
8) -(C₀-C₄)-alkylène-O-NH-C(=NH)-NH₂,
9) -(C₁-C₆)-alkylène-NH-C(O)-O-(C₁-C₄)-alkylène-aryle, où aryle est non substitué ou substitué par -NH₂ ou est substitué par -NH₂ et une, deux ou trois fois par R15,
10) -(C₀-C₄)-alkylène-NH-C(O)-(C₁-C₄)-alkyle,
11) -(C₀-C₄)-alkylène-(C₆-C₁₄)-aryle, où aryle est non substitué ou substitué par -NH₂ ou est substitué par -NH₂ et une, deux ou trois fois par R15, ou
12) -(C₁-C₄)-alkylène-SOₓ-(C₁-C₄)-alkylène-NH₂ dans lequel x est l'entier zéro, 1 ou 2,
R3 est
1) -(C₁-C₄)-alkyle,
2) -(C₀-C₄)-alkylène-(C₃-C₈)-cycloalkyle,
3) -(C₁-C₆)-alkylène-aryle, où aryle est substitué indépendamment les uns des autres une, deux ou trois fois par R15,
4) -(C₁-C₆)-alkylène-NH-C(O)-O-(C₁-C₄)-alkylène-aryle, où aryle est substitué indépendamment les uns des autres une, deux ou trois fois par R15,
5) -(C₁-C₆)-alkylène-NH-PG,
6) -(C₁-C₆)-alkylène-O-PG,
7) -(C₁-C₆)-alkyle, ou
8) atome d'hydrogène,
où PG est t-butyl-, t-butyloxycarbonyle ou benzyloxycarbonyle,
R4 est -N(R6)₂,
où R6 sont identiques ou différents et sont indépendamment l'un de l'autre
1) atome d'hydrogène,
2) -(C₁-C₆)-alkyle,
3) -(C₀-C₄)-alkylène-(C₃-C₁₂)-cycloalkyle, où cycloalkyle est non substitué ou substitué indépendamment les uns des autres une, deux, trois ou quatre fois par R11, halogène, - C(O)-O-R11, -(C₁-C₄)-alkyl-O-R11 ou -O-(C₁-C₄)-alkyle,
4) -(C₀-C₄)-alkylène-C(R11)(R12)-(C₃-C₁₂)-cycloalkyle, où cycloalkyle est non substitué ou substitué indépendamment les uns des autres une, deux ou trois fois par R11, halogène, -C(O)-O-R11, -(C₁-C₄)-alkyl-O-R11 ou -O-(C₁-C₄)-alkyle,
5) -(C₀-C₄)-alkylène-Het, où Het signifie un système de cycles hétérocycliques à 4 à 15 chaînons ayant de 4 à 15 atomes du cycle qui sont présents dans un, deux ou trois systèmes de cycles liés ensemble, et qui comprennent un, deux, trois ou quatre hétéroatomes identiques ou différents de la série oxygène, azote ou soufre, où Het ou alkylène sont non substitués ou substitués indépendamment l'un de l'autre une, deux ou trois fois par R11, halogène, -C(O)-O-R11, -(C₁-C₄)-alkyl-O-R11 ou -O-(C₁-C₄)-alkyle,
6) -(C₀-C₆)-alkylène-aryle, où aryle ou alkylène est non substitué ou substitué indépendamment l'un de l'autre une, deux ou trois fois par R11, halogène, -C(O)-O-R11, -(C₀-C₄)-alkyl-O-R11 ou -O-(C₁-C₄)-alkyle,
7) -(C₀-C₄)-alkylène-C(R11)(R12)-aryle, où aryle ou alkylène est non substitué ou substitué indépendamment l'un de l'autre une, deux ou trois fois par R11, halogène, -C(O)-O-R11, -(C₀-C₄)-alkyl-O-R11 ou -O-(C₁-C₄)-alkyle,
8) 1,2,3,4-tétrahydronaphtalényle,
9) -(C₀-C₄)-alkylène-CH(R11)-C(O)-NH₂,
10) -(C₀-C₄)-alkylène-CH(R11)-C(O)-NH-(C₁-C₄)-alkyle,
11) -(C₀-C₄)-alkylène-CH(R11)-C(O)-NH-CH(R12)-R13,
12) -(C₀-C₆)-alkylène-C(O)-O-R11, où alkylène est non substitué ou substitué indépendamment les uns des autres une ou deux fois par R11, halogène, -C(O)-O-R11, -(C₁-C₄)-alkyl-O-R11 ou -O-(C₁-C₄)-alkyle,
13) -(C₀-C₄)-alkylène-C(R11)(R12)-C(O)-O-R11, ou
14) -(C₁-C₃)-fluoroalkyle,
ou les deux radicaux R6 forment avec l'atome de N auquel ils sont liés un cycle mono- ou bicyclique ayant de 4 à 9 atomes du cycle qui est saturé, partiellement saturé ou aromatique, où le cycle est non substitué ou substitué une ou deux fois par -(C₁-C₄)-alkyle, -C(O)-O-R11, halogène, -(C₁-C₄)-alkyl-O-R11 ou phényle,
R7 est atome d'hydrogène ou -(C₁-C₄)-alkyle,
R9 est atome d'hydrogène ou -(C₁-C₄)-alkyle,
R11 et R12 sont identiques ou différents et sont indépendamment l'un de l'autre
1) atome d'hydrogène,
2) -(C₁-C₄)-alkyle,
3) -(C₀-C₄)-alkylène-phényle, où phényle est non substitué ou substitué indépendamment les uns des autres une, deux ou trois fois par halogène, -OH ou -O-(C₁-C₄)-alkyle,
4) -(C₀-C₄)-alkylène-(C₃-C₁₂)-cycloalkyle, où cycloalkyle est non substitué ou substitué indépendamment les uns des autres une, deux, trois ou quatre fois par R13, halogène, -C(O)-O-R13, -(C₁-C₄)-alkyl-O-R13, -O-(C₁-C₄)-alkyle ou -(C₀-C₄)-alkylène-phényle,
5) -(C₀-C₄)-alkylène-C(O)-N(R13)₂ ou
6) -(C₀-C₄)-alkylène-indolyle,
R13 est
1) atome d'hydrogène,
2) -(C₁-C₄)-alkyle,
3) -(C₀-C₄)-alkylène-C(O)-O-R14,
4) -(C₀-C₄)-alkylène-C(O)-R14 ou
5) -(C₀-C₄)-alkylène-O-R14,
R14 est atome d'hydrogène, -(C₁-C₄)-alkyle, -NH₂ ou -OH, et R15 est atome d'hydrogène, -(C₁-C₄)-alkyle, -O-CF₃, -NH₂, -OH, -CF₃ ou halogène.

3. Composé de formule I selon la revendication 1 ou 2, **caractérisé en ce que**
R1 est
1) atome d'hydrogène ou
2) -(C₁-C₄)-alkyle,
R2 est
1) -(C₁-C₆)-alkylène-NH₂,
2) -(C₁-C₄)-alkylène-pyridyl-NH₂,
3) -(C₁-C₄)-alkylène-pipéridinyl-NH₂,
4) -(C₁-C₆)-alkylène-NH-C(=NH)-NH₂,
5) -(C₀-C₄)-alkylène-(C₃-C₆)-cycloalkyl-NH₂,
6) -(C₁-C₆)-alkylène-NH-C(=NH)-(C₁-C₄)-alkyle,
7) -(C₁-C₄)-alkylène-O-NH-C(=NH)-NH₂,
8) -(C₁-C₆)-alkylène-NH-C(O)-O-(C₁-C₄)-alkylène-phényle, où phényle est non substitué ou substitué par -NH₂ ou est substitué par -NH₂ et une, deux ou trois fois par R15,
9) -(C₁-C₄)-alkylène-NH-C(O)-(C₁-C₆)-alkyle,
10) -(C₁-C₄)-alkylène-phényle, où phényle est non substitué ou substitué par -NH₂ ou est substitué par - NH₂ et une, deux ou trois fois par R15,
11) -(C₁-C₄)-alkylène-SO₂-(C₁-C₄)-alkylène-NH₂ ou
12) -(C₁-C₄)-alkylène-S-(C₁-C₄)-alkylène-NH₂,
R3 est
1) -((C₁-C₄)-alkyle,
2) -((C₁-C₄)-alkylène-(C₃-C₆)-cycloalkyle,
3) -((C₁-C₄)-alkylène-phényle, où phényle est substitué indépendamment les uns des autres une, deux ou trois fois par R15,
4) -((C₁-C₆)-alkylène-NH-C(O)-O-(C₁-C₄)-alkylène-phényle, où phényle est substitué indépendamment les uns des autres une, deux ou trois fois par R15,
5) atome d'hydrogène,
R4 est -N(R6)₂,
où R6 sont identiques ou différents et sont indépendamment l'un de l'autre
1) atome d'hydrogène,
2) -((C₁-C₄)-alkyle,
3) -((C₀-C₄)-alkylène-(C₃-C₁₂)-cycloalkyle, où cycloalkyle est choisi dans le groupe de cyclohexyle, cyclopentyle, cyclobutyle, cyclopropyle, adamantanyle, 1,7,7-triméthylbicyclo[3,1,1]heptanyle, décahydronaphtalényle, tétrahydronaphtalényle, octahydro-4,7-méthanoindényle ou bicyclo[2,2,1]heptanyle et dans lequel cycloalkyle est non substitué ou substitué indépendamment les uns des autres une, deux, trois ou quatre fois par -(C₁-C₄)-alkyle, -C(O)-O-R11 ou -(C₁-C₄)-alkylène-phényle, où phényle est non substitué ou substitué par halogène,
4) -((C₀-C₄)-alkylène-C(R11)(R12)-(C₃-C₁₂)-cycloalkyle, où cycloalkyle est choisi dans le groupe de cyclohexyle, cyclopentyle, cyclobutyle, cyclopropyle, adamantanyle, 1,7,7-triméthylbicyclo[3,1,1]heptanyle, décahydronaphtalényle, tétrahydronaphtalényle, octahydro-4,7-méthanoindényle ou bicyclo[2,2,1]heptanyle et dans lequel cycloalkyle est non substitué ou substitué indépendamment les uns des autres une, deux, trois ou quatre fois par -(C₁-C₄)-alkyle, -C(O)-O-R11 ou -(C₁-C₄)-alkylène-phényle où phényle est non substitué ou substitué par halogène,
5) -((C₀-C₄)-alkylène-Het, où Het est choisi dans le groupe d'acridinyle, azépinyle, azétidinyle, aziridinyle, benzimidazalinyle, benzimidazolyle, benzo[1,3]dioxolyle, benzofuranyle, benzothiofuranyle, benzothiophényle, benzoxazolyle, benzthiazolyle, benztriazolyle, benztétrazolyle, benzisoxazolyle, benzisothiazolyle, carbazolyle, 4aH-carbazolyle, carbolinyle, quinazolinyle, quinoléinyle, 4H-quinoléizinyle, quinoxalinyle, quinuclidinyle, chromanyle, chroményle, cinnolinyle, décahydroquinoléinyle, dibenzofuranyle, dibenzothiophényle, dihydrofurane[2,3-b]-tétrahydrofuranyle, dihydrofuranyle, dioxolyle, dioxanyle, 2H, 6H-1,5,2-dithiazinyle, furanyle, furazanyle, imidazolidinyle, imidazolinyle, imidazolyle, 1H-indazolyle, indolinyle, indolizinyle, indolyle, 3H-indolyle, isobenzofuranyle, isochromanyle, isoindazolyle, isoindolinyle, isoindolyle, isoquinoléinyle (benzimidazolyle), isothiazolidinyle, 2-isothiazolinyle, isothiazolyle, isoxazolyle, isoxazolidinyle, 2-isoxazolinyle, morpholinyle, naphtyridinyle, octahydroisoquinoléinyle, oxadiazolyle, 1,2,3-oxadiazolyle, 1,2,4-oxadiazolyle, 1,2,5-oxadiazolyle, 1,3,4-oxadiazolyle, oxazolidinyle, oxazolyle, oxazolidinyle, oxothiolanyle, pyrimidinyle, phénanthridinyle, phénanthrolinyle, phénazinyle, phénothiazinyle, phénoxathiinyle, phénoxazinyle, phtalazinyle, pipérazinyle, pipéridinyle, ptéridinyle, purynyle, pyranyle, pyrazinyle, pyroazolidinyle, pyrazolinyle, pyrazolyle, pyridazinyle, pyridooxazolyle, pyridoimidazolyle, pyridothiazolyle, pyridothiophényle, pyridinyle, pyridyle, pyrimidinyle, pyrrolidinyle, pyrrolinyle, 2H-pyrrolyle, pyrrolyle, tétrahydrofuranyle, tétrahydroisoquinoléinyle, tétrahydroquinoléinyle, tétrahydropyridinyle, 6H-1,2,5-thiadazinyle, 1,2,3-thiadiazolyle, 1,2,4-thiadiazolyle, 1,2,5-thiadiazolyle, 1,3,4-thiadiazolyle, thianthrényle, thiazolyle, thiényle, thiénoimidazolyle, thiénooxazolyle, thiénopyridine, thiénothiazolyle, thiomorpholinyle, thiophényle, triazinyle, 1,2,3-triazolyle, 1,2,3-triazolyle, 1,2,4-triazolyle, 1,2,5-triazolyle, 1,3,4-triazolyle et xanthényle, où Het ou alkylène est non substitué ou substitué indépendamment l'un de l'autre une ou deux fois par -(C₁-C₄)-alkyle,
6) -((C₁-C₆)-alkylène-phényle, où phényle ou alkylène sont non substitués ou substitués indépendamment l'un de l'autre une ou deux fois par halogène, phényle, -C(0)-0-R11, -(C₁-C₄)-alkyl-O-R11, -O-(C₁-C₄)-alkyle ou -(C₁-C₄)-alkyle,
7) -((Co-C4)-alkylène-C(R111)(R12)-phényle, où phényle est non substitué ou substitué indépendamment les uns des autres une, deux ou trois fois par phényle ou fluor,
8) 1,2,3,4-tétrahydronaphtalényle,
9) -((C₀-C₄)-alkylène-CH(R11)-C(O)-NH₂,
10) -((C₀-C₄)-alkylène-CH(R11)-C(O)-NH-(C1-C4)-alkyle,
11) -((C₀-C₄)-alkylène-CH(R11)-C(O)-NH-CH(R12)-R13,
12) -((C₁-C₆)-alkylène-C(O)-O-R11, où alkylène est non substitué ou substitué indépendamment les uns des autres une ou deux fois par halogène, phényle, -C(O)-O-R11, -(C₁-C₄)-alkyl-O-R11, -O-(C₁-C₄)-alkyle ou -(C₁-C₄)-alkyle,
13) -((C₀-C₄)-alkylène-C(R11)(R12)-C(O)-O-R11, ou
14) -((C₁-C₃)-fluoroalkyle,
ou les deux radicaux R6 forment ensemble avec l'atome de N auquel ils sont liés un cycle mono- ou bicyclique choisi dans le groupe de pyrrolidine, pipéridine, 2-aza-bicyclo[3,2,2]nonane et 7-aza-bicyclo[2,2,1]heptane, où le cycle est non substitué ou substitué une ou deux fois par -(C₁-C₄)-alkyle, -C(O)-O-R11, -(C₁-C₄)-alkyl-O-R11 ou phényle,
R7 est atome d'hydrogène ou -(C₁-C₄)-alkyle,
R9 est atome d'hydrogène ou -(C₁-C₄)-alkyle,
R11 et R12 sont identiques ou différents et sont indépendamment l'un de l'autre
1) atome d'hydrogène,
2) -(C₁-C₄)-alkyle,
3) -(C₀-C₄)-alkylène-phényle, où phényle est non substitué ou substitué indépendamment les uns des autres une, deux ou trois fois par -OH, halogène ou -O-(C₁-C₄)-alkyle,
4) -(C₀-C₄)-alkylène-(C₃-C₁₂)-cycloalkyle, où cycloalkyle est choisi dans le groupe de cyclohexyle, cyclopentyle, cyclobutyle, cyclopropyle, adamantanyle, 1,7,7-triméthylbicyclo[3,1,1]heptanyle, décahydronaphtalényle, tétrahydronaphtalényle, octahydro-4,7-méthanoindényle ou bicyclo[2,2,1]heptanyle et dans lequel cycloalkyle est non substitué ou substitué indépendamment les uns des autres une, deux, trois ou quatre fois par -(C₁-C₄)-alkyle, -C(O)-O-R13 ou phényle, ou
5) -(C₀-C₄)-alkylène-indolyle,
R13 est
1) atome d'hydrogène,
2) -(C₁-C₄)-alkyle,
3) -(C₀-C₄)-alkylène-C(O)-O-R14,
4) -(C₀-C₄)-alkylène-C(O)-R14 ou
5) -(C₀-C₄)-alkylène-O-R14, et
R14 est atome d'hydrogène, -(C₁-C₄)-alkyle, -NH₂ ou -OH et
R15 est atome d'hydrogène, -(C₁-C₄)-alkyle, -O-CF₃, -NH₂, - OH, -CF₃ ou halogène.

4. Composé de formule I selon les revendications 1 à 3, **caractérisé en ce que**
R1 est
1) atome d'hydrogène ou
2) -(C₁-C₄)-alkyle,
R2 est
1) -(C₁-C₆)-alkylène-NH₂,
2) -(C₁-C₄)-alkylène-pyridyl-NH₂,
3) -(C₁-C₄)-alkylène-pipéridinyl-NH₂,
4) -(C₁-C₄)-alkylène-NH-C(=NH)-NH₂,
5) -(C₁-C₆)-alkylène-NH-C(=NH)-(C₁-C₄)-alkyle,
6) -(C₁-C₄)-alkylène-(C₃-C₆)-cycloalkyl-NH₂,
7) -(C₁-C₄)-alkylène-O-NH-C(=NH)-NH₂,
8) -(C₁-C₆)-alkylène-NH-C(O)-O-(C₁-C₄)-alkylène- phényle,
9) -(C₁-C₄)-alkylène-NH-C(O)-(C₁-C₆)-alkyle,
10) -(C₁-C₄)-alkylène-phényl-NH₂,
11) -(C₁-C₄)-alkylène-SO₂-(C₁-C₄)-alkylène-NH₂ ou
12) -(C₁-C₄)-alkylène-S-(C₁-C₄)-alkylène-NH₂,
R3 est
1) -(C₁-C₄)-alkyle,
2) -(C₁-C₄)-alkylène-(C₃-C₆)-cycloalkyle,
3) -(C₁-C₄)-alkylène-phényle, où phényle est non substitué ou substitué par -OH,
4) -(C₁-C₆)-alkylène-NH-C(O)-O-(C₁-C₄)-alkylène- phényle,
5) atome d'hydrogène,
R4 est -N(R6)₂,
où R6 sont identiques ou différents et sont indépendamment l'un de l'autre
1) atome d'hydrogène,
2) -(C₁-C₆)-alkyle,
3) -(C₀-C₄)-alkylène-(C₃-C₈)-cycloalkyle, où cycloalkyle est choisi parmi le groupe de cyclohexyle, cyclopentyle, cyclopropyle, adamantanyle, 1,7,7-triméthylbicyclo[3,1,1]heptanyle, décahydronaphtalène, octahydro-4,7-méthanoindényle ou bicyclo[2,2,1]heptanyle et dans lequel cycloalkyle est non substitué ou substitué indépendamment les uns des autres une, deux ou trois fois par -(C₁-C₄)-alkyle ou phényle,
4) -C(R11)(R12)-adamantanyle,
5) -CH(R11)-C(O)-NH-CH(R12)-R13,
6) -(C₀-C₄)-alkylène-Het, où Het est choisi dans le groupe de benzimidazolyle, isoxazolyle, pipéridine, pyridine, pyrrolidinyle, thiophényle et benzo[1,3]dioxol,
7) 1,2,3,4-tétrahydronaphtalényle,
8) -(C₀-C₄)-alkylène-C(R11)(R12)-phényle, où phényle est non substitué ou substitué indépendamment les uns des autres une, deux ou trois fois par phényle ou fluor,
9) -CH(R11)-C(O)-NH₂,
10) -CH(R11)-C(O)-NH-CH(R12)-CH2-OH,
11) -(C₁-C₆)-alkylène-phényle, où phényle ou alkylène sont non substitués ou substitués indépendamment l'un de l'autre une ou deux fois par chlore, fluor, -C(O)-O-R11, -(C₁-C₄)-alkyl-O-R11, -O-(C₁-C₄)-alkyle, phényle ou -(C₁-C₄)-alkyle,
12) -CH(R11)-C(O)-NH-(C₁-C₄)-alkyle,
13) -(C₀-C₄)-alkylène-C(R11)(R12)-bicyclo[3,1,1]heptanyle, où bicyclo[3,1,1]heptanyle est non substitué ou substitué une à quatre fois par -(C₁-C₄)-alkyle,
14) -(C₁-C₆)-alkylène-C(O)-O-R11, où alkylène est non substitué ou substitué indépendamment les uns des autres une ou deux fois par chlore, fluor, -C(O)-O-R11, -(C₁-C₄)-alkyl-O-R11, -O-(C₁-C₄)-alkyle, phényle ou -(C₁-C₄)-alkyle,
15) -(C₀-C₄)-alkylène-C(R11)(R12)-C(O)-O-R11, ou
16) -CH₂-CF₂-CF₃,
ou les deux radicaux R6 forment ensemble avec l'atome de N auquel ils sont liés un cycle mono- ou bicyclique choisi dans le groupe de pyrrolidines, 2-azabicyclo[3,2,2]nonane et 7-azabicyclo[2,2,1]heptane, où le cycle est non substitué ou substitué une ou deux fois par -(C₁-C₄)-alkyle, -C(O)-O-R11, -(C₁-C₄)-alkyl-O-R11 ou phényle,
R7 est atome d'hydrogène ou -(C₁-C₄)-alkyle,
R9 est atome d'hydrogène ou -(C₁-C₄)-alkyle,
R11 et R12 sont identiques ou différents et sont indépendamment l'un de l'autre
1) atome d'hydrogène,
2) -(C₁-C₄)-alkyle,
3) -(C₀-C₄)-alkylène-phényle, où phényle est non substitué ou substitué indépendamment les uns des autres une, deux ou trois fois par -OH, halogène ou -O-(C₁-C₄)-alkyle,
4) -(C₀-C₄)-alkylène-(C₃-C₁₂)-cycloalkyle, où cycloalkyle est choisi dans le groupe de cyclohexyle, cyclopentyle, cyclobutyle, cyclopropyle, adamantanyle, 1,7,7-triméthylbicyclo[3,1,1]heptanyle, décahydronaphtalényle, octahydro-4,7-méthanoindényle ou bicyclo[2,2,1]heptanyle et dans lequel cycloalkyle est non substitué ou substitué indépendamment les uns des autres une, deux, trois ou quatre fois par -(C₁-C₄)-alkyle, -C(O)-O-R13 ou phényle ou
5) -(C₀-C₄)-alkylène-indolyle,
R13 est
1) atome d'hydrogène,
2) -(C₁-C₄)-alkyle,
3) -(C₀-C4)-alkylène-C(O)-O-R14,
4) -(C₀-C₄)-alkylène-C(O)-R14 ou
5) -(C₀-C₄)-alkylène-O-R14,
R14 est atome d'hydrogène, -(C₁-C₄)-alkyle, -NH₂ ou -OH et
R15 est atome d'hydrogène, -(C₁-C₄)-alkyle, -O-CF₃, -NH₂, - OH, -CF₃ ou halogène.

5. Composé de formule I selon une ou plusieurs des revendications 1 à 4, **caractérisé en ce qu'**il s'agit du composé de formule I acide (S)-6-amino-2-(3-{(S)-1-[(S)-1-((S)-1-méthoxycarbonyl-2-méthyl-propylcarbamoyl)-2-méthyl-propylcarbamoyl]-2-phényl-éthyl}-sulfamidyl)-hexanoïque, acide (S)-6-amino-2-{3-[(R)-1-(bicyclo[2,2,1]hept-2-ylcarbamoyl)-2-cyclohexyl-éthyl]-sulfamidyl}-hexanoïque, acide (S)-6-amino-2-[3-((S)-1-cyclohexylcarbamoyl-2-phényl-éthyl)-sulfamidyl]-hexanoïque, acide (S)-6-acétimidoylamino-2-{[(S)-2-cyclohexyl-1-((1R,2S,4R)-1,7,7-triméthyl-bicyclo[2,2,1]hept-2-ylcarbamoyl)-éthylsulfamidyl]}-hexanoïque, 3-(6-amino-pyridin-3-yl)-2-[(S)-2-cyclohexyl-1-((1 R,2S,4R)-1,7,7-triméthylbicyclo[2,2,1]hept-2-ylcarbamoyl)-éthylsulfamidyl]-propionate d'éthyle, acide (S)-6-amino-2-{[(S)-1-((1R,2S,4R)-1,7,7-triméthylbicyclo[2,2,1]hept-2-ylcarbamoyl)-propylsulfamidyl]-}-hexanoïque, acide (S)-2-{[(S)-2-cyclohexyl-1-((1R,2S,4R)-1,7,7-triméthylbicyclo[2,2,1]hept-2-ylcarbamoyl)-éthylsulfamidyl]-}-5-guanidinopentanoïque, acide (S)-6-amino-2-{[(S)-2-cyclobutyl-1-((1R,2S,4R)-1,7,7-triméthyl-bicyclo[2,2,1]hept-2-ylcarbamoyl)-éthylsulfamidyl]}hexanoïque, acide (S)-5-amino-2-({2-cyclohexyl-1-[(R)-(1,2,3,4tétrahydro-naphtalén-2-yl)carbamoyl]-éthylsulfamidyl})-pentanoïque, (S)-2-{[(S)-2-cyclohexyl-1-((1R,2S,4R)-1,7,7-triméthylbicyclo[2,2,1]hept-2-ylcarbamoyl)-éthylsulfamidyl]}-6-hexanoylaminohexanoate d'éthyle, acide (S)-6-amino-2-{[(S)-1-((1R,2S,4R)-1,7,7-triméthyl-bicyclo[2,2,1]hept-2-ylcarbamoyl)-butylsulfamidyl]}-hexanoïque, acide (S)-6-amino-2-{[(S)-3-cyclohexyl-1-((1R,2S,4R)-1,7,7-triméthyl-bicyclo[2,2,1]hept-2-ylcarbamoyl)-propylsulfamidyl]-méthyl}-hexanoïque, acide (S)-6-amino-2-{[(S)-2-cyclohexyl-1-(décahydro-naphtalén-2-ylcarbamoyl)-éthylsulfamidyl]}-hexanoïque, acide (S)-2-{[(S)-1-(adamantan-1-ylcarbamoyl)-2-cyclohexyl-éthylsulfamidyl]}-6-amino-hexano'ique, acide (S)-2-[(S)-2-cyclohexyl-1-((1R,2S,4R)-1,7,7-triméthyl-bicyclo[2,2,1]-hept-2-ylcarbamoyl)-éthylsulfamidyl]-3-pyridin-3-yl-propionique, acide (S)-2-[(S)-2-cyclohexyl-1-((1R,2S,4R)-1,7,7-triméthyl-bicyclo[2,2,1]hept-2-ylcarbamoyl)-éthylsulfamidyl]-3-pyridin-4-yl-propionique, acide (S)-6-amino-2-{[(S)-2-cyclohexyl-1-((1S,2S,3S,5R)-2,6,6-triméthyl-bicyclo[3,1,1]hept-3-ylcarbamoyl)-éthylsulfamidyl]}-hexanoïque, acide (S)-6-amino-2-{[(S)-2-cyclohexyl-1-(3,3,5-triméthyl-cyclohexylcarbamoyl)-éthylsulfamidyl]}-hexanoïque, acide (S)-6-amino-2-{[(S)-1-(4-tert-butyl-cyclohexylcarbamoyl)-2-cyclohexyl-éthylsulfamidyl]}-hexano'ique, acide (S)-6-amino-2-{[(S)-2-cyclohexyl-1-(3-méthylcyclohexylcarbamoyl)-éthylsulfamidyl]}-hexanoïque, acide (S)-6-amino-2-{[(S)-2-cyclohexyl-1-((1R,2S,4R)-1,7,7-triméthylbicyclo[2,2,1]hept-2-ylcarbamoyl)-éthylsulfamidyl]}-hexanoïque, acide 3-(6-amino-pyridin-3-ylméthyl)-2-[(S)-2-cyclohexyl-1-((1R,2S,4R)-1,7,7-triméthyl-bicyclo[2,2,1]hept-2-yl-carbamoyl)-éthylsulfamidyl]-propionique, acide 2-[(S)-2-cyclohexyl-1-((1R,2S,4R)-1,7,7-triméthyl-bicyclo[2,2,1]hept-2-ylcarbamoyl)-éthylsulfamidyl]-3-pipéridin-4-yl-propionique, acide (S)-3-(4-amino-phényl)-2-[(S)-2-cyclohexyl-1-((1R,2S,4R)-1,7,7-triméthylbicyclo[2,2,1]hept-2-ylcarbamoyl)-éthylsulfamidyl]-propionique, acide (S)-6-amino-2-[((S)-1-cyclohexylméthyl-2-oxo-2-pipéridin-1-yléthylsulfamidyl)]-hexanoïque, acide (S)-5-amino-2-{[(S)-2-cyclohexyl-1-((1R,2S,4R)-1,7,7-triméthyl-bicyclo[2,2,1]hept-2-ylcarbamoyl)-éthylsulfamidyl]}-pentanoïque, acide (S)-6-amino-2-{[(S)-2-cyclohexyl-1-((S)-1-isobutylcarbamoyl-2-méthyl-propylcarbamoyl)-éthylsulfamidyl]}-hexanoïque, acide (S)-6-amino-2-{[(S)-1-((S)-1-isobutylcarbamoyl-2-méthyl-propylcarbamoyl)-2-phényl-éthylsulfamidyl]}-hexanoïque, acide (S)-6-amino-2-[((S)-2-cyclohexyl-1-isobutylcarbamoyl-éthylsulfamidyl)]-hexanoïque, acide (S)-6-amino-2-{[(S)-1-((1R,2R,4S)-bicyclo[2,2,1]hept-2-ylcarbamoyl)-2-cyclohexyl-éthylsulfamidyl]}-hexanoïque, acide (S)-6-amino-2-{[(S)-2-cyclohexyl-1-((1R,2S,4R)-1,7,7-triméthyl-bicyclo[2,2,1]hept-2-ylcarbamoyl)-éthylsulfamidyl]}-hexanoïque, acide (S)-6-amino-2-{[(S)-1-((1S,4R)-bicyclo[2,2,1]hept-2-ylcarbamoyl)-2-cyclohexyl-éthylsulfamidyl]}-hexanoïque, acide (S)-6-amino-2-{[(S)-2-cyclohexyl-1-(octahydro-4,7-méthano-indén-5-ylcarbamoyl)-éthylsulfamidyl]}-hexanoïque, acide (S)-6-amino-2-[((S)-1-tert-butylcarbamoyl-2-cyclohexyl-éthylsulfamidyl)]-hexanoïque, acide (S)-2-{[(S)-1-(adamantan-1-ylcarbamoyl)-2-phényl-éthylsulfamidyl]}-6-amino-hexanoïque, acide (S)-6-amino-2-{[(S)-1-((1S,4R)-bicyclo[2,2,1]hept-2-ylcarbamoyl)-2-phényl-éthylsulfamidyl]}-hexanoïque, acide (S)-2-{[(S)-1-(adamantan-1-ylcarbamoyl)-2-(4-hydroxy-phényl)-éthylsulfamidyl]}-6-amino-hexano'ique, acide (S)-6-amino-2-{[(S)-2-phényl-1-((1R,2S,4R)-1,7,7-triméthylbicyclo[2,2,1]hept-2-ylcarbamoyl)-éthylsulfamidyl]}-hexanoïque, acide (S)-6-amino-2-({[(S)-cyclohexyl-(1,7,7-triméthylbicyclo[2,2,1]hept-2-ylcarbamoyl)-méthyl]-sulfamidyl})-hexanoïque, acide (S)-6-amino-2-{[(S)-2-cyclohexyl-1-((1R,2R,4R)-1,7,7-triméthyl-bicyclo[2,2,1]hept-2-ylcarbamoyl)-éthylsulfamidyl]}-hexanoïque, acide (S)-6-amino-2-{[(S)-2-cyclopropyl-1-((1R,2R,4R)-1,7,7-triméthyl-bicyclo[2,2,1]hept-2-ylcarbamoyl)-éthylsulfamidyl]}-hexanoïque, acide (S)-6-amino-2-{[(S)-2-cyclohexyl-1-(3,5-diméthyladamantan-1-ylcarbamoyl)-éthylsulfamidyl]}-hexanoïque, acide (S)-6-amino-2-{[(S)-2-cyclohexyl-1-(3-isopropyl-adamantan-1-ylcarbamoyl)-éthylsulfamidyl]}-hexanoïque, (S)-6-amino-2-{[(S)-2-cyclohexyl-1-((1R,2R,4R)-1,7,7-triméthyl-bicyclo[2,2,1]hept-2-ylcarbamoyl)-éthylsulfamidyl]}-hexanoate de tert-butyle ou acide (S)-2-{[(S)-1-(adamantan-1-ylcarbamoyl)-3-méthyl-butylsulfamidyl]}-6-amino-hexanoïque.

6. Procédé de préparation du composé de formule I selon une ou plusieurs des revendications 1 à 5, **caractérisé en ce qu'**il comprend
a) la réaction d'un composé de formule II 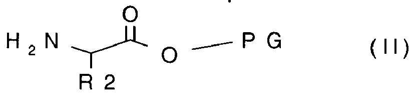 dans laquelle R2 et PG ont les significations mentionnées dans le composé de formule I, avec un composé de formule IX 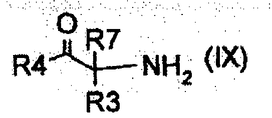 dans laquelle R3, R4, R7 et PG ont les significations mentionnées dans le composé de formule I, pour donner un composé de formule X 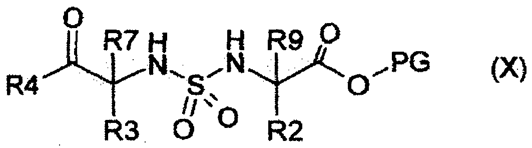 dans laquelle R2, R3, R4, R7, R9 et PG ont les significations mentionnées dans le composé de formule I, et alors la transformation en un composé de formule I, ou
b) le fractionnement d'un composé de formule I préparé par le procédé a), ou d'un précurseur convenable de formule I qui existe sous des formes énantiomères en raison de sa structure chimique, par formation de sels avec des acides ou des bases énantiopurs, chromatographie en phases stationnaires chirales ou dérivatisation à l'aide de composés énantiopurs chiraux tels que les acides aminés, séparation des diastéréoisomères obtenus de cette façon, et élimination des groupements auxiliaires chiraux en énantiomères purs, ou
c) soit l'isolement, sous forme libre, du composé de formule I préparé par les procédés a) ou b), soit la transformation en sels physiologiquement tolérés dans le cas où des groupements acides ou basiques sont présents.

7. Médicament **caractérisé par** une teneur efficace d'au moins un composé de formule I selon une ou plusieurs des revendications 1 à 5 conjointement avec un support pharmaceutiquement convenable et physiologiquement toléré, un additif et/ou d'autres principes actifs et excipients.

8. Utilisation du composé de formule I 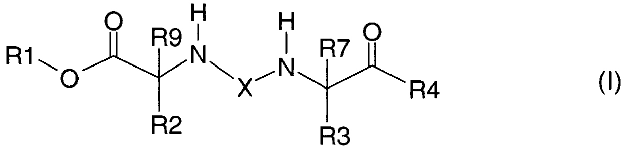 et/ou d'une forme stéréoisomère du composé de formule I et/ou de mélanges de ces formes dans une proportion quelconque, et/ou d'un sel physiologiquement toléré du composé de formule I, selon les revendications 1 à 5 , pour la fabrication d'un médicament destiné à la prophylaxie, la prévention secondaire et la thérapie d'un ou plusieurs troubles associés à des thromboses, des embolies, l'hypercoagulabilité ou des changements fibrotiques choisis dans la série comprenant l'infarctus du myocarde, l'angine de poitrine et d'autres formes de syndrome coronarien aigu, l'accident vasculaire cérébral, les troubles vasculaires périphériques, la thrombose veineuse profonde, l'embolie pulmonaire, les événements emboliques ou thrombotiques provoqués par des arythmies cardiaques, les événements cardiovasculaires tels que la resténose suite à une revascularisation et une angioplastie et des opérations analogues telles que les implantations de stents et les opérations de pontage, ou la réduction du risque de thrombose suite à des opérations chirurgicales telles que les opérations sur le genou et la hanche, ou la coagulation intravasculaire disséminée, un état septique et d'autres événements intravasculaires associés à l'inflammation, ou l'athérosclérose, le diabète et le syndrome métabolique et les séquelles de ceux-ci, la croissance tumorale et la métastase tumorale, les troubles articulaires inflammatoires et dégénératifs tels que la polyarthrite rhumatoïde et l'arthrose, les altérations du système hémostatique telles que les dépôts de fibrine, les changements fibrotiques du poumon tels que la bronchopneumopathie chronique obstructive, le syndrome de détresse respiratoire chez l'adulte ou les dépôts de fibrine dans l'oeil suite à des opérations de l'oeil ou la prévention ou le traitement de cicatrices.
